# EUROPEAN PATENT SPECIFICATION

(11) **EP 2 943 509 B1**
(45) Date of publication and mention of the grant of the patent: **10.04.2019**
(21) Application number: 14700572.2
(22) Date of filing: 09.01.2014
(51) Int. Cl.: C07K 16/28, A61P 35/00, A61K 39/395, A61K 39/00

(54) **USE OF ANTIBODIES AGAINST ICAM-1 IN COMBINATION WITH AN ANTI CANCER DRUG IN THE TREATMENT OF PATIENTS.**
VERWENDUNG VON ANTIKÖRPERN GEGEN ICAM-1 IN KOMBINATION MIT EINEM ANTI-KREBS-MEDIKAMENT IN DER BEHANDLUNG VON PATIENTEN
UTILISATION D'ANTICORPS CONTRE ICAM-1 EN COMBINAISON AVEC UN ANTI CANCEREUX DANS LE TRAITEMENT DES PATIENTS.

(30) Priority: 09.01.2013 GB 201300346
(43) Date of publication of application: 18.11.2015
(73) Proprietor: BioInvent International AB, 223 70 Lund (SE)
(72) Inventor: FRENDEUS, Björn, 224 68 Lund (SE); HANSSON, Markus, 241 93 Eslöv (SE)
(74) Representative: Potter Clarkson
(86) International application number: PCT/EP2014/050274
(87) International publication number: WO 2014/108456

(56) References cited:
- WO-A1-2010/112110
- WO-A1-2012/007516

## Description

The present invention relates to ICAM-1 antibodies and their use in combination with known anticancer agents in the treatment of cancers such as multiple myeloma.

### Background

Multiple myeloma (also referred to as MM or myeloma) is a malignancy of B cells and accounts for 10% to 20% of total haematological malignancies. At present, it is an incurable disease with a median age at diagnosis of 65-70 years, and with very few patients diagnosed below the age of 40. Multiple myeloma is the second most common hematologic malignancy in the United States with a worldwide incidence of 4 per 100,000 individuals. In the United States, 19,920 new cases of multiple myeloma and more than 10,000 deaths are expected in 2008 to be myeloma-related (American Cancer Society, 2008). The disease has a slight male preponderance and is found more frequently in African Americans and less commonly in Asian populations (Kyle & Rajkumar, Blood. 2008 Mar 15;111(6):2962-72. Review).

The diagnosis of myeloma carries a grave prognosis, with a median survival of 3 to 4 years with currently available treatments, although individuals with severe forms of the disease may have a median survival of only 2 years, even with optimal treatment (Kyle & Rajkumar, Blood. 2008 Mar 15;111(6):2962-72. Review).

The typical clinical picture of myeloma is a patient with severe pain due to pathological bone fractures, particularly in the rib cage or vertebral column (Kyle & Rajkumar, 2004, N Engl J Med. 2004 Oct 28;351 (18):1860-73. Review). Other common features are renal failure, hypercalcemia, bone marrow insufficiency with anemia and thrombocytopenia, and also increased risks of infection and thromboembolic complications such as venous thrombosis and pulmonary embolism. Organ failure is sometimes caused by pathological deposition of fibrillar aggregates of immunoglobulin light chains, called AL-amyloidosis. When present, it typically involves heart and kidneys resulting in severe cardiac arrhythmias and/or failure, and renal malfunction and failure, respectively.

Multiple myeloma is characterized by a great unmet medical need - currently available drugs for treatment of multiple myeloma are non-curative and associated with significant toxicity and development of drug resistance. Multiple myeloma plasma cells typically do not express CD20, or show low and heterogeneous CD20 expression, making CD20 targeted therapies unlikely to be effective in this disease (Kapoor et al. Haematol, 2008, 141:135-248).

A range of approaches have been developed for treating individuals with multiple myeloma, including the use of: Melfalan (and other alkylating substances such as cyclophosphamide); other chemotherapeutic agents (such as adriamycin, vincristin and cisplatin); high steroid dosages; interferon; bisphosphonates (such as pamidronate or zoledronate). In addition, autologous and allogeneic stem-cell transplantation have been used.

Despite recent advances in the development of novel therapies for treating or preventing multiple myeloma, the actual benefit of those drugs on patient survival and quality of life are as yet limited (Kumar et al, Blood, 2008, 111(5):2516-20). Furthermore, the present treatments are associated with severe side-effects in a significant proportion of patients. For example, chemotherapy results in increased sensitivity to infection, nausea, loss of hair and organ damage; steroid treatment may result in weight gain, diabetes, increased sensitivity to infection; osteoporosis and mental disturbances; interferon treatment may lead to fatigue, fever, muscle pain and depression; and bisphosphonate treatment rarely but sometimes results in kidney damage and bone necrosis. Procedures deploying stem-cell transplantation is accompanied by significant rates of relapse and transplant-related morbidity and mortality. The novel myeloma drugs, comprising thalidomide, bortezomib and lenalidomide, also have side-effects limiting their use in many patients.

In recent years, substantial progress has been made in understanding the pathogenesis and molecular mechanisms of multiple myeloma. Genetic studies have revealed the occurrence of a vast array of different chromosomal changes, often carrying prognostic relevance, connected with this disease. Briefly, these chromosomal translocations often involve the immunoglobulin (Ig) H locus (14q32.3) and juxtaposes various transforming genes to segments promoted by the Ig enhancer, causing a disregulated expression and potentially malignant transformation (Hideshima et al. Nat Rev Cancer. 2007. 7(8): 585-598). The therapeutic effect of proteasome inhibition with bortezomib in myeloma was first demonstrated in myeloma cells *in vitro,* and is probably a result of direct cytotoxicity and of a decrease in the expression of adhesion molecules and various growth, survival and angiogenic factors (Kyle & Rajkumar N Engl J Med. 2004 Oct 28;351 (18): 1860-73. Review). The transcription factor NFκB, has enhanced activity in myeloma due to proteasomal degradation of its normal regulator protein IκB, and bortezomib reinstates NPκB homeostasis by inhibiting proteasome activity.

The bone marrow microenvironment, consisting of bone osteoclasts, endothelial cells, bone marrow stem cells, as well as extracellular matrix proteins, has a crucial role in multiple myeloma pathogenesis (Hideshima et al., Nat Rev Cancer. 2007. 7(8): 585-598), and provide factors mediating growth, survival and drug resistance of the malignant plasma cells. Various adhesion molecules expressed by the myeloma cells are important for this interaction, for example ICAM-1.

The intercellular adhesion molecule-1 (ICAM-1) is highly expressed and involved in the pathogenesis of multiple types of human malignancies, including myeloma (Huang, et al. Hybridoma. 1993. 12(6): 661-675; Huang et al. Cancer Res. 1995. 55(3): 610-616; Smallshaw et al. Immunother 1997. 2004. 27(6): 419-424; Schmidmaier, Int J Biol Markers. 2006. 21(4): 218-222), melanoma (Wang et al. Int J Cancer. 2006. 118(4): 932-941; Johnson et al., Immunobiology. 1988. 178(3): 275-284), lung cancer (Grothey et al. Br J Cancer. 1998. 77(5): 801-807), gastric cancer (Maruo et al. Int J Cancer. 2002. 100(4): 486-490), bladder cancer (Roche et al. Thromb Haemost. 2003. 89(6): 1089-1097), breast cancer (Rosette C, et al. Carcinogenesis. 2005. 26(5): 943-950), prostate cancer (Aalinkeel R et al. Cancer Res 2004. 64(15): 5311-21), and lymphoma (Horst et al. Leukemia. 1991. 5(10): 848-853). Increased ICAM-1 expression is associated with development of drug-induced resistance (Schmidmaier et al. Int J Biol Markers. 2006. 21(4): 218-222), tumour cell aggressiveness (Miele et al., Exp Cell Res 214 (1), 231 1994) and poor prognosis Dowlati et al., Clin Cancer Res 14 (5), 1407 (2008).

Standard treatment for myeloma in younger patients (*i.e.* less than 65 years of age) has consisted of conditioning with vincristine-adriamycin-dexamethasone followed by high-dose melphalan with autologous stem cell support. During the previous decade, this regime was shown to prolong median survival by approximately 1 year, in spite of achieving complete remission in the bone marrow in only a minority of patients (Harousseau JL. Hematology Am Soc Hematol Educ Program. 2008;2008:306-12). Due to the risks attached to high-dose treatment, elderly patients have primarily been offered treatment with low-dose melphalan combined with prednisone.

In recent years, other therapies have been approved for the treatment of relapsed myeloma. These new drugs comprise the proteasome-inhibitor bortezomib (Velcade®), and the "immunomodulatory" drugs, thalidomide and lenalidomide (Revlimid®), and constitute a significant progress in treatment options. The overall response rate for relapsed myeloma patients with these drugs is usually around 30%, but generally higher when the drug is combined with intermittent dexamethasone. Based on these findings the new drugs, combined with dexamethasone and/or chemotherapy, are now in clinical trials as first line treatment for myeloma (http://clinicaltrials.gov/ct2/search) with promising preliminary results (American Society of Hematology, December 6-9, 2008).

Although bortezomib, lenalidomide and thalidomide have shown a survival benefit in comparison to traditional therapy in relapsed myeloma patients (Rajkumar Blood. 2005. 106(13): 4050-4053; Richardson et al. Blood. 2006. 108(10): 3458-3464; Richardson et al. N Engl J Med. 2005. 352(24): 2487-2498; Singhal et al. N Engl J Med. 1999. 341(21): 1565-1571), the goal of increasing long-term survival or a cure has yet not been reached. Furthermore, the newer drugs also have serious side effects, for example increased risks of thromboembolism, neuropathy and immune and bone marrow suppression, limiting their use in a significant number of patients.

Despite recent advances in the development of novel therapies the actual benefit of these drugs on patient survival and quality of life are modest with many patients either not responding or developing drug resistance and subsequently relapsing, warranting development of novel more effective and complimentary therapeutics to combat multiple myeloma. Accordingly, new potential targets and drugs for myeloma therapy are required.

The evolution of targeted immunotherapy and antibody-based drugs over the past two decades has significantly improved physicians' armature to combat cancer and hematological malignancy (1). In hematological malignancies, the CD20-specific mouse human chimeric monoclonal antibody rituximab provides the prime example of a therapeutic antibody, which when used as monotherapy (2) (3) or when combined with conventional chemotherapeutic drugs (4-6) has significantly improved non-Hodgkin lymphoma patient's survival (7, 8). A significant fraction of patients with hematological disorders, however, remain ineligible for anti-CD20 treatment owing to tumor cell lack of CD20 expression or inherent or acquired resistance to CD20 therapy (9). Development of a new generation of antibodies with activity against these currently incurable cancers is therefore highly warranted to improve clinical outcome.

The applicants herein describe a novel human ICAM-1 antibody targeting the B11 epitope that has broad, highly efficacious and potent anti-tumor activity compared to currently available treatment against different transplanted CD20-expressing and CD20-negative human B cell tumors *in vivo.* The ICAM-1 B11 antibody was isolated from the naive antibody library n-CoDeR® (Biolnvent) by differential biopanning and programmed cell death (PCD) screening based on its targeting of a tumor B cell upregulated surface receptor and its competitive programmed cell death inducing properties. To the best of our knowledge this is first time functional screening methodology has been successfully applied to on the one hand identify novel functions of a previously well-characterised receptor (ICAM-1) and at the same time generating an antibody against the same target with such significant therapeutic potential. This "function-first-approach" to therapeutic antibody discovery therefore constitutes an important and complementary strategy to more conventional approaches utilizing panning against recombinant target protein or target transfected cells, where the retrieved antibody pool is restricted to specificities against pre-defined target receptor(s).

WO2010/112110 describes an antibody or antigen-binding fragment thereof with binding specificity for ICAM-1, and the use thereof in medicine for the treatment of cancers, such as multiple myeloma.

Our finding that IgG B11 has competitive *in vivo* anti-tumor activity compared to rituximab against CD20 expressing tumors, along with an apparent frequent ICAM-1 expression in different lymphoma subtypes, indicates that it may be applicable for treatment of CD20 expressing lymphomas. While overall rituximab has significantly improved Non-Hodgkin's lymphoma (NHL) patient survival when used either alone or in combination with chemotherapy (8), a significant fraction of patients with relapsed or refractory CD20⁺ follicular lymphomas does not respond to initial therapy with rituximab (2) and more than half of prior rituximab responding patients acquire resistance and no longer benefit from retreatment (63).

Importantly, it is shown herein that IgG B11 has broad and potent *in vivo* anti-myeloma activity. While CD20 is broadly expressed during B cell development from the early pre-B cell stage until after B cell exposure to antigen, antibody secreting plasma cells and cancers originating from this stage including multiple myeloma, typically do not express or show low and heterogenous expression of CD20. Multiple Myeloma is therefore unlikely to be effectively treated with CD20 targeted therapies like rituximab (23).

In contrast, observations suggest that ICAM-1 may be a suitable target for myeloma therapy, and in particular for targeted therapy with an antibody like IgG B11; Recent reports describe that ICAM-1 is strongly expressed by primary multiple myeloma plasma cells.

Consistent with these observations it is demonstrated herein that the vast majority of myeloma cells, at levels increased compared to patient's non-myelomatous lymphocytes, expressed the epitope targeted by B11.

In agreement with direct cytotoxicity being an important mechanism for IgG B11 anti-tumor activity, it is demonstrated that IgG B11 anti-tumor activity correlated with IgG binding and saturation of tumor cell expressed ICAM-1 receptors. Further, consistent with a major mode-of-action being direct tumor cell cytotoxicity, IgG B11, in addition to its documented programmed cell death inducing properties (10), conferred Fc:FcγR-dependent anti-tumor activity both *in vivo* and *in vitro.* Accumulating evidence suggest that interactions between antibody constant domain (Fc) and host Fc gamma receptors (FcγR) are instrumental for rituximab and other cancer antibodies' clinical activity, at least in distinct patient groups (52, 53, 65-67).

Thus, in independent studies NHL patients homozygous for the FcγRIIIa allele with highest affinity for the antibody constant Fc domain have shown improved survival compared to patients carrying at least one lower affinity FcγRIIIa allele in response to treatment with rituximab, and rituximab *in vivo* anti-tumor activity critically depends on antibody Fc:host FcyR-interactions (54).

While there is currently no antibody approved for treatment of multiple myeloma, Fc:FcγR-engaging therapeutic antibodies have transformed the way hematologic and solid cancers are being treated (1). Preclinical data demonstrate that ImiDs currently used and developed for myeloma treatment enhance Fc:FcR-dependent anti-tumor activity (Wu et al (2008) Clin Cancer Res, 14 pp4650-7). IMiDs are structural and functional analogues of thaliodomide that represent a promising new class of immunomodulators for treatment of a varienty of inflammatory, aufoimmune and neoplastic diseases.

These observations suggest that provided appropriate myeloma associated receptors and Fc:FcγR-engaging antibodies targeting these structures can be identified, such target specific antibodies may improve myeloma treatment and disease outcome when used alone or in combination with immunomodulatory drugs (1). Therefore, based on available literature and herein presented data it is shown that ICAM-1:IgG B11 may provide an attractive axis for myeloma therapy.

In further support for ICAM-1 targeted intervention of myeloma therapy, ICAM-1 is implicated in multiple myeloma pathogenesis and development of drug-resistance at multiple levels (12, 14, 22). Multiple myeloma is characterized by the infiltration and expansion of malignant plasma cells in the bone marrow, and myeloma cells depend on interactions with stromal cells to proliferate and survive. ICAM-1, by binding to its ligands integrin αLβ2, integrin αMβ2, and muc-1, is involved in cell adhesive events triggering multiple cell signaling pathways promoting multiple myeloma cell increased proliferation, migration, resistance to PCD, and development of cell adhesion molecule induced drug-resistance (12, 68, 69). Planned studies aim at investigating IgG B11's effect on ICAM-1 dependent human myeloma-human stromal cell interactions using the *scid-hu* in vivo experimental model (70) and *in vitro* co-cultures of myeloma cells with osteoblasts or osteoclasts (71).

The improved anti-tumor activity of IgG B11 compared to rituximab and bortezomib is intriguing from a mechanistic point of view. Improved anti-tumor activity did not result from a greater number of ICAM-1 epitopes expressed by tumor cells compared to rituximab epitopes, indicating that IgG B11 either delivered stronger death signals or induced cell death by different signaling or effector pathways compared to rituximab. The ability to trigger cell death via pathways distinct from those of rituximab and bortezomib opens up the possibility for combined treatment as triggering of complementary death pathways is thought to enhance the likelihood of therapeutic efficacy and possibility to cure. Thus, the differential signaling pathways used by ImiDs, lenalidomide, bortezomib and dexamethasone to trigger myeloma cell apoptosis (via caspase-8 or caspase-9) have provided an important rationale for combining these agents in myeloma therapy (26, 72-74).

Our finding that myeloma cell ICAM-1 expression is retained following treatment with bortezomib, revlimid, alkeran, and dexamethasone in vivo, demonstrate the molecular requirements for IgG B11 combined treatment with these drugs.

A therapeutic cancer antibody must in addition to exerting significant anti-tumor activity be safe and tolerable by patients. ICAM-1 shows restricted expression and tissue distribution under normal physiological circumstances but is upregulated on several cell types in response to tissue injury or inflammatory stress, raising safety concerns about treatment with anti-ICAM-1 antibodies. Previous studies by independent investigators demonstrated, however, that anti-ICAM-1 antibody was well tolerated by different patient groups (75-79). Our preclinical safety assessment of B11 indicates that it will be safe and well-tolerated and there is no evidence that B11 will enhance or interfere with critical immune cell function.

From a pharmacological perspective and owing to its fully human nature, B11 is expected to be low or non-immunogenic compared to previously developed mouse or chimeric ICAM-1 antibodies (80), and to have a half-life typical for human IgGs (2-3 weeks). Thus, based on its significant preclinical anti-myeloma activity and an expected safe profile, clinical trials with IgG B11 in multiple myeloma have now commenced in the US.

In summary, the applicants have identified a novel cell death inducing ICAM-1 antibody (IgG B11) that has enhanced anti-myeloma activity compared to currently used treatments including bortezomib (Velcade), Dexamethasone, Revlimid, and Alkeran in preclinical models of multiple myeloma. As the applicants have shown that B11 appears to induce cell death by different signaling or effector pathways than the above current treatments, this provides rationale for combined treatments using B11 together with current anti-cancer agents such as bortezomib (Velcade), Dexamethasone, Revlimid, and Alkeran.

From the examples herein described, it can be seen that an anti ICAM-1 antibody can combine with already known conventional chemotherapeutic treatments to provide an improved outcome for the patient (e.g. decreased tumour size).

Therefore, in a first aspect of the invention there is provided:
A composition comprising: (i) an antibody or an antigen-binding fragment thereof with binding specificity for ICAM-1, wherein the antibody or antigen-binding fragment thereof comprises the following amino acid sequences:
FSNAWMSWVRQAPG and
AFIWYDGSNKYYADSVKGR and
ARYSGWYFDY and
CTGSSSNIGAGYDVH and
DNNNRPS and
CQSYDSSLSAWL; (ii) a further anticancer agent selected from IMiDs and bortezomib; and (iii) a pharmaceutically acceptable excipient, diluent or carrier.

In one embodiment of any aspect of the invention, the further anticancer agent is selected from bortezomib and lenalidomide.

Also disclosed herein, the further anticancer agent (ii) is selected from Bortezomib (Velcade), Dexamethasone, Revlimid and Alkeran.

In one embodiment the further anticancer agent (ii) is present in an individually effective dose.

In a further embodiment the further anticancer agent (ii) is present in a lower than individually effective dose.

In a second aspect of the invention there is provided a therapeutic system for the treatment of cancer comprising a combination of component (i) an antibody or an antigen-binding fragment thereof with binding specificity for ICAM-1, wherein the antibody or an antigen-binding fragment thereof comprises the following amino acid sequences:
FSNAWMSWVRQAPG and
AFIWYDGSNKYYADSVKGR and
ARYSGWYFDY and
CTGSSSNIGAGYDVH and
DNNNRPS and
CQSYDSSLSAWL;
and component (ii) a further anticancer agent selected from IMiDs and bortezomib, the components (i) and (ii) being provided for the use in the treatment of cancer, wherein each of components (i) and (ii) optionally additionally comprises a pharmaceutically acceptable excipient, diluent or carrier, and wherein components (i) and (ii) are administered in combination with one another.

In an embodiment of the invention the administration of component (i) precedes administration of component (ii).

In a further embodiment the administration of component (ii) precedes administration of component (i).

In a further embodiment the administration of component (i) occurs at the same time as administration of component (ii).

Conveniently, each of components (i) and (ii) additionally comprises a pharmaceutically acceptable excipient, diluent or carrier.

Also disclosed herein in a third aspect is a use of an antibody or an antigen-binding fragment thereof with binding specificity for ICAM-1, or a variant, fusion or derivative of said antibody or an antigen-binding fragment, or a fusion of a said variant or derivative thereof, with binding specificity for ICAM-1; together with a further anticancer agent, in the manufacture of a medicament for the treatment of cancer.

In a fourth aspect of the invention, there is provided an antibody or an antigen-binding fragment thereof with binding specificity for ICAM-1, wherein the antibody or antigen-binding fragment thereof comprises the following amino acid sequences:
FSNAWMSWVRQAPG and
AFIWYDGSNKYYADSVKGR and
ARYSGWYFDY and
CTGSSSNIGAGYDVH and
DNNNRPS and
CQSYDSSLSAWL;
together with a further anticancer agent selected from IMiDs and bortezomib, for use in the treatment of cancer.

Also disclosed herein in a fifth aspect is a method for treating cancer, the method comprising the step of administering to the patient an effective amount of an antibody or an antigen-binding fragment thereof with binding specificity for ICAM-1, or a variant, fusion or derivative of said antibody or an antigen-binding fragment, or a fusion of a said variant or derivative thereof, with binding specificity for ICAM-1; together with a further anticancer agent.

Also disclosed herein in a sixth aspect is the use of a composition as defined in the first aspect in the manufacture of a medicament for the treatment of cancer.

In a seventh aspect of the invention there is provided a composition as defined in the first aspect for use in the treatment of cancer.

Also disclosed herein in an eighth aspect is a method for treating cancer, the method comprising the step of administering to the patient an effective amount of a composition as defined in the first aspect.

Also disclosed herein, the further anticancer agent of the composition, system, use, antibody or method of any one of the preceding aspects is selected from Bortezomib, Dexamethasone, Revlimid and Alkeran.

In a further embodiment, the further anticancer agent of the composition, system, use, antibody or method of any one of the preceding aspects is present in an individually effective dose.

In a further embodiment, the further anticancer agent of the composition, system, use, antibody or method of any one of the preceding aspects is present in a lower than individually effective dose.

Cancer treatments promote tumour regression by inhibiting tumour cell proliferation, inhibiting angiogenesis (growth of new blood vessels that is necessary to support tumour growth) and/or prohibiting metastasis by reducing tumour cell motility or invasiveness.

The antibodies of the invention may be effective in adult and pediatric oncology including in solid phase tumours/malignancies, locally advanced tumours, human soft tissue sarcomas, metastatic cancer, including lymphatic metastases, blood cell malignancies including multiple myeloma, acute and chronic leukemias, and lymphomas, head and neck cancers including mouth cancer, larynx cancer and thyroid cancer, lung cancers including small cell carcinoma and non-small cell cancers, breast cancers including small cell carcinoma and ductal carcinoma, gastrointestinal cancers including esophageal cancer, stomach cancer, colon cancer, colorectal cancer and polyps associated with colorectal neoplasia, pancreatic cancers, liver cancer, urologic cancers including bladder cancer and prostate cancer, malignancies of the female genital tract including ovarian carcinoma, uterine (including endometrial) cancers, and solid tumour in the ovarian follicle, kidney cancers including renal cell carcinoma, brain cancers including intrinsic brain tumours, neuroblastoma, astrocytic brain tumours, gliomas, metastatic tumour cell invasion in the central nervous system, bone cancers including osteomas, skin cancers including malignant melanoma, tumour progression of human skin keratinocytes, squamous cell carcinoma, basal cell carcinoma, hemangiopericytoma and Karposi's sarcoma.

Therapeutic compositions can be administered in therapeutically effective dosages alone or in combination with adjuvant cancer therapy such as surgery, chemotherapy, radiotherapy, thermotherapy, and laser therapy, and may provide a beneficial effect, e.g. reducing tumour size, slowing rate of tumour growth, inhibiting metastasis, or otherwise improving overall clinical condition, without necessarily eradicating the cancer.

The composition can also be administered in therapeutically effective amounts as a portion of an anti-cancer cocktail. An anti-cancer cocktail is a mixture of the compound or modulator of the invention with one or more anti-cancer drugs in addition to a pharmaceutically acceptable carrier for delivery. The use of anti-cancer cocktails as a cancer treatment is routine. Anti-cancer drugs that are well known in the art and can be used as a treatment in combination with the polypeptide or modulator of the invention include: Actinomycin D, Aminoglutethimide, Asparaginase, Bleomycin, Busulfan, Carboplatin, Carmustine, Chlorambucil, Cisplatin (cis-DDP), Cyclophosphamide, Cytarabine HCI (Cytosine arabinoside), Dacarbazine, Dactinomycin, Daunorubicin HCI, Doxorubicin HCI, Estramustine phosphate sodium, Etoposide (V16-213), Floxuridine, 5-Fluorouracil (5-Fu), Flutamide, Hydroxyurea (hydroxycarbamide), Ifosfamide, Interferon Alpha-2a, Interferon Alpha-2b, Leuprolide acetate (LHRH-releasing factor analog), Lomustine, Mechlorethamine HCI (nitrogen mustard), Melphalan, Mercaptopurine, Mesna, Methotrexate (MTX), Mitomycin, Mitoxantrone HCI, Octreotide, Plicamycin, Procarbazine HCI, Streptozocin, Tamoxifen citrate, Thioguanine, Thiotepa, Vinblastine sulfate, Vincristine sulfate, Amsacrine, Azacitidine, Hexamethylmelamine, Interleukin-2, Mitoguazone, Pentostatin, Semustine, Teniposide, and Vindesine sulfate.

In addition, therapeutic compositions of the invention may be used for prophylactic treatment of cancer. There are hereditary conditions and/or environmental situations (e.g. exposure to carcinogens) known in the art that predispose an individual to developing cancers. Under these circumstances, it may be beneficial to treat these individuals with therapeutically effective doses of the antibodies or antigen-binding fragments of the invention to reduce the risk of developing cancers.

*In vitro* models can be used to determine the effective doses of the antibody or antigen-binding fragment thereofs of the invention as a potential cancer treatment. These *in vitro* models include proliferation assays of cultured tumour cells, growth of cultured tumour cells in soft agar (see Freshney, (1987) Culture of Animal Cells: A Manual of Basic Technique, Wily-Liss, New York, NY Ch 18 and Ch 21), tumour systems in nude mice as described in Giovanella et al., J. Natl. Can. Inst., 52: 921-30 (1974), mobility and invasive potential of tumour cells in Boyden Chamber assays as described in Pilkington et al., Anticancer Res., 17: 4107-9 (1997), and angiogenesis assays such as induction of vascularization of the chick chorioallantoic membrane or induction of vascular endothelial cell migration as described in Ribatta et al., Intl. J. Dev. Biol., 40: 1189-97 (1999) and Li et al., Clin. Exp. Metastasis, 17:423-9 (1999), respectively. Suitable tumour cells lines are available, e.g. from American Type Tissue Culture Collection catalogues.

In one embodiment, the cancer to be treated is a Hematological neoplasm

Hematological neoplasms affect blood, bone marrow, and lymph nodes. As the three are intimately connected through the immune system, a disease affecting one of the three will often affect the others as well: although lymphoma is technically a disease of the lymph nodes, it often spreads to the bone marrow, affecting the blood and occasionally producing a paraprotein.

Hematological malignancies may derive from either of the two major blood cell lineages: myeloid and lymphoid cell lines. The myeloid cell line normally produces granulocytes, erythrocytes, thrombocytes, macrophages and mast cells; the lymphoid cell line produces B, T, NK and plasma cells. Lymphomas, lymphocytic leukemias, and myeloma are from the lymphoid line, while acute and chronic myelogenous leukemia, myelodysplastic syndromes and myeloproliferative diseases are myeloid in origin.

In one embodiment, the cancer to be treated is a Lymphoproliferative disorder (LPD)

Lymphoproliferative disorders (LPDs) refer to several conditions in which lymphocytes are produced in excessive quantities. They typically occur in patients who have compromised immune systems.

### Examples of LPDs include

- Follicular lymphoma
- Chronic lymphocytic leukemia
- Acute lymphoblastic leukemia
- Hairy cell leukemia
- Lymphomas
- Multiple myeloma
- Waldenstrom's macroglobulinemia
- Wiskott-Aldrich syndrome
- Post-transplant lymphoproliferative disorder
- Autoimmune lymphoproliferative syndrome (ALPS)
- Systemic lupus erythematusus (SLE)

In one embodiment, the cancer to be treated is a lymphoma or a non-Hodgkin's lymphoma (NHL).

Lymphoma is a cancer that begins in the lymphatic cells of the immune system and presents as a solid tumor of lymphoid cells. These malignant cells often originate in lymph nodes, presenting as an enlargement of the node (a tumor). Lymphomas are closely related to lymphoid leukemias, which also originate in lymphocytes but typically involve only circulating blood and the bone marrow (where blood cells are generated in a process termed haematopoiesis) and do not usually form static tumors. There are many types of lymphomas, and in turn, lymphomas are a part of the broad group of diseases called hematological neoplasms.

In one embodiment, the cancer to be treated is a plasma cell disorder (also known as plasma cell dyscrasias).

Cancers can take the form of the disorders (plasma cell dyscrasias). Plasma cell dyscrasias are produced as a result of malignant proliferation of a monoclonal population of plasma cells that may or may not secrete detectable levels of a monoclonal immunoglobulin or paraprotein commonly referred to as M protein. Common plasma cell dyscrasias include monoclonal gammopathy of undetermined significance (MGUS), multiple myeloma, solitary plasmacytoma of bone, extramedullary plasmacytoma, Waldenstrom's macroglobulinemia (WM), primary amyloidosis, and heavy-chain disease.

In a further embodiment, the cancer to be treated is multiple myeloma.

By 'treatment' we include both therapeutic and prophylactic treatment of a subject/patient. The term 'prophylactic' is used to encompass the use of a polypeptide or composition described herein which either prevents or reduces the likelihood of the occurrence or development of cancer (such as multiple myeloma) in a patient or subject.

A 'therapeutically effective amount', or 'effective amount', or 'therapeutically effective', as used herein, refers to that amount which provides a therapeutic effect for a given condition and administration regimen. This is a predetermined quantity of active material calculated to produce a desired therapeutic effect in association with the required additive and diluent, *i.e.* a carrier or administration vehicle. Further, it is intended to mean an amount sufficient to reduce or prevent a clinically significant deficit in the activity, function and response of the host. Alternatively, a therapeutically effective amount is sufficient to cause an improvement in a clinically significant condition in a host.

In a preferred embodiment, the uses of the first and second aspect of the invention and the method of the third aspect, comprise the step of administering to a patient in need thereof an amount of between about 0.02mg/kg to 20mg/kg of the antibody, antigen-binding fragment, variant, fusion or derivative thereof.

In a particularly preferred embodiment, the amount of the antibody, antigen-binding fragment, variant, fusion or derivative administered to a patient is approximately between: 0.02mg/kg to 0.10mg/kg; or 0.10mg to 0.20mg/kg; or 0.20mg to 0.30mg/kg; or 0.30mg to 0.40mg/kg; or 0.40mg to 0.50mg/kg; or 0.50mg to 0.60mg/kg; or 0.60mg to 0.70mg/kg; or 0.70mg to 0.80mg/kg; or 0.80mg to 0.90mg/kg; or 0.90mg to 1.00mg/kg; or 1.00mg to 1.10mg/kg; or 1.10mg to 1.20mg/kg; or 1.20mg to 1.30mg/kg; or 1.30mg to 1.40mg/kg; or 1.40mg to 1.50mg/kg; or 1.50mg to 1.60mg/kg; or 1.60mg to 1.70mg/kg; or 1.70mg to 1.80mg/kg; or 1.80mg to 1.90mg/kg; or 1.90mg to 2.00mg/kg; or 2.00mg/kg to 2.10mg/kg; or 2.10mg to 2.20mg/kg; or 2.20mg to 2.30mg/kg; or 2.30mg to 2.40mg/kg; or 2.40mg to 2.50mg/kg; or 2.50mg to 2.60mg/kg; or 2.60mg to 2.70mg/kg; or 2.70mg to 2.80mg/kg; or 2.80mg to 2.90mg/kg; or 2.90mg to 3.00mg/kg; or 3.00mg to 3.10mg/kg; or 3.10mg to 3.20mg/kg; or 3.20mg to 3.30mg/kg; or 3.30mg to 3.40mg/kg; or 3.40mg to 3.50mg/kg; or 3.50mg to 3.60mg/kg; or 3.60mg to 3.70mg/kg; or 3.70mg to 3.80mg/kg; or 3.80mg to 3.90mg/kg; or 3.90mg to 4.00mg/kg; or 4.00mg to 4.10mg/kg; or 4.10mg to 4.20mg/kg; or 4.20mg to 4.30mg/kg; or 4.30mg to 4.40mg/kg; or 4.40mg to 4.50mg/kg; or 4.50mg to 4.60mg/kg; or 4.60mg to 4.70mg/kg; or 4.70mg to 4.80mg/kg; or 4.80mg to 4.90mg/kg; or 4.90mg to 5.00mg/kg; or 5.00mg/kg to 6.00mg/kg; or 6.00mg to 7.00mg/kg; or 7.00mg to 8.00mg/kg; or 8.00mg to 9.00mg/kg; or 9.00mg to 10.00mg/kg; or 10.00mg to 11.00mg/kg; or 11.00mg to 12.00mg/kg; or 12.00mg to 13.00mg/kg; or 13.00mg to 14.00mg/kg; or 14.00mg to 15.00mg/kg; or 15.00mg to 16.00mg/kg; or 16.00mg to 17.00mg/kg; or 17.00mg to 18.00mg/kg; or 18.00mg to 19.00mg/kg; or 19.00mg to 20.00mg/kg.

In an alternative embodiment, the amount of the antibody, antigen-binding fragment, variant, fusion or derivative administered to a patient is approximately: 0.02mg/kg; or 0.03mg/kg; or 0.04mg/kg; or 0.05mg/kg; or 0.06mg/kg; or 0.07mg/kg; or 0.08mg/kg; or 0.09mg/kg; or 0.10mg/kg; or 0.15mg/kg; or 0.20mg/kg; or 0.25mg/kg; or 0.30mg/kg; or 0.35mg/kg; or 0.40mg/kg; or 0.45mg/kg; or 0.50mg/kg; or 0.60mg/kg; or 0.70mg/kg; or 0.80mg/kg; or 0.90mg/kg; or 1.00mg/kg; or 1.10mg/kg; or 1.20mg/kg; or 1.30mg/kg; or 1.40mg/kg; or 1.50mg/kg; or 1.60mg/kg; or 1.70mg/kg; or 1.80mg/kg; or 1.90mg/kg; or 2.00mg/kg; or 2.10mg/kg; or 2.20mg/kg; or 2.30mg/kg; or 2.40mg/kg; or 2.50mg/kg; or 2.60mg/kg; or 2.70mg/kg; or 2.80mg/kg; or 2.90mg/kg; or 3.00mg/kg; or 3.10mg/kg; or 3.20mg/kg; or 3.30mg/kg; or 3.40mg/kg; or 3.50mg/kg; or 3.60mg/kg; or 3.70mg/kg; or 3.80mg/kg; or 3.90mg/kg; or 4.00mg/kg; or 4.10mg/kg; or 4.20mg/kg; or 4.30mg/kg; or 4.40mg/kg; or 4.50mg/kg; or 4.60mg/kg; or 4.70mg/kg; or 4.80mg/kg; or 4.90mg/kg; or 5.00mg/kg; or 6.00mg/kg; or 7.00mg/kg; or 8.00mg/kg; or 9.00mg/kg; or 10.00mg/kg; or 11.00mg/kg; or 12.00mg/kg; or 13.00mg/kg; or 14.00mg/kg; or 15.00mg/kg; or 16.00mg/kg; or 17.00mg/kg; or 18.00mg/kg; or 19.00mg/kg; or 20.00mg/kg.

As will be appreciated by those skilled in the art, treatment with antibodies can offer therapeutic advantages with low toxicity in their ability to target cancerous cells and sparing surrounding tissues. The tolerability may reflect the dynamic actions of immunoglobulins, utilizing physiological mechanisms such as natural killer (NK)-cell mediated cell-death or directly inducing apoptosis rather than necrosis of tumour cells.

As is appreciated by those skilled in the art, the precise amount of a antibody or antigen-binding fragment thereof may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, *etc.,* as is well known in the art.

In a further aspect of the invention there is provided a kit of parts comprising:
(a) a composition as defined in the first aspect of the invention.
(b) apparatus for administering the composition; and
(c) intructions for use.

In one embodiment of the invention, components (i) and (ii) of the compositon of the kit are administered together.

In a further embodiment of the invention, components (i) and (ii) of the compositon of the kit are administered serperately.

Also disclosed herein, the further anticancer agent of the composition of the kit of parts is selected from Bortezomib, Dexamethasone, Revlimid and Alkeran.

In a further embodiment, the composition, system, use, antibody or method of any of the preceding aspects of the invention or otherwise disclosed herein comprises ICAM-1 localised on the surface of a plasma cell.

In a further embodiment, the composition, system, use, antibody or method of any of the preceding aspects of the invention or otherwise disclosed herein comprises an antibody or antigen-binding fragment, or variant, fusion or derivative thereof, capable of specifically binding ICAM-1 localised on the surface of a cell and inducing programmed cells death or apoptosis of that cell.

In a further embodiment, the composition, system, use, antibody or method of any of the preceding aspects of the invention or otherwise disclosed herein comprises the effective amount of the antibody, antigen-binding fragment, variant, fusion or derivative thereof being between about 0.1µg to 5g of the antibody, antigen-binding fragment, variant, fusion or derivative thereof.

In a particularly preferred embodiment the effective amount of the antibody, antigen-binding fragment, variant, fusion or derivative thereof is approximately:
0.10µg; or 0.15µg; or 0.20µg; or 0.25µg; or 0.30µg; or 0.35µg; or 0.40µg; or 0.45µg; or 0.50µg; or 0.60µg; or 0.70µg; or 0.80µg; or 0.90µg; or 1.00µg; or 1.10µg; or 1.20µg; or 1.30µg; or 1.40µg; or 150µg; or 1.60µg; or 1.70µg; or 1.80µg; or 1.90µg; or 2.00µg; or 2.10µg; or 2.20µg; or 2.30µg; or 2.40µg; or 2.50µg; or 2.60µg; or 2.70µg; or 2.80µg; or 2.90µg; or 3.00µg; or 3.10µg; or 320µg; or 3.30µg; or 3.40µg; or 3.50µg; or 3.60µg; or 3.70µg; or 3.80µg; or 3.90µg; or 4.00µg; or 4.10µg; or 4.20µg; or 4.30µg; or 4.40µg; or 4.50µg; or 4.60µg; or 4.70µg; or 4.80µg; or 4.90µg; or 5.00µg; or 6.00µg; or 7.00µg; or 8.00µg; or 9.00µg; or 10.00µg; or 11.00µg; or 12.00µg; or 13.00µg; or 14.00µg; or 15.00µg; or 16.00µg; or 17.00µg; or 18.00µg; or 19.00µg; or 20.00µg; or 21.00µg; or 22.00µg; or 23.00µg; or 24.00µg; or 25.00µg; or 26.00µg; or 27.00µg; or 28.00µg; or 29.00µg; or 30.00µg; or 31.00µg; or 32.00µg; or 33.00µg; or 34.00µg; or 35.00µg; or 36.00µg; or 37.00µg; or 38.00µg; or 39.00µg; or 40.00µg; or 41.00µg; or 42.00µg; or 43.00µg; or 44.00µg; or 45.00µg; or 46.00µg; or 47.00µg; or 48.00µg; or 49.00µg; or 50.00µg; or 51.00µg; or 52.00µg; or 53.00µg; or 54.00µg; or 55.00µg; or 56.00µg; or 57.00µg; or 58.00µg; or 59.00µg; or 60.00µg; or 61.00µg; or 62.00µg; or 63.00µg; or 64.00µg; or 65.00µg; or 66.00µg; or 67.00µg; or 68.00µg; or 69.00µg; or 70.00µg; or 71.00µg; or 72.00µg; or 73.00µg; or 74.00µg; or 75.00µg; or 76.00µg; or 77.00µg; or 78.00µg; or 79.00µg; or 80.00µg; or 81.00µg; or 82.00µg; or 83.00µg; or 84.00µg; or 85.00µg; or 86.00µg; or 87.00µg; or 88.00µg; or 89.00µg; or 90.00µg; or 91.00µg; or 92.00µg; or 93.00µg; or 94.00µg; or 95.00µg; or 96.00µg; or 97.00µg; or 98.00µg; or 99.00µg; or 100.00µg (0.10 mg); or 0.15mg; or 0.20mg; or 0.25mg; or 0.30mg; or 0.35mg; or 0.40mg; or 0.45mg; or 0.50mg; or 0.60mg; or 0.70mg; or 0.80mg; or 0.90mg; or 1.00mg; or 1.10mg; or 1.20mg; or 1.30mg; or 1.40mg; or 1.50mg; or 1.60mg; or 1.70mg; or 1.80mg; or 1.90mg; or 2.00mg; or 2.10mg; or 2.20mg; or 2.30mg; or 2.40mg; or 2.50mg; or 2.60mg; or 2.70mg; or 2.80mg; or 2.90mg; or 3.00mg; or 3.10mg; or 3.20mg; or 3.30mg; or 3.40mg; or 3.50mg; or 3.60mg; or 3.70mg; or 3.80mg; or 3.90mg; or 4.00mg; or 4.10mg; or 4.20mg; or 4.30mg; or 4.40mg; or 4.50mg; or 4.60mg; or 4.70mg; or 4.80mg; or 4.90mg; or 5.00mg; or 6.00mg; or 7.00mg; or 8.00mg; or 9.00mg; or 10.00mg; or 11.00mg; or 12.00mg; or 13.00mg; or 14.00mg; or 15.00mg; or 16.00mg; or 17.00mg; or 18.00mg; or 19.00mg; or 20.00mg; or 21.00mg; or 22.00mg; or 23.00mg; or 24.00mg; or 25.00mg; or 26.00mg; or 27.00mg; or 28.00mg; or 29.00mg; or 30.00mg; or 31.00mg; or 32.00mg; or 33.00mg; or 34.00mg; or 35.00mg; or 36.00mg; or 37.00mg; or 38.00mg; or 39.00mg; or 40.00mg; or 41.00mg; or 42.00mg; or 43.00mg; or 44.00mg; or 45.00mg; or 46.00mg; or 47.00mg; or 48.00mg; or 49.00mg; or 50.00mg; or 51.00mg; or 52.00mg; or 53.00mg; or 54.00mg; or 55.00mg; or 56.00mg; or 57.00mg; or 58.00mg; or 59.00mg; or 60.00mg; or 61.00mg; or 62.00mg; or 63.00mg; or 64.00mg; or 65.00mg; or 66.00mg; or 67.00mg; or 68.00mg; or 69.00mg; or 70.00mg; or 71.00mg; or 72.00mg; or 73.00mg; or 74.00mg; or 75.00mg; or 76.00mg; or 77.00mg; or 78.00mg; or 79.00mg; or 80.00mg; or 81.00mg; or 82.00mg; or 83.00mg; or 84.00mg; or 85.00mg; or 86.00mg; or 87.00mg; or 88.00mg; or 89.00mg; or 90.00mg; or 91.00mg; or 92.00mg; or 93.00mg; or 94.00mg; or 95.00mg; or 96.00mg; or 97.00mg; or 98.00mg; or 99.00mg; or 100.0Omg (0.10 g); or 0.15g; or 0.20g; or 0.25g; or 0.30g; or 0.35g; or 0.40g; or 0.45g; or 0.50g; or 0.60g; or 0.70g; or 0.80g; or 0.90g; or 1.00g; or 1.10g; or 1.20g; or 1.30g; or 1.40g; or 1.50g; or 1.60g; or 1.70g; or 1.80g; or 1.90g; or 2.00g; or 2.10g; or 2.20g; or 2.30g; or 2.40g; or 2.50g; or 2.60g; or 2.70g; or 2.80g; or 2.90g; or 3.00g; or 3.10g; or 3.20g; or 3.30g; or 3.40g; or 3.50g; or 3.60g; or 3.70g; or 3.80g; or 3.90g; or 4.00g; or 4.10g; or 4.20g; or 4.30g; or 4.40g; or 4.50g; or 4.60g; or 4.70g; or 4.80g; or 4.90g; or 5.00g

In a particularly preferred embodiment the effective amount of the antibody, antigen-binding fragment, variant, fusion or derivative thereof is approximately between:
0.10µg to 0.20µg; or 0.20µg to 0.30µg; or 0.30µg to 0.40µg; or 0.40µg to 0.50µg; or 0.50µg to 0.60µg; or 0.60µg to 0.70µg; or 0.70µg to 0.80µg; or 0.80µg to 0.90µg; or 0.90µg to 1.00µg; or 1.00µg to 1.10µg; or 1.10µg to 1.20µg; or 1.20µg to 1.30µg; or 1.30µg to 1.40µg; or 1.40µg to 1.50µg; or 1.50µg to 1.60µg; or 1.60µg to 1.70µg; or 1.70µg to 1.80µg; or 1.80µg to 1.90µg; or 1.90µg to 2.00µg; or 2.00µg to 2.10µg; or 2.10µg to 2.20µg; or 2.20µg to 2.30µg; or 2.30µg to 2.40µg; or 2.40µg to 2.50µg; or 2.50µg to 2.60µg; or 2.60µg to 2.70µg; or 2.70µg to 2.80µg; or 2.80µg to 2.90µg; or 2.90µg to 3.00µg; or 3.00µg to 3.10µg; or 3.10µg to 3.20µg; or 3.20µg to 3.30µg; or 3.30µg to 3.40µg; or 3.40µg to 3.50µg; or 3.50µg to 3.60µg; or 3.60µg to 3.70µg; or 3.70µg to 3.80µg; or 3.80µg to 3.90µg; or 3.90µg to 4.00µg; or 4.00µg to 4.10µg; or 4.10µg to 4.20µg; or 4.20µg to 4.30µg; or 4.30µg to 4.40µg; or 4.40µg to 4.50µg; or 4.50µg to 4.60µg; or 4.60µg to 4.70µg; or 4.70µg to 4.80µg; or 4.80µg to 4.90µg; or 4.90µg to 5.00µg; or 5.00µg to 6.00µg; or 6.00µg to 7.00µg; or 7.00µg to 8.00µg; or 8.00µg to 9.00µg; or 9.00µg to 10.00µg; or 10.00µg to 11.00µg; or 11.00µg to 12.00µg; or 12.00µg to 13.00µg; or 13.00µg to 14.00µg; or 14.00µg to 15.00µg; or 15.00µg to 16.00µg; or 16.00µg to 17.00µg; or 17.00µg to 18.00µg; or 18.00µg to 19.00µg; or 19.00µg to 20.00µg; or 20.00µg to 21.00µg; or 21.00µg to 22.00µg; or 22.00µg to 23.00µg; or 23.00µg to 24.00µg; or 24.00µg to 25.00µg; or 25.00µg to 26.00µg; or 26.00µg to 27.00µg; or 27.00µg to 28.00µg; or 28.00µg to 29.00µg; or 29.00µg to 30.00µg; or 30.00µg to 31.00µg; or 31.00µg to 32.00µg; or 32.00µg to 33.00µg; or 33.00µg to 34.00µg; or 34.00µg to 35.00µg; or 35.00µg to 36.00µg; or 36.00µg to 37.00µg; or 37.00µg to 38.00µg; or 38.00µg to 39.00µg; or 39.00µg to 40.00µg; or 40.00µg to 41.00µg; or 41.00µg to 42.00µg; or 42.00µg to 43.00µg; or 43.00µg to 44.00µg; or 44.00µg to 45.00µg; or 45.00µg to 46.00µg; or 46.00µg to 47.00µg; or 47.00µg to 48.00µg; or 48.00µg to 49.00µg; or 49.00µg to 50.00µg; or 50.00µg to 51.00µg; or 51.00µg to 52.00µg; or 52.00µg to 53.00µg; or 53.00µg to 54.00µg; or 54.00µg to 55.00µg; or 55.00µg to 56.00µg; or 56.00µg to 57.00µg; or 57.00µg to 58.00µg; or 58.00µg to 59.00µg; or 59.00µg to 60.00µg; or 60.00µg to 61.00µg; or 61.00µg to 62.00µg; or 62.00µg to 63.00µg; or 63.00µg to 64.00µg; or 64.00µg to 65.00µg; or 65.00µg to 66.00µg; or 66.00µg to 67.00µg; or 67.00µg to 68.00µg; or 68.00µg to 69.00µg; or 69.00µg to 70.00µg; or 70.00µg to 71.00µg; or 71.00µg to 72.00µg; or 72.00µg to 73.00µg; or 73.00µg to 74.00µg; or 74.00µg to 75.00µg; or 75.00µg to 76.00µg; or 76.00µg to 77.00µg; or 77.00µg to 78.00µg; or 78.00µg to 79.00µg; or 79.00µg to 80.00µg; or 80.00µg to 81.00µg; or 81.00µg to 82.00µg; or 82.00µg to 83.00µg; or 83.00µg to 84.00µg; or 84.00µg to 85.00µg; or 85.00µg to 86.00µg; or 86.00µg to 87.00µg; or 87.00µg to 88.00µg; or 88.00µg to 89.00µg; or 89.00µg to 90.00µg; or 90.00µg to 91.00µg; or 91.00µg to 92.00µg; or 92.00µg to 93.00µg; or 93.00µg to 94.00µg; or 94.00µg to 95.00µg; or 95.00µg to 96.00µg; or 96.00µg to 97.00µg; or 97.00µg to 98.00µg; or 98.00µg to 99.00µg; or 99.00µg to 100.00µg; or 100.00µg (0.10mg) to 0.20mg; or 0.20mg to 0.30mg; or 0.30mg to 0.40mg; or 0.40mg to 0.50mg; or 0.50mg to 0.60mg; or 0.60mg to 0.70mg; or 0.70mg to 0.80mg; or 0.80mg to 0.90mg; or 0.90mg to 1.00mg; or 1.00mg to 1.10mg; or 1.10mg to 1.20mg; or 1.20mg to 1.30mg; or 1.30mg to 1.40mg; or 1.40mg to 1.50mg; or 1.50mg to 1.60mg; or 1.60mg to 1.70mg; or 1.70mg to 1.80mg; or 1.80mg to 1.90mg; or 1.90mg to 2.00mg; or 2.00mg to 2.10mg; or 2.10mg to 2.20mg; or 2.20mg to 2.30mg; or 2.30mg to 2.40mg; or 2.40mg to 2.50mg; or 2.50mg to 2.60mg; or 2.60mg to 2.70mg; or 2.70mg to 2.80mg; or 2.80mg to 2.90mg; or 2.90mg to 3.00mg; or 3.00mg to 3.10mg; or 3.10mg to 3.20mg; or 3.20mg to 3.30mg; or 3.30mg to 3.40mg; or 3.40mg to 3.50mg; or 3.50mg to 3.60mg; or 3.60mg to 3.70mg; or 3.70mg to 3.80mg; or 3.80mg to 3.90mg; or 3.90mg to 4.00mg; or 4.00mg to 4.10mg; or 4.10mg to 4.20mg; or 4.20mg to 4.30mg; or 4.30mg to 4.40mg; or 4.40mg to 4.50mg; or 4.50mg to 4.60mg; or 4.60mg to 4.70mg; or 4.70mg to 4.80mg; or 4.80mg to 4.90mg; or 4.90mg to 5.00mg; or 5.00mg to 6.00mg; or 6.00mg to 7.00mg; or 7.00mg to 8.00mg; or 8.00mg to 9.00mg; or 9.00mg to 10.00mg; or 10.00mg to 11.00mg; or 11.00mg to 12.00mg; or 12.00mg to 13.00mg; or 13.00mg to 14.00mg; or 14.00mg to 15.00mg; or 15.00mg to 16.00mg; or 16.00mg to 17.00mg; or 17.00mg to 18.00mg; or 18.00mg to 19.00mg; or 19.00mg to 20.00mg; or 20.00mg to 21.00mg; or 21.00mg to 22.00mg; or 22.00mg to23.00mg; or 23.00mg to 24.00mg; or 24.00mg to 25.00mg; or 25.00mg to 26.00mg; or 26.00mg to 27.00mg; or 27.00mg to 28.00mg; or 28.00mg to 29.00mg; or 29.00mg to 30.00mg; or 30.00mg to 31.00mg; or 31.00mg to 32.00mg; or 32.00mg to 33.00mg; or 33.00mg to 34.00mg; or 34.00mg to 35.00mg; or 35.00mg to 36.00mg; or 36.00mg to 37.00mg; or 37.00mg to 38.00mg; or 38.00mg to 39.00mg; or 39.00mg to 40.00mg; or 40.00mg to 41.00mg; or 41.00mg to 42.00mg; or 42.00mg to 43.00mg; or 43.00mg to 44.00mg; or 44.00mg to 45.00mg; or 45.00mg to 46.00mg; or 46.00mg to 47.00mg; or 47.00mg to 48.00mg; or 48.00mg to 49.00mg; or 49.00mg to 50.00mg; or 50.00mg to 51.00mg; or 51.00mg to 52.00mg; or 52.00mg to 53.00mg; or 53.00mg to 54.00mg; or 54.00mg to 55.00mg; or 55.00mg to 56.00mg; or 56.00mg to 57.00mg; or 57.00mg to 58.00mg; or 58.00mg to 59.00mg; or 59.00mg to 60.00mg; or 60.00mg to 61.00mg; or 61.00mg to 62.00mg; or 62.00mg to 63.00mg; or 63.00mg to 64.00mg; or 64.00mg to 65.00mg; or 65.00mg to 66.00mg; or 66.00mg to 67.00mg; or 67.00mg to 68.00mg; or 68.00mg to 69.00mg; or 69.00mg to 70.00mg; or 70.00mg to 71.00mg; or 71.00mg to 72.00mg; or 72.00mg to 73.00mg; or 73.00mg to 74.00mg; or 74.00mg to 75.00mg; or 75.00mg to 76.00mg; or 76.00mg to 77.00mg; or 77.00mg to 78.00mg; or 78.00mg to 79.00mg; or 79.00mg to 80.00mg; or 80.00mg to 81.00mg; or 81.00mg to 82.00mg; or 82.00mg to 83.00mg; or 83.00mg to 84.00mg; or 84.00mg to 85.00mg; or 85.00mg to 86.00mg; or 86.00mg to 87.00mg; or 87.00mg to 88.00mg; or 88.00mg to 89.00mg; or 89.00mg to 90.00mg; or 90.00mg to 91.00mg; or 91.00mg to 92.00mg; or 92.00mg to 93.00mg; or 93.00mg to 94.00mg; or 94.00mg to 95.00mg; or 95.00mg to 96.00mg; or 96.00mg to 97.00mg; or 97.00mg to 98.00mg; or 98.00mg to 99.00mg; or 99.00mg to 100.00mg; or 100.00mg (0.10g) to 0.20g; or 0.20g to 0.30g; or 0.30g to 0.40g; or 0.40g to 0.50g; or 0.50g to 0.60g; or 0.60g to 0.70g; or 0.70g to 0.80g; or 0.80g to 0.90g; or 0.90g to 1.00g; or 1.00g to 1.10g; or 1.10g to 1.20g; or 1.20g to 1.30g; or 1.30g to 1.40g; or 1.40g to 1.50g; or 1.50g to 1.60g; or 1.60g to 1.70g; or 1.70g to 1.80g; or 1.80g to 1.90g; or 1.90g to 2.00g; or 2.00g to 2.10g; or 2.10g to 2.20g; or 2.20g to 2.30g; or 2.30g to 2.40g; or 2.40g to 2.50g; or 2.50g to 2.60g; or 2.60g to 2.70g; or 2.70g to 2.80g; or 2.80g to 2.90g; or 2.90g to 3.00g; or 3.00g to 3.10g; or 3.10g to 3.20g; or 3.20g to 3.30g; or 3.30g to 3.40g; or 3.40g to 3.50g; or 3.50g to 3.60g; or 3.60g to 3.70g; or 3.70g to 3.80g; or 3.80g to 3.90g; or 3.90g to 4.00g; or 4.00g to 4.10g; or 4.10g to 4.20g; or 4.20g to 4.30g; or 4.30g to 4.40g; or 4.40g to 4.50g; or 4.50g to 4.60g; or 4.60g to 4.70g; or 4.70g to 4.80g; or 4.80g to 4.90g; or 4.90g to 5.00g.

In a further embodiment, the antibody or antigen-binding fragment, or a variant, fusion or derivative thereof of the composition, system, use, antibody or method of any of the preceding aspects of the invention or otherwise disclosed herein, comprises or consists of an intact antibody.

By "antibody" we include substantially intact antibody molecules, as well as chimeric antibodies, humanised antibodies, human antibodies (wherein at least one amino acid is mutated relative to the naturally occurring human antibodies), single chain antibodies, bi-specific antibodies, antibody heavy chains, antibody light chains, homo-dimers and heterodimers of antibody heavy and/or light chains, and antigen binding fragments and derivatives of the same.

The term 'antibody' also includes all classes of antibodies, including IgG, IgA, IgM, IgD and IgE. Thus, the antibody may be an IgG molecule, such as an IgG1, IgG2, IgG3, or IgG4 molecule. Preferably, the antibody of the invention is an IgG molecule, or an antigen-binding fragment, or variant, fusion or derivative thereof.

In a further embodiment of the composition, system, use, antibody or method of any of the preceding aspects of the invention or otherwise disclosed herein, the antibody comprises or consists of an intact antibody. Alternatively, the antibody or antigen-binding fragment, or variant, fusion or derivative thereof, may consist essentially of an intact antibody. By "consist essentially of" we mean that the antibody or antigen-binding fragment, variant, fusion or derivative thereof consists of a portion of an intact antibody sufficient to display binding specificity for ICAM-1.

In a further embodiment of the composition, system, use, antibody or method of any of the preceding aspects of the invention or otherwise disclosed herein, the antibody is a non-naturally occurring antibody. Of course, where the antibody is a naturally occurring antibody, it is provided in an isolated form (*i.e.* distinct from that in which it is found in nature).

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent-parented antibody (Morrison et al (1984) Proc. Natl. Acad. Sci. USA 81, 6851-6855).

Antigenic specificity is conferred by variable domains and is independent of the constant domains, as known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

Thus, by "antigen-binding fragment" we mean a functional fragment of an antibody that is capable of binding to ICAM-1.

Exemplary antigen-binding fragments may be selected from the group consisting of Fv fragments (e.g. single chain Fv and disulphide-bonded Fv), and Fab-like fragments (e.g. Fab fragments, Fab' fragments and F(ab)₂ fragments).

In one embodiment, the antigen-binding fragment of the composition, system, use, antibody or method of any of the preceding aspects of the invention or otherwise disclosed herein is a single chain Fv (scFv) or a disulphide-bonded Fv.

Conveniently, the antigen-binding fragment is a Fab' fragment or a F(ab)₂

The advantages of using antibody fragments, rather than whole antibodies, are several-fold. The smaller size of the fragments may lead to improved pharmacological properties, such as better penetration of solid tissue. Moreover, antigen-binding fragments such as Fab, Fv, ScFv and dAb antibody fragments can be expressed in and secreted from E. *coli,* thus allowing the facile production of large amounts of the said fragments.

Also included within the scope of the invention are modified versions of antibodies and an antigen-binding fragments thereof, e.g. modified by the covalent attachment of polyethylene glycol or other suitable polymer.

Methods of generating antibodies and antibody fragments are well known in the art. For example, antibodies may be generated via any one of several methods which employ induction of *in vivo* production of antibody molecules, screening of immunoglobulin libraries (Orlandi. et al, 1989. Proc. Natl. Acad. Sci. U.S.A. 86:3833-3837; Winter et al., 1991, Nature 349:293-299) or generation of monoclonal antibody molecules by cell lines in culture. These include, but are not limited to, the hybridoma technique, the human B-cell hybridoma technique, and the Epstein-Barr virus (EBV)-hybridoma technique (Kohler et al., 1975. Nature 256:4950497; Kozbor et al., 1985. J. Immunol. Methods 81:31-42; Cote et al., 1983. Proc. Natl. Acad. Sci. USA 80:2026-2030; Cole et al., 1984. Mol. Cell. Biol. 62:109-120).

Conveniently, the invention provides an antibody or antigen-binding fragment, or a variant, fusion or derivative thereof, wherein the antibody is a recombinant antibody (*i.e.* wherein it is produced by recombinant means).

In a preferred embodiment of the compositions, systems, uses, antibodies or methods of the invention or otherwise disclosed herein, the antibody is a monoclonal antibody. Suitable monoclonal antibodies to selected antigens may be prepared by known techniques, for example those disclosed in *"*Monoclonal Antibodies: A manual of techniques", H Zola (CRC Press, 1988) and in *"*Monoclonal Hybridoma Antibodies: Techniques and Applications", J G R Hurrell (CRC Press, 1982).

Antibody fragments can also be obtained using methods well known in the art (see, for example, Harlow & Lane, 1988, "Antibodies: A Laboratory Manual", Cold Spring Harbor Laboratory, New York). For example, antibody fragments for use in the methods and uses of the present invention can be prepared by proteolytic hydrolysis of the antibody or by expression in *E. coli* or mammalian cells (e.g. Chinese hamster ovary cell culture or other protein expression systems) of DNA encoding the fragment. Alternatively, antibody fragments can be obtained by pepsin or papain digestion of whole antibodies by conventional methods.

Preferably, the invention, or other aspects disclosed herein, provide a composition, system, use, antibody or method wherein the antibody or antigen-binding fragment thereof is a human antibody or humanised antibody.

It will be appreciated by persons skilled in the art that for human therapy or diagnostics, humanised antibodies may be used. Humanised forms of non-human (e.g. murine) antibodies are genetically engineered chimeric antibodies or antibody fragments having minimal-portions derived from non-human antibodies. Humanised antibodies include antibodies in which complementary determining regions of a human antibody (recipient antibody) are replaced by residues from a complementary determining region of a non human species (donor antibody) such as mouse, rat or rabbit having the desired functionality. In some instances, Fv framework residues of the human antibody are replaced by corresponding non-human residues. Humanised antibodies may also comprise residues which are found neither in the recipient antibody nor in the imported complementarity determining region or framework sequences. In general, the humanised antibody will comprise substantially all of at least one, and typically two, variable domains, in which all or substantially all of the complementarity determining regions correspond to those of a non-human antibody and all, or substantially all, of the framework regions correspond to those of a relevant human consensus sequence. Humanised antibodies optimally also include at least a portion of an antibody constant region, such as an Fc region, typically derived from a human antibody (see, for example, Jones et al., 1986. Nature 321:522-525; Riechmann et al., 1988, Nature 332:323-329; Presta, 1992, Curr. Op. Struct. Biol. 2:593-596).

Methods for humanising non-human antibodies are well known in the art. Generally, the humanised antibody has one or more amino acid residues introduced into it from a source which is non-human. These non-human amino acid residues, often referred to as imported residues, are typically taken from an imported variable domain. Humanisation can be essentially performed as described (see, for example, Jones et al., 1986, Nature 321:522-525; Reichmann et al., 1988. Nature 332:323-327; Verhoeyen et al., 1988, Science 239:1534-1536I; US 4,816,567) by substituting human complementarity determining regions with corresponding rodent complementarity determining regions. Accordingly, such humanised antibodies are chimeric antibodies, wherein substantially less than an intact human variable domain has been substituted by the corresponding sequence from a non-human species. In practice, humanised antibodies may be typically human antibodies in which some complementarity determining region residues and possibly some framework residues are substituted by residues from analogous sites in rodent antibodies.

Human antibodies can also be identified using various techniques known in the art, including phage display libraries (see, for example, Hoogenboom & Winter, 1991, J. Mol. Biol. 227:381; Marks et al., 1991, J. Mol. Biol. 222:581; Cole et al., 1985, In: Monoclonal antibodies and Cancer Therapy, Alan R. Liss, pp. 77; Boerner et al., 1991. J. Immunol. 147:86-95, Soderlind et al., 2000, Nat Biotechnol 18:852-6 and WO 98/32845).

Once suitable antibodies are obtained, they may be tested for activity, such as binding specificity or a biological activity of the antibody, for example by ELISA, immunohistochemistry, flow cytometry, immunoprecipitation, Western blots, *etc.* The biological activity may be tested in different assays with readouts for that particular feature.

Conveniently, the antibody or antigen-binding fragment thereof of the invention comprises one or more of the following amino acid sequences (CDR regions):
FSNAWMSWVRQAPG and/or
AFIWYDGSNKYYADSVKGR and/or
ARYSGWYFDY and/or
CTGSSSNIGAGYDVH and/or
DNNNRPS and/or
CQSYDSSLSAWL

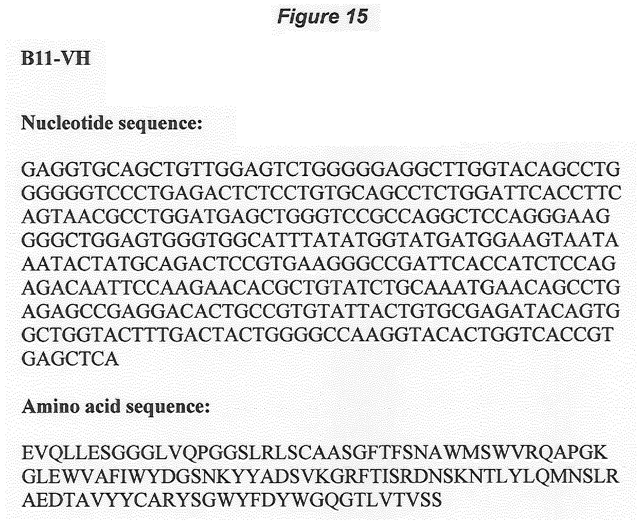

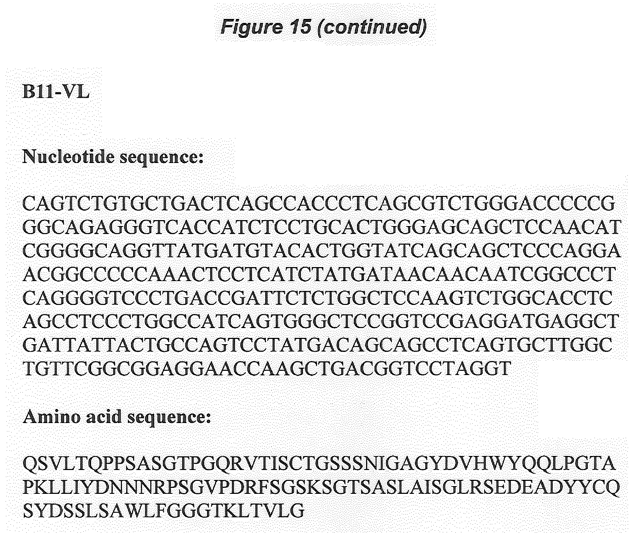

Alternatively, the antibody or antigen-binding fragment thereof of the invention comprises one or more of the variable regions shown in figure 15.

The term 'amino acid' as used herein includes the standard twenty genetically-encoded amino acids and their corresponding stereoisomers in the 'D' form (as compared to the natural 'L' form), omega-amino acids other naturally-occurring amino acids, unconventional amino acids (e.g. α,α-disubstituted amino acids, N-alkyl amino acids, *etc*.) and chemically derivatised amino acids (see below).

When an amino acid is being specifically enumerated, such as 'alanine' or 'Ala' or 'A', the term refers to both L-alanine and D-alanine unless explicitly stated otherwise. Other unconventional amino acids may also be suitable components for polypeptides of the present invention, as long as the desired functional property is retained by the polypeptide. For the peptides shown, each encoded amino acid residue, where appropriate, is represented by a single letter designation, corresponding to the trivial name of the conventional amino acid.

In one embodiment, the polypeptides as defined herein comprise or consist of L-amino acids.

It will be appreciated by persons skilled in the art that the methods and uses of the invention encompass variants, fusions and derivatives of the defined polypeptides, as well as fusions of a said variants or derivatives, provided such variants, fusions and derivatives have binding specificity for ICAM-1.

Variants may be made using the methods of protein engineering and site-directed mutagenesis well known in the art using the recombinant polynucleotides (see example, see Molecular Cloning: a Laboratory Manual, 3rd edition, Sambrook & Russell, 2001, Cold Spring Harbor Laboratory Press).

By 'fusion' of said polypeptide we include a polypeptide fused to any other polypeptide. For example, the said polypeptide may be fused to a polypeptide such as glutathione-S-transferase (GST) or protein A in order to facilitate purification of said polypeptide. Examples of such fusions are well known to those skilled in the art. Similarly, the said polypeptide may be fused to an oligo-histidine tag such as His6 or to an epitope recognised by an antibody such as the well-known Myc-tag epitope. Fusions to any variant or derivative of said polypeptide are also included in the scope of the invention. It will be appreciated that fusions (or variants or derivatives thereof) which retain desirable properties, such as have binding specificity for ICAM-1, are preferred.

The fusion may comprise a further portion which confers a desirable feature on the said polypeptide of the invention; for example, the portion may be useful in detecting or isolating the polypeptide, or promoting cellular uptake of the polypeptide. The portion may be, for example, a biotin moiety, a radioactive moiety, a fluorescent moiety, for example a small fluorophore or a green fluorescent protein (GFP) fluorophore, as well known to those skilled in the art. The moiety may be an immunogenic tag, for example a Myc-tag, as known to those skilled in the art or may be a lipophilic molecule or polypeptide domain that is capable of promoting cellular uptake of the polypeptide, as known to those skilled in the art.

By 'variants' of the polypeptide we include insertions, deletions and substitutions, either conservative or non-conservative. In particular we include variants of the polypeptide where such changes do not substantially alter the activity of the said polypeptide. In particular, we include variants of the polypeptide where such changes do not substantially alter the binding specificity for ICAM-1.

The polypeptide variant may have an amino acid sequence which has at least 75% identity with one or more of the amino acid sequences given above, for example at least 80%, at least 90%, at least 95%, at least 96%, at least 97%, at least 98%, or at least 99% identity with one or more of the amino acid sequences specified above.

The percent sequence identity between two polypeptides may be determined using suitable computer programs, for example the GAP program of the University of Wisconsin Genetic Computing Group and it will be appreciated that percent identity is calculated in relation to polypeptides whose sequences have been aligned optimally.

The alignment may alternatively be carried out using the Clustal W program (as described in Thompson et al., 1994, Nuc. Acid Res. 22:4673-4680).

The parameters used may be as follows:
- Fast pairwise alignment parameters: K-tuple(word) size; 1, window size; 5, gap penalty; 3, number of top diagonals; 5. Scoring method: x percent.
- Multiple alignment parameters: gap open penalty; 10, gap extension penalty; 0.05.
- Scoring matrix: BLOSUM.

Alternatively, the BESTFIT program may be used to determine local sequence alignments.

The polypeptide, variant, fusion or derivative used in the compositions, systems, uses, antibodies or methods of the invention or otherwise disclosed herein may comprise one or more amino acids which have been modified or derivatised.

Chemical derivatives of one or more amino acids may be achieved by reaction with a functional side group. Such derivatised molecules include, for example, those molecules in which free amino groups have been derivatised to form amine hydrochlorides, p-toluene sulphonyl groups, carboxybenzoxy groups, *t*-butyloxycarbonyl groups, chloroacetyl groups or formyl groups. Free carboxyl groups may be derivatised to form salts, methyl and ethyl esters or other types of esters and hydrazides. Free hydroxyl groups may be derivatised to form O-acyl or O-alkyl derivatives. Also included as chemical derivatives are those peptides which contain naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine and ornithine for lysine. Derivatives also include peptides containing one or more additions or deletions as long as the requisite activity is maintained. Other included modifications are amidation, amino terminal acylation (e.g. acetylation or thioglycolic acid amidation), terminal carboxylamidation (e.g. with ammonia or methylamine), and the like terminal modifications.

It will be further appreciated by persons skilled in the art that peptidomimetic compounds may also be useful. Thus, by 'polypeptide' we include peptidomimetic compounds which are capable of binding ICAM-1. The term 'peptidomimetic' refers to a compound that mimics the conformation and desirable features of a particular peptide as a therapeutic agent.

For example, the polypeptides of the invention include not only molecules in which amino acid residues are joined by peptide (-CO-NH-) linkages but also molecules in which the peptide bond is reversed. Such retro-inverso peptidomimetics may be made using methods known in the art, for example such as those described in Meziere et al. (1997) J. Immunol. 159, 3230-3237. This approach involves making pseudopeptides containing changes involving the backbone, and not the orientation of side chains. Retro-inverse peptides, which contain NH-CO bonds instead of CO-NH peptide bonds, are much more resistant to proteolysis. Alternatively, the polypeptide of the invention may be a peptidomimetic compound wherein one or more of the amino acid residues are linked by a -y(CH₂NH)- bond in place of the conventional amide linkage.

In a further alternative, the peptide bond may be dispensed with altogether provided that an appropriate linker moiety which retains the spacing between the carbon atoms of the amino acid residues is used; it may be advantageous for the linker moiety to have substantially the same charge distribution and substantially the same planarity as a peptide bond.

It will be appreciated that the polypeptide may conveniently be blocked at its N- or C-terminus so as to help reduce susceptibility to exoproteolytic digestion.

A variety of uncoded or modified amino acids such as D-amino acids and N-methyl amino acids have also been used to modify mammalian peptides. In addition, a presumed bioactive conformation may be stabilised by a covalent modification, such as cyclisation or by incorporation of lactam or other types of bridges, for example see Veber et al., 1978, Proc. Natl. Acad. Sci. USA 75:2636 and Thursell et al., 1983, Biochem. Biophys. Res. Comm. 111:166.

A common theme among many of the synthetic strategies has been the introduction of some cyclic moiety into a peptide-based framework. The cyclic moiety restricts the conformational space of the peptide structure and this frequently results in an increased specificity of the peptide for a particular biological receptor. An added advantage of this strategy is that the introduction of a cyclic moiety into a peptide may also result in the peptide having a diminished sensitivity to cellular peptidases.

Thus, exemplary polypeptides useful in the compositions, systems, uses, antibodies or methods of the invention or otherwise disclosed herein comprise terminal cysteine amino acids. Such a polypeptide may exist in a heterodetic cyclic form by disulphide bond formation of the mercaptide groups in the terminal cysteine amino acids or in a homodetic form by amide peptide bond formation between the terminal amino acids. As indicated above, cyclising small peptides through disulphide or amide bonds between the N- and C-terminus cysteines may circumvent problems of specificity and half-life sometime observed with linear peptides, by decreasing proteolysis and also increasing the rigidity of the structure, which may yield higher specificity compounds. Polypeptides cyclised by disulphide bonds have free amino and carboxy-termini which still may be susceptible to proteolytic degradation, while peptides cyclised by formation of an amide bond between the N-terminal amine and C-terminal carboxyl and hence no longer contain free amino or carboxy termini. Thus, the peptides of the present invention can be linked either by a C-N linkage or a disulphide linkage.

The present invention is not limited in any way by the method of cyclisation of peptides, but encompasses peptides whose cyclic structure may be achieved by any suitable method of synthesis. Thus, heterodetic linkages may include, but are not limited to formation via disulphide, alkylene or sulphide bridges. Methods of synthesis of cyclic homodetic peptides and cyclic heterodetic peptides, including disulphide, sulphide and alkylene bridges, are disclosed in US 5,643,872. Other examples of cyclisation methods are discussed and disclosed in US 6,008,058.

A further approach to the synthesis of cyclic stabilised peptidomimetic compounds is ring-closing metathesis (RCM). This method involves steps of synthesising a peptide precursor and contacting it with an RCM catalyst to yield a conformationally restricted peptide. Suitable peptide precursors may contain two or more unsaturated C-C bonds. The method may be carried out using solid-phase-peptide-synthesis techniques. In this embodiment, the precursor, which is anchored to a solid support, is contacted with a RCM catalyst and the product is then cleaved from the solid support to yield a conformationally restricted peptide.

Another approach, disclosed by D. H. Rich in Protease Inhibitors, Barrett and Selveson, eds., Elsevier (1986) has been to design peptide mimics through the application of the transition state analogue concept in enzyme inhibitor design. For example, it is known that the secondary alcohol of staline mimics the tetrahedral transition state of the scissile amide bond of the pepsin substrate.

In summary, terminal modifications are useful, as is well known, to reduce susceptibility by proteinase digestion and therefore to prolong the half-life of the peptides in solutions, particularly in biological fluids where proteases may be present. Polypeptide cyclisation is also a useful modification because of the stable structures formed by cyclisation and in view of the biological activities observed for cyclic peptides.

Thus, in one embodiment the polypeptide used in the compositions, systems, uses, antibodies or methods of the invention or otherwise disclosed herein is cyclic. However, in an alternative embodiment, the polypeptide is linear.

In a preferred embodiment of the composition, system, use, antibody or method of the invention or otherwise disclosed herein, the antibody or antigen-binding fragment, or variant, fusion or derivative thereof, is capable of specifically binding ICAM-1 localised on the surface of a cell and inhibiting and/or preventing proliferation of that cell.

In an alternative embodiment, the antibody or antigen-binding fragment, or a variant, fusion or derivative thereof, is capable of specifically binding ICAM-1 localised on the surface of a cell and inducing apoptosis of that cell.

In an alternative embodiment, the antibody or antigen-binding fragment, or variant, fusion or derivative thereof, is capable of specifically binding ICAM-1 localised on the surface of a cell and inducing antibody-dependent cell cytotoxicity against that cell.

The antibody, antigen-binding fragment, and/or fusion, derivative or variants thereof, and medicaments of the invention or otherwise disclosed herein may be delivered using an injectable sustained-release drug delivery system. These are designed specifically to reduce the frequency of injections. An example of such a system is Nutropin Depot which encapsulates recombinant human growth hormone (rhGH) in biodegradable microspheres that, once injected, release rhGH slowly over a sustained period. Preferably, delivery is performed intra-muscularly (i.m.) and/or sub-cutaneously (s.c.) and/or intravenously (i.v.).

The antibody, antigen-binding fragment, and/or fusion, derivative or variants thereof, and medicaments of the invention or otherwise disclosed herein can be administered by a surgically implanted device that releases the drug directly to the required site. For example, Vitrasert releases ganciclovir directly into the eye to treat CMV retinitis. The direct application of this toxic agent to the site of disease achieves effective therapy without the drug's significant systemic side-effects.

Electroporation therapy (EPT) systems can also be employed for the administration of the antibody, antigen-binding fragment, and/or fusion, derivative or variants thereof, medicaments and pharmaceutical compositions of the invention. A device which delivers a pulsed electric field to cells increases the permeability of the cell membranes to the drug, resulting in a significant enhancement of intracellular drug delivery.

The antibody, antigen-binding fragment, and/or fusion, derivative or variants thereof, and medicaments of the invention or otherwise disclosed herein can also be delivered by electro-incorporation (EI). EI occurs when small particles of up to 30 microns in diameter on the surface of the skin experience electrical pulses identical or similar to those used in electroporation. In EI, these particles are driven through the stratum corneum and into deeper layers of the skin. The particles can be loaded or coated with drugs or genes or can simply act as "bullets" that generate pores in the skin through which the drugs can enter.

An alternative method of delivery of the antibody or antigen-binding fragment thereof, and medicaments of the invention or otherwise disclosed herein is the ReGel® injectable system that is thermo-sensitive. Below body temperature, ReGel is an injectable liquid while at body temperature it immediately forms a gel reservoir that slowly erodes and dissolves into known, safe, biodegradable polymers. The active substance is delivered over time as the biopolymers dissolve.

The antibody, antigen-binding fragment, and/or fusion, derivative or variants thereof, and medicaments of the invention or otherwise disclosed herein can also be delivered orally. The process employs a natural process for oral uptake of vitamin B₁₂ and/or vitamin D in the body to co-deliver proteins and peptides. By riding the vitamin B₁₂ and/or vitamin D uptake system, the antibody, antigen-binding fragment, and/or fusion, derivative or variants thereof, and medicaments of the invention can move through the intestinal wall. Complexes are synthesised between vitamin B₁₂ analogues and/or vitamin D analogues and the drug that retain both significant affinity for intrinsic factor (IF) in the vitamin B₁₂ portion/vitamin D portion of the complex and significant bioactivity of the active substance of the complex.

The antibody, antigen-binding fragment, and/or fusion, derivative or variants thereof, and medicaments of the invention or otherwise disclosed herein can be introduced to cells by "Trojan peptides". These are a class of polypeptides called penetratins which have translocating properties and are capable of carrying hydrophilic compounds across the plasma membrane. This system allows direct targeting of oligopeptides to the cytoplasm and nucleus, and may be non-cell type specific and highly efficient. See Derossi et al. (1998), Trends Cell Biol 8, 84-87.

Preferably, the medicament of the present invention or otherwise disclosed herein is a unit dosage containing a daily dose or unit, daily sub-dose or an appropriate fraction thereof, of the active ingredient.

The antibody, antigen-binding fragment, and/or fusion, derivative or variants thereof and/or medicaments of the invention or otherwise disclosed herein will normally be administered orally or by any parenteral route, in the form of a pharmaceutical composition comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses.

In human therapy, the antibody, antigen-binding fragment, and/or fusion, derivative or variants thereof, and medicaments of the invention or otherwise disclosed herein can be administered alone but will generally be administered in admixture with a suitable pharmaceutical excipient, diluent or carrier selected with regard to the intended route of administration and standard pharmaceutical practice.

For example, the antibody, antigen-binding fragment, and/or fusion, derivative or variants thereof, and medicaments of the invention or otherwise disclosed herein can be administered orally, buccally or sublingually in the form of tablets, capsules, ovules, elixirs, solutions or suspensions, which may contain flavouring or colouring agents, for immediate-, delayed- or controlled-release applications. The antibody, antigen-binding fragment, and/or fusion, derivative or variants thereof, and medicaments of the invention or otherwise disclosed herein may also be administered via intracavernosal injection.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxy-propylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, cellulose, milk sugar or high molecular weight polyethylene glycols. For aqueous suspensions and/or elixirs, the antibody, antigen-binding fragment, and/or fusion, derivative or variants thereof, medicaments and pharmaceutical compositions of the invention may be combined with various sweetening or flavouring agents, colouring matter or dyes, with emulsifying and/or suspending agents and with diluents such as water, ethanol, propylene glycol and glycerin, and combinations thereof.

The antibody, antigen-binding fragment, and/or fusion, derivative or variants thereof, and medicaments of the invention or otherwise dislosed herein can also be administered parenterally, for example, intravenously, intra-arterially, intraperitoneally, intra-thecally, intraventricularly, intrasternally, intracranially, intra-muscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Medicaments and pharmaceutical compositions suitable for parenteral administration include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The medicaments and pharmaceutical compositions may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

The antibody, antigen-binding fragment, and/or fusion, derivative or variants thereof, and medicaments of the invention or otherwise disclosed herein can also be administered intranasally or by inhalation and are conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoro-ethane, a hydrofluoroalkane such as 1,1,1,2-tetrafluoroethane (HFA 134A3 or 1,1,1,2,3,3,3-heptafluoropropane (HFA 227EA3), carbon dioxide or other suitable gas. In the case of a pressurised aerosol, the dosage unit may be determined by providing a valve to deliver a metered amount. The pressurised container, pump, spray or nebuliser may contain a solution or suspension of the active agent, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of an antibody, antigen-binding fragment, and/or fusion, derivative or variants thereof, of the invention and a suitable powder base such as lactose or starch.

Aerosol or dry powder formulations are preferably arranged so that each metered dose or "puff" contains an effective amount of an agent or polynucleotide of the invention for delivery to the patient. It will be appreciated that he overall daily dose with an aerosol will vary from patient to patient, and may be administered in a single dose or, more usually, in divided doses throughout the day.

Alternatively, the antibody, antigen-binding fragment, and/or fusion, derivative or variants thereof, and medicaments of the invention or otherwise disclosed herein can be administered in the form of a suppository or pessary, or they may be applied topically in the form of a lotion, solution, cream, gel, ointment or dusting powder. The antibody, antigen-binding fragment, and/or fusion, derivative or variants thereof, and medicaments of the invention may also be transdermally administered, for example, by the use of a skin patch. They may also be administered by the ocular route, particularly for treating diseases of the eye.

For ophthalmic use, the antibody, antigen-binding fragment, and/or fusion, derivative or variants thereof, and medicaments of the invention or otherwise disclosed herein can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the antibody, antigen-binding fragment, and/or fusion, derivative or variants thereof, and medicaments of the invention or otherwise disclosed herein can be formulated as a suitable ointment containing the active agent suspended or dissolved in, for example, a mixture with one or more of the following: mineral oil, liquid petrolatum, white petrolatum, propylene glycol, polyoxyethylene polyoxypropylene agent, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Generally, in humans, oral or parenteral administration of the antibody, antigen-binding fragment, and/or fusion, derivative or variants thereof, medicaments and pharmaceutical compositions of the invention or otherwise disclosed herein is the preferred route, being the most convenient.

For veterinary use, the antibody, antigen-binding fragment, and/or fusion, derivative or variants thereof, and medicaments of the invention or otherwise disclosed herein are administered as a suitably acceptable formulation in accordance with normal veterinary practice and the veterinary surgeon will determine the dosing regimen and route of administration which will be most appropriate for a particular animal.

The antibody or antigen-binding fragment, or variant, fusion or derivative thereof, as defined herein may be formulated as described in the accompanying Examples.

According to a further embodiment, the effectiveness of antibody, antigen binding fragment, variant, fusion or derivative thereof according to the present invention or otherwise disclosed herein in alleviating the symptoms, preventing or treating disease may be improved by serial administering or administration in combination with another agent that is effective for the same clinical indication, such as another antibody or a fragment thereof directed against a different epitope than that of the antibody according to the invention, or one or more conventional therapeutic agents known for the intended therapeutic indication.

For example, the additional agent may be one or more agent selected from the group consisting or comprising of: Revlimid; Talibomib; Melphalan; Bortezomib; Velcade; cytostatic agents; Predison (a hormonal therapy) or similar hormonal drugs; tyrosine kinase inhibitors

As discussed above, when administered according to the compositions, systems, uses, and methods of the invention or otherwise disclosed herein, the antibody and/or antigen-binding fragment and/or variant, fusion or derivative as defined herein is capable of inducing apoptosis of, and/or directing antibody-dependent cell-mediated cytotoxicity (ADCC) against, cancer and/or tumour cells (such as CD20-positive and CD20-negative multiple myeloma cancer cells and tumours). In addition, the antibody and/or antigen-binding fragment and/or variant, fusion or derivative is capable of binding soluble intercellular adhesion molecule 1 (sICAM-1), thereby inhibiting angiogenesis, cell-adhesion mediated drug-resistance and tumour-cell-escape from immunosurveillance.

The accompanying Examples demonstrate that an exemplary antibody as defined herein (termed antibody "B11") has significant *in vivo* and *in vitro* anti-tumour activity when administered according to the methods and uses of the invention or otherwise disclosed herein. In addition to its significant direct anti-myeloma activity, B11 may also act to inhibit angiogenesis-driven tumour growth and counteract tumour escape from immunosurveillance.

As used herein, the singular forms "a", "and", and "the" include plural referents unless the context clearly dictates otherwise. Thus, for example, reference to "an antibody" includes a plurality of such antibodies and reference to "the dosage" includes reference to one or more dosages and equivalents thereof known to those skilled in the art, and so forth.

### EXAMPLES

The following examples embody various aspects of the invention. It will be appreciated that the specific antibodies used in the examples serve to illustrate the principles of the invention and are not intended to limit its scope.

The following examples are described with reference to the accompanying figures in which:

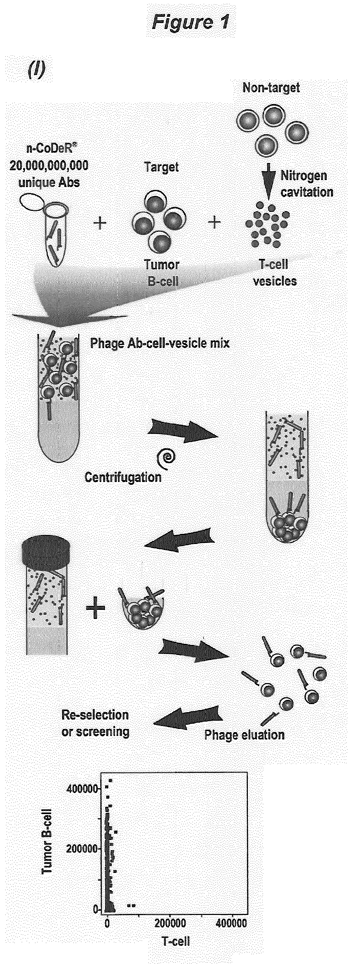

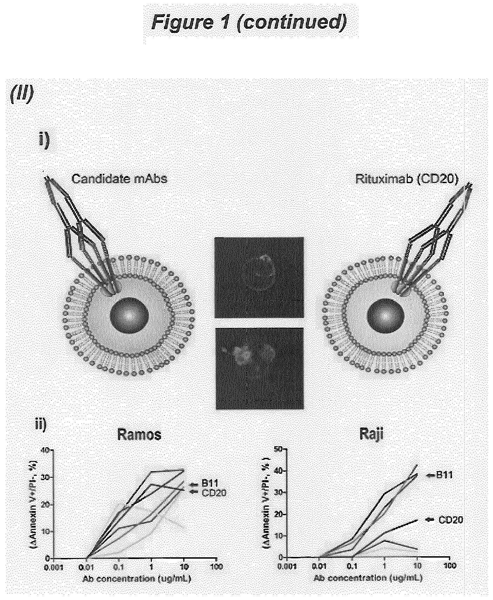

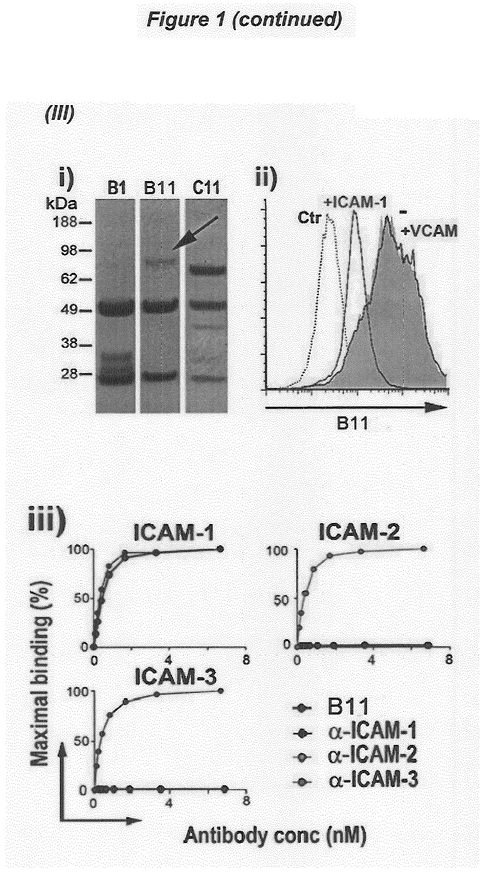

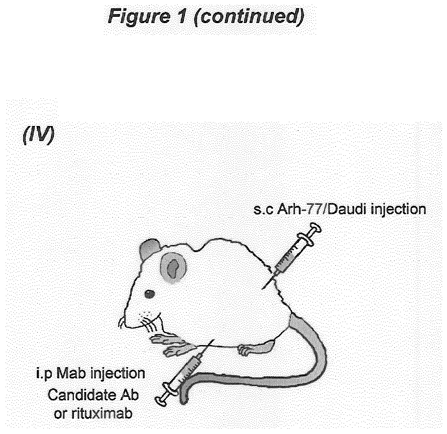

**Figure** 1 shows how a novel ICAM-1 antibody which has significant anti-tumor activity against CD20-expressing tumors *in vivo* is isolated by combined differential biopanning and programmed cell death screening. (I) Differential biopanning for antibodies specific for tumor associated receptors. (II) Programmed Cell Death (PCD) screening. (III) Target identification. (IV) *In vivo* anti-tumor activity.
   Figure 1 (I) shows how antibodies with selectivity for B lymphoma target cells were retrieved by using a competition sequential differential biopanning methodology, where target cell antigens in the form of whole cells along with excess subtractor cell antigens in the form of membrane vesicles were subjected in chorus to the naive n-CoDeR® antibody phage library.
   Figure 1 (II) shows how high-throughput programmed cell death screening was used to isolate multiple B cell lymphoma PCD-inducing antibodies. B-cell lymphoma cells were cultured in the presence of titrated concentrations of candidate antibodies and hyper-cross-linking for overnight, and apoptosis was monitored after combined staining with annexin V-AF488 and propidium iodide using flow cytometry. Figure 1 (II) (i) illustrates the membrane blebbing and cell membrane permeability to macromolecules typical of early apoptotic and late apoptotic cells, respectively, induced by functionally isolated B11 antibody. The graphs of figure 1 (II) (ii) presents the percentage of dead cells measured as annexin V-488-positivity.
   Figure 1 (III) shows how target identification was done on Raji or Ramos B lymphoma cells lysed and immunoprecipitated of the fully human IgG's, followed by crosslinking with protein A sepharose. Antibody-specific bands were excised and subjected to tryptic digestion and analyzed by MALDI-TOF. The single band precipitated by B11 was identified as ICAM-1. The established identity of ICAM-1 was confirmed by blocking studies by up to 50-fold molar excess soluble recombinant ICAM-1 or VCAM. The MFI of B11 to PC-3 cells was determined by flow cytometry. Dashed line represents the negative control antibody and gray solid histogram represents the MFI of B11 with no preblocking. B11 was preblocked by recombinant VCAM or ICAM-1. Figure 1 (III) (iii) shows how ELISA plates were coated with recombinant human ICAM-1, ICAM-2, or ICAM-3, and binding of B11 to ICAM-1 was detected using a luminescence protocol. Anti-ICAM-2 and α-ICAM-3 antibodies were used as positive controls to detect ICAM-2 and ICAM-3 respectively.
   Figure 1 (IV) shows how that, in order to further investigate the therapeutic potential of PCD-inducing ICAM-1 antibodies, the *in vivo* anti-tumor activity of B11 was evaluated in tumor models comprising immunodeficient *scid* mice transplanted with either of two well-characterized CD20-expressing tumor B cell lines ARH-77 or Daudi.

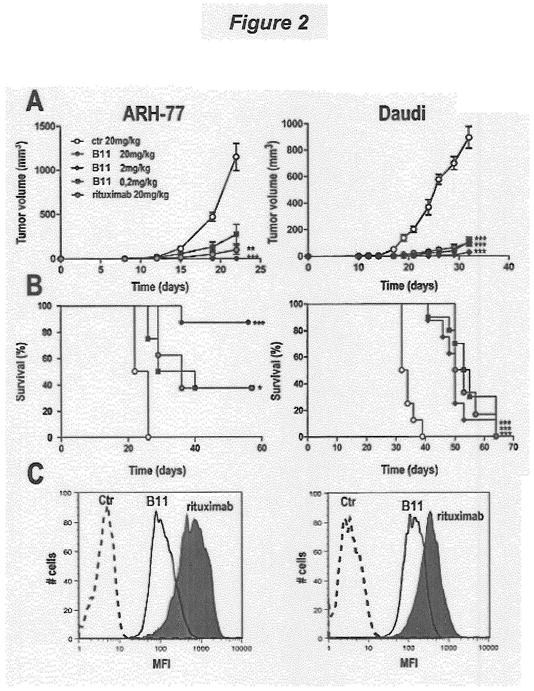

**Figure 2****:** B11 shows significant *in vivo* anti-myeloma efficacy and potency in SCID/ARH-77 myeloma and Daudi xenograft models.
   Tumor cells were injected subcutaneously into the left flank of SCID mice. Mice received twice-weekly intraperitoneal injections with B11, control antibody or rituximab at doses of 20, 2 and/or 0.2mg/kg commencing one day after tumor cell inoculation. There were 8 to 10 animals per treatment group. (A) Tumor volume as a function of antibody dose. (B) Kaplan-Meier survival graph as a function of antibody dose. (C) Epitope expression analyzed by flow cytometry. Statistical significance was calculated relative to control antibody treatment using Kruskal-Wallis Test (Nonparametric ANOVA) with Dunn's Multiple Comparisons Test (tumor volume) or the log-rank test (mouse survival) using Graphpad Instat or Prism software, respectively. Statistical significance was considered for *p < 0.05, **p < 0.01, and ***p < 0.001.

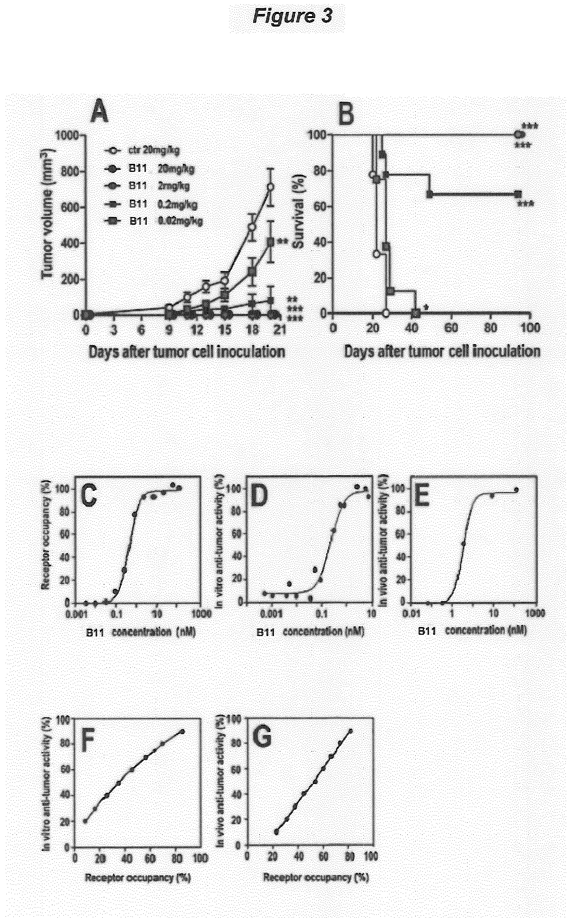

**Figure 3****:** B11 shows significant *in vivo* anti-myeloma efficacy and potency in SCID/ARH-77 myeloma xenograft model.
   Tumor cells were injected subcutaneously into the left flank of SCID mice. Mice received twice-weekly intraperitoneal injections with B11 at doses of 0.02-20 mg/kg commencing day after tumor cell inoculation. There were 8 -10 animals per treatment group. (A) Tumor volume as a function of antibody dose. (B) Kaplan-Meier survival graph as a function of antibody dose. Statistical significance was calculated relative to control antibody treatment using Kruskal-Wallis Test (Nonparametric ANOVA) with Dunn's Multiple Comparisons Test (tumor volume) or the log-rank test (mouse survival) using Graphpad Instat or Prism software, respectively. Statistical significance was considered for *p < 0.05, **p < 0.01, and ***p < 0.001. Graphs illustrate representative experiments out of several performed. (C) The epitope saturation of B11 on tumor cell lines was plotted as a function of B11 concentration. (D) Daudi B lymphoma cells were incubated with B11 or control antibody in the presence of cross-linking secondary Fab'2 goat anti-human-Fc antibody for 16 hours and the cell death-induction was determined after staining of cells with Annexin V/propidium iodide. The cell-death induction by B11 was plotted as a function of concentration. The experiments were done in triplicates and each experiment was repeated at least five times. The graph presents the normalized pooled data from the individual experiments (n=5x3). (E) Blood samples were collected at different time-points during course of *in vivo* xenograft experimentation and analyzed by ELISA to determine B11 trough levels. The *in vivo* anti-tumor activity was plotted as a function of trough B11 serum concentrations and fitted using five-parameter log-log curve and XLfit software. (F) Correlation between *in vitro* anti-tumor activity and B11 epitope saturation. (G) Correlation between *in vivo* anti-tumor activity and B11 epitope saturation.
**Figure 4** shows that Multiple myeloma patients have significant B11 epitope expression. (A) Multiple myeloma patient characteristics and B11 epitope expression. (B) B11 epitope on myeloma cells versus normal B cells.
**Figure 5** shows that B11 has broad and ICAM-1-dependent anti-myeloma activity *in vivo.*
   NCI-H929, EJM, RPMI-8226, or OPM-2 myeloma cells were injected subcutaneously into the left flank of SCID mice at Day 0. Antibody treatment with 2 mg/kg B11 or control IgG1 was started at Day 1 and was continued on a twice-weekly intraperitoneal dosing regimen. Mice were sacrificed when tumor sizes reached the ethical limit. IgG B11 had no effect on tumor growth in animals xenografted with the ICAM-1-negative cell line OPM-2, demonstrating that anti-myeloma activity was ICAM-1-dependent. (A) shows data from one representative experiment out of two performed (filled circles show B11 treatment, empty circles show control IgG1 treatment). (B) shows pooled and normalized data from two independent experiments (n = 8 to 10 animals per treatment group. Filled bars show B11 treatment and empty bars show control IgG1 treatment). Statistical significance was calculated relative to control antibody treatment using Mann Whitney non-parametric analysis and Graph Pad Instat program. Statistical significance was considered for *p < 0.05, **p < 0.01, ***p < 0.001.

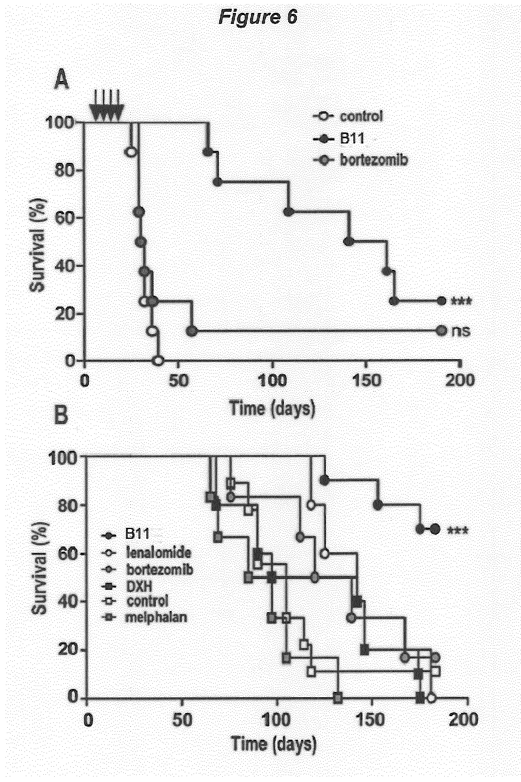

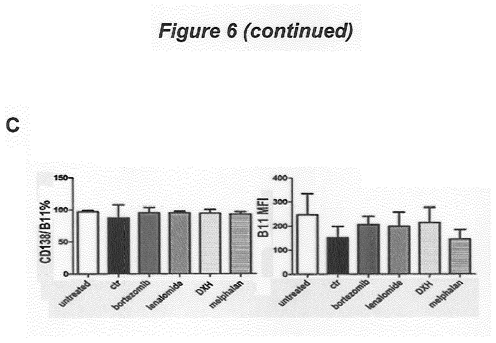

**Figure 6****:** B11 confers protection against advanced experimental multiple myeloma.
   ARH-77 cells or RPMI-8226 were injected intraveniously into the SCID mice. (A) ARH-77 model. Animals received intravenous injections with antibody at 2 mg/kg or bortezomib (Velcade) at 0.5 mg/kg on Days 7, 10, 13, and 16 (as indicated by arrows in the graph). (B) RPMI-8226-model. B11 or control mAb was administered i.v at 2 mg /kg, twice weekly for 8 weeks, bortezomib, at 1 mg/kg, once weekly for 8 weeks, lenalidomide p.o with 2 mg/kg for 2 cycles consisting of 5 days of treatment and 2 days of wash out, melphalan i.v at 3 mg/kg, once weekly for 8 weeks, and dexamethasone (DXH) at 6 mg/kg/inj 3 times / week for 2 consecutive weeks. There were 6-10 mice per treatment group. Statistical significance was calculated using Log-rank Graphpad Prism software and determined at ***p < 0.001. (C) To study the ICAM-1 level on human cells, the cells from different organs were stained and gated for CD38+/mCD45-/B11+. Left panel indicates the percentage of B11 positive cells in CD38+/mCD45 population and right panel the mean fluorescent intensity of the positive cells.

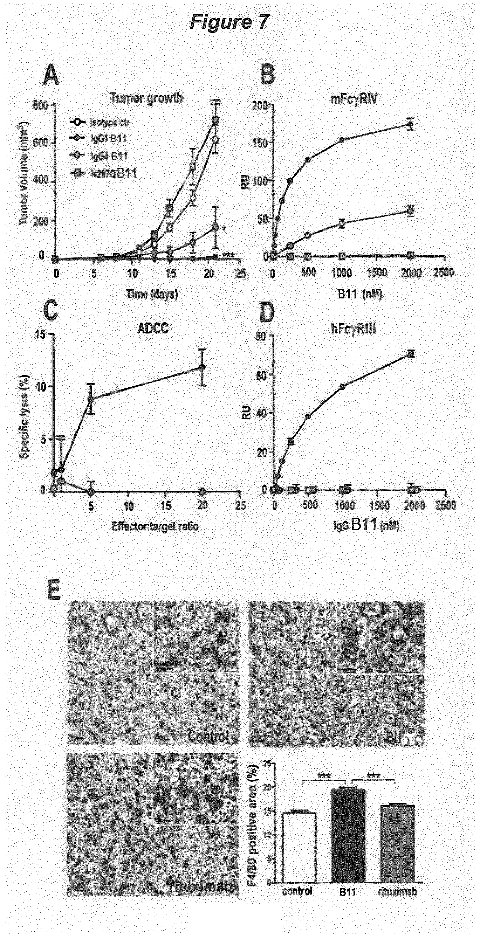

**Figure 7** shows that B11 FcyR-binding ability correlates with *in vitro* and *in vivo* anti-tumor activity.
   (A) ARH-77 cells were injected subcutaneously into the left flank of SCID mice (n = 8 per group). Mice were treated with the different B11 isotypes twice weekly. (B) Binding of B11 isotypes to different recombinant FcγRs was determined using Biacore. B11 IgG1, but not IgG4 or N297Q- mutant, showed strong binding to murine FcγRIV, a principal Fc-receptor involved in Fc-mediated activity in mouse. (C) ADCC was examined using natural killer cells at different ratios as effector cells and the B lymphoma cell line (CL-01) as target cells. As expected, only B11 mediated FcγRIIIA-dependent ADCC of tumor cells. (D) A correlation between ADCC activity and anti-tumor efficacy was observed since B11 isotypes bound human FcγRIIIA, a principal human ADCC-mediating receptor, with different affinities. (E) ARH-77 xenograft tissues from the treated mice were stained and quantificated for the F4/80 positive area which showed that macrophage infiltration of tumor tissue in B11 treated mice was significantly increased than that in rituximab and control IgG treated mice. Bar = 40µm. Diagram shows the percentage of F4/80 positive area in the measured tissues. Statistical significance was calculated relative to control antibody treatment using Kruskal-Wallis Test (Nonparametric ANOVA) with Dunn's Multiple Comparisons Test. Statistical significance was considered for *p < 0.05, **p < 0.01, and ***p < 0.001.

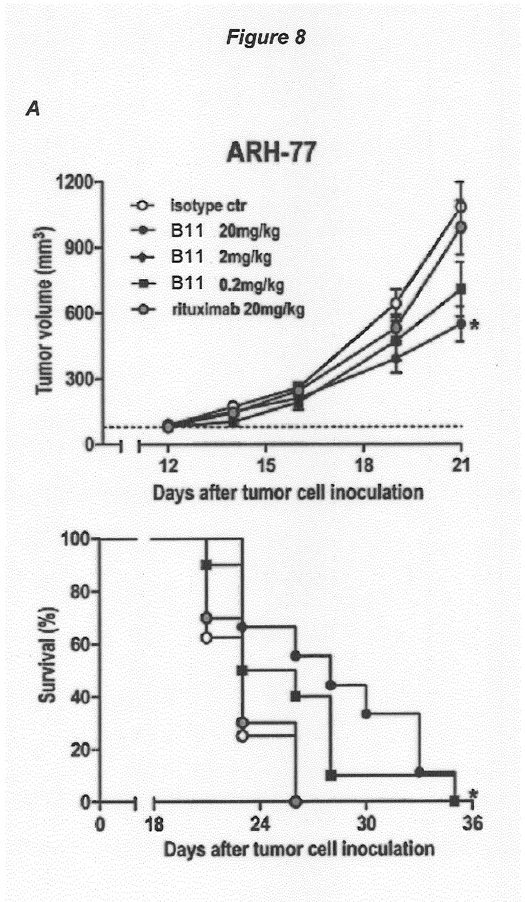

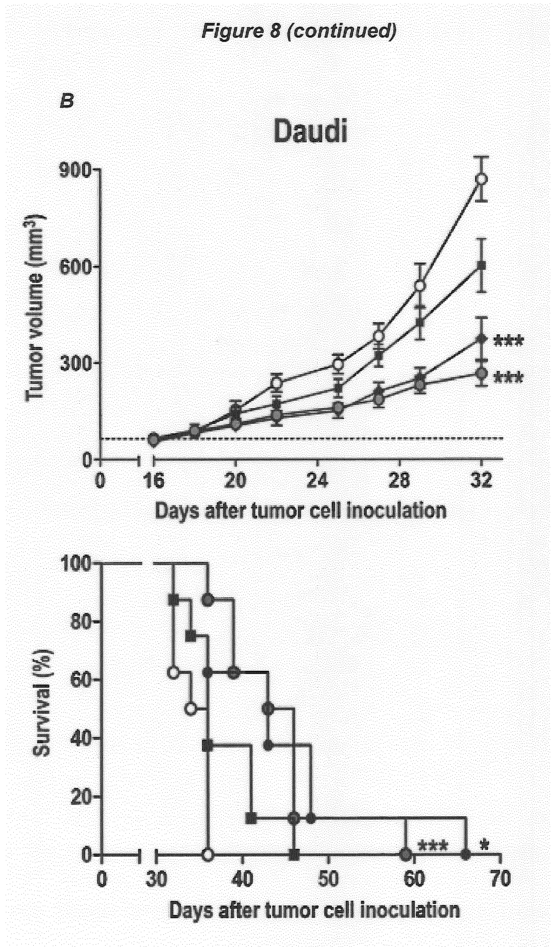

**Figure 8** shows the high efficacy and potency of B11/IgG B11 compared to rituximab in the (A) ARH-77 and (B) Daudi xenograft models.

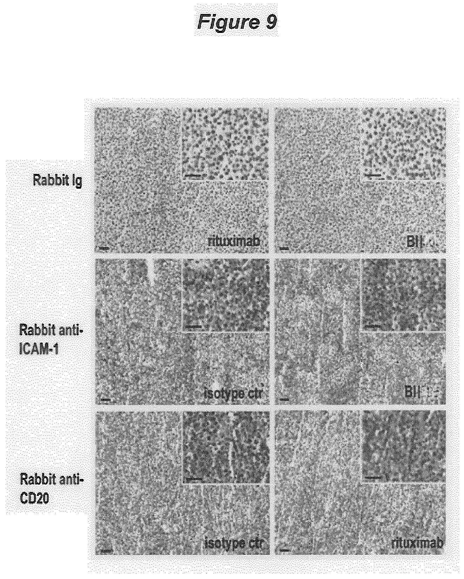

**Figure 9** shows via immunohistochemical analysis that tumours treated with either of B11, Rituximab or control all express similar and significant amounts of CD20 and ICAM-1 antibody-targeted epitopes.

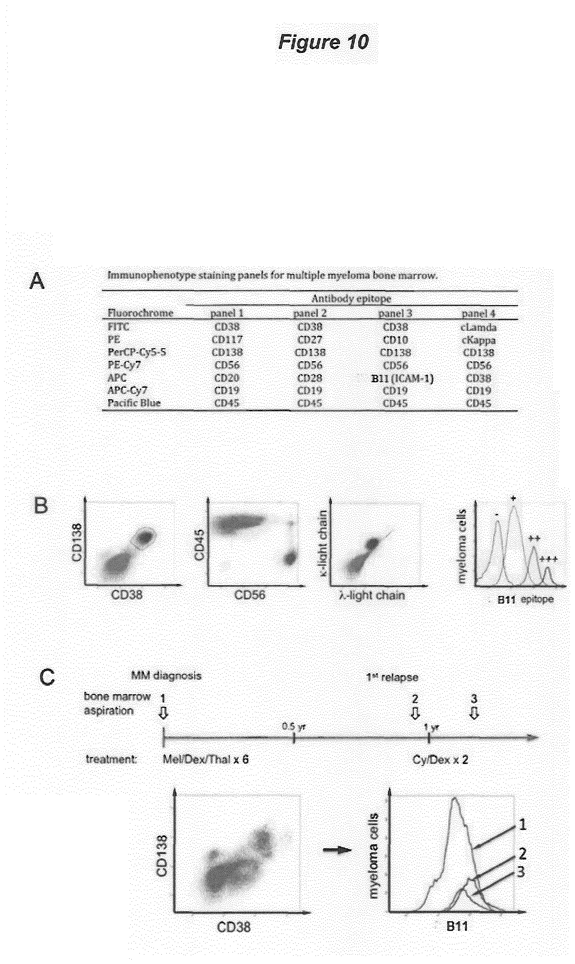

**Figure 10** shows (A) immunophenotype staining panels for MM bone marrow cells. (B) shows flow cytometry analysis used for selecting for high CD38, CD138 and CD56 expression and a loss of CD45, confirming monoclonal expression. B11 epitope expression was subsequently measured for patients #7 (+), #8 (++) and #10 (+++). (C) shows B11 epitope expression in myeloma cells of a patient (1), after relapse (2) and after treatment following the relapse (3).

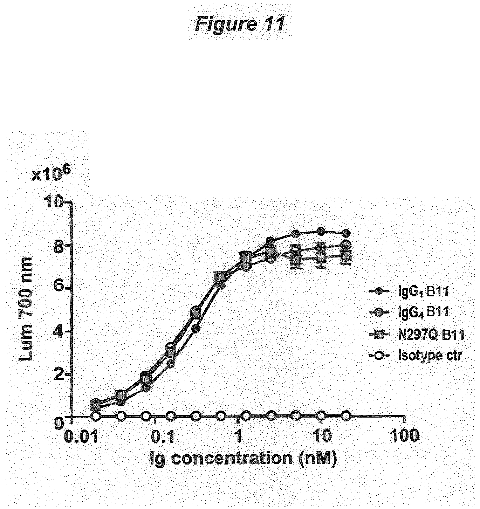

**Figure 11** shows the near identical EC₅₀ values for binding affinities of target protein of the generated isotype switch variants of B11.

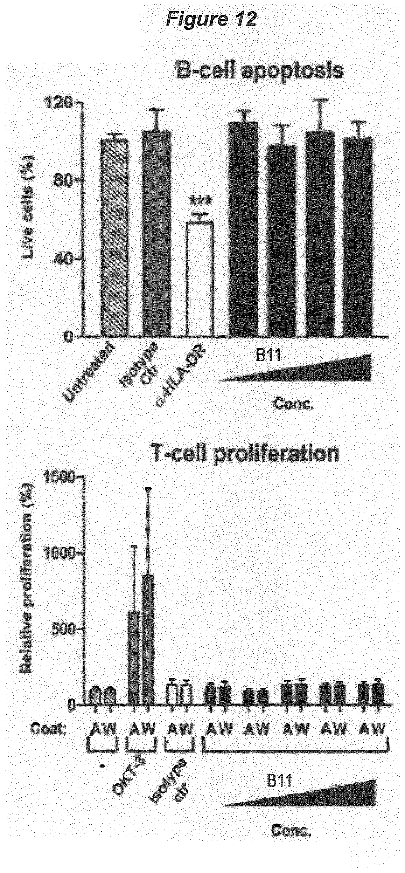

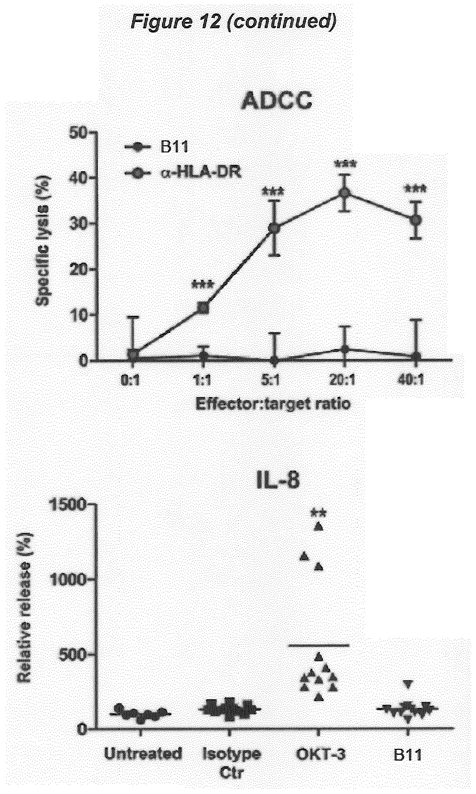

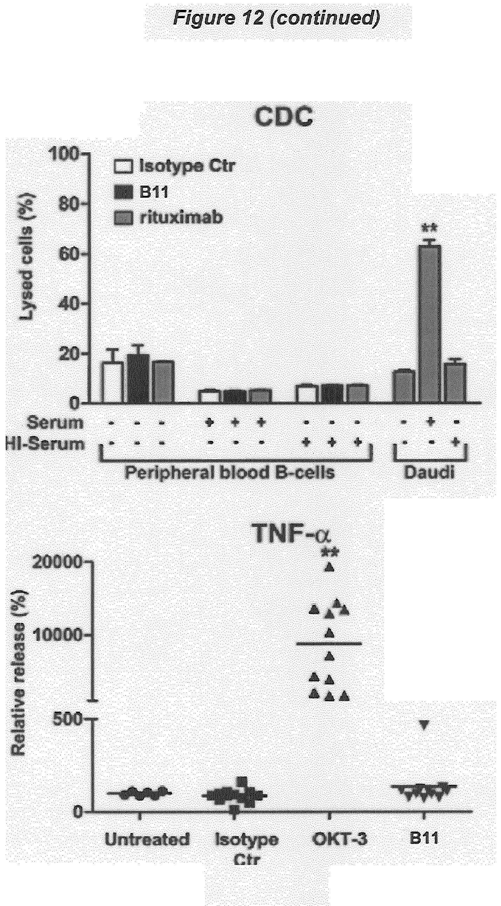

**Figure 12** shows that B-cell apoptosis, T-cell proliferation, Antibody-Dependent Cell-Mediated Cytotoxicity (ADCC), Complement-Dependent cytotoxicity (CDC) and cytokine release (specifically TNF-α and IL-8) are all not significantly affected by B11 treatment.

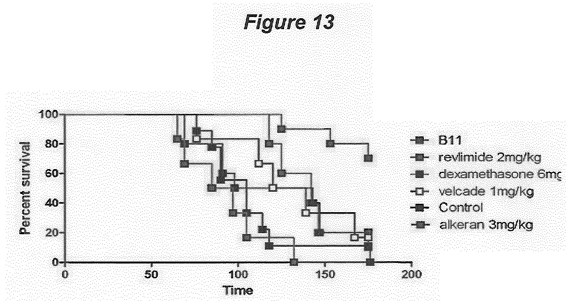

**Figure 13** shows that B11 significantly enhances survival in a disseminated MM xenograft model compared to control treated mice (p<0,001) and compared to four different SOC therapies (p<0,05 compared to revlimide or velcade, p<0,001 compared to dexamethasone or alkeran). N= 6-10/group

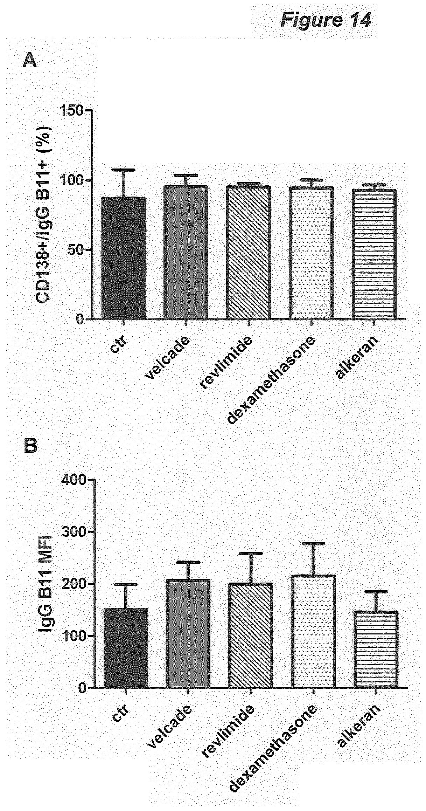

**Figure 14** shows that treatment of mice xenografted with disseminated MM cells with standard of care drugs does neither affect the number of MM cells that express ICAM-1 (A), nor the levels of ICAM-1 expression (B).
**Figure 15** shows the B11 antibody variable heavy (B11-VH) and variable light (B11-VL) nucleotide and amino acid sequences.

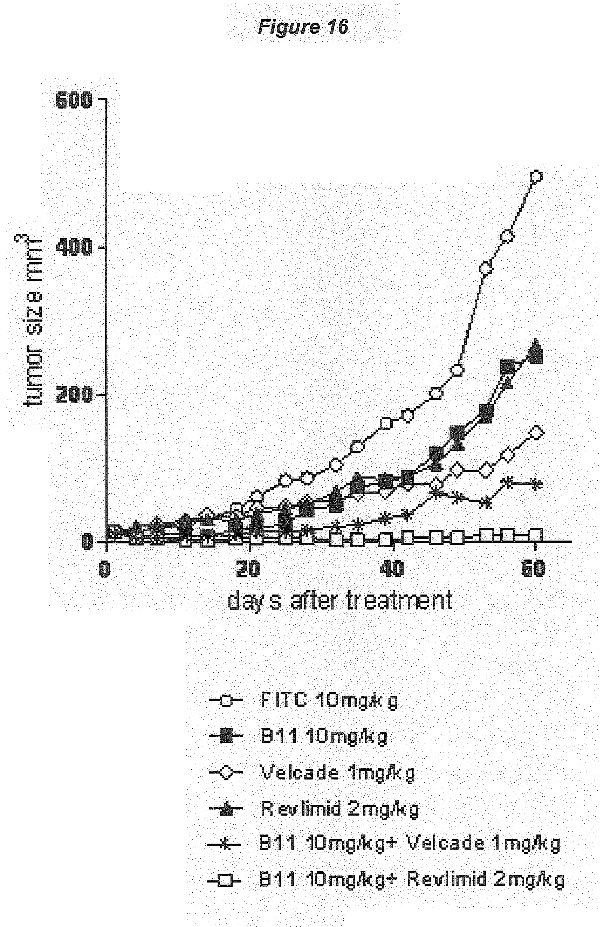

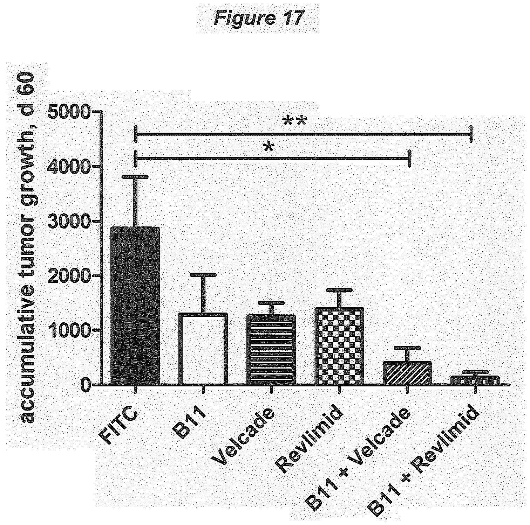

**Figures 16** **and** **17** show *in vivo* efficacy of B11 in combination with REVLIMID® or VELCADE® in a subcutaneous xenograft myeloma model. RPMI-8226 cells were injected subcutaneously into the left flank of NOD/*SCID* mice. Mice were treated with B11, REVLIMID®, VELCADE® or combinations. Treatment started when tumors reached 3 x 3 mm (n=5). Statistical significance was calculated relative to control antibody treatment using Student's t-test (Graphpad Prism software). Statistical significance was considered for *p<0.05, **p<0.01 ***p<0.001.
**Figure 16** shows tumour volume as a function of treatment
**Figure 17** shows accumulative tumour growth.

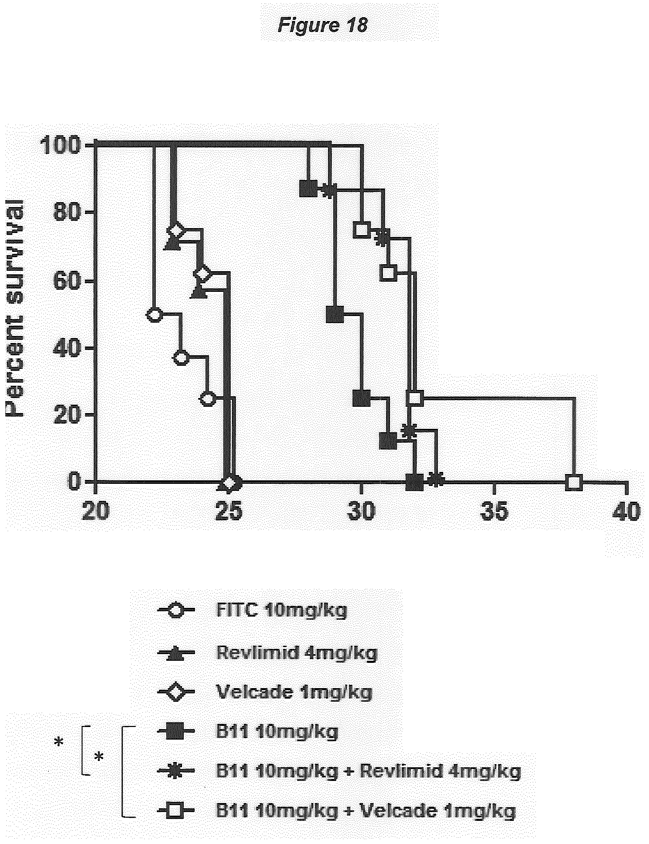

**Figure 18** shows *in vivo* efficacy of B11 in combination with REVLIMID® or VELCADE® in a dissiminated xenograft myeloma model. U266 cells were injected intravenously into NOG mice. Mice were treated with B11, REVLIMID®, VELCADE® or combinations. Treatment started D4 after inoculation (n=8). Kaplan-Meier survival graph. Statistical significance was calculated relative to B11 single treatment using the Log-rank (Graphpad Prism software). Statistical significance was considered for *p<0.05, **p<0.01 ***p<0.001.

### Example 1- Materials and methods utilised in the invention

### Reagents, cells, and animals

Several batches of IgG₁ B11 were either stably expressed from CHO cells or transiently in HEK293 cells. IgG₄ B11 and 297Q B11 were transiently expressed in HEK293 cells. Control antibodies IgG₁CT17 or IgG₁FITC-8GA were transiently expressed in HEK293 cells. Endotoxin-levels of antibodies were found to be <0.1 IU/mL as determined by the LAL-Amoebocyte test. Rituximab (Roche), Velcade (bortezomib) (Janssen-Cilag), lenalidomide (celgene), melphalan (GlaxoSmithKline) and dexamethasone (Mylan) were purchased from local pharmacies in (Lund, Sweden or Dijon, France). ARH-77, RPMI-8226 and Daudi cell lines were obtained from American Type Culture Collection (ATCC, Sweden), NCI-H929, EJM, and OPM-2 cell lines were obtained from Deutsche Sammlung von Mikroorganismen und Zellkulturen (DSMZ, Germany). All cells were maintained in culture medium recommended by the supplier. Logarithmic growth of cells was ensured before harvesting the cells for xenografting. Female *scid* mice on CT.17 background were obtained from Taconic, Denmark, and were used in subsequent studies at age 7-8 weeks. Animal experiments were performed according to ethical guidelines of animal experimentation and all procedures with animals were reviewed and approved by local Lund/Malmö ethical committee.

### Analyses of multiple myeloma patient cell ICAM-1 expression

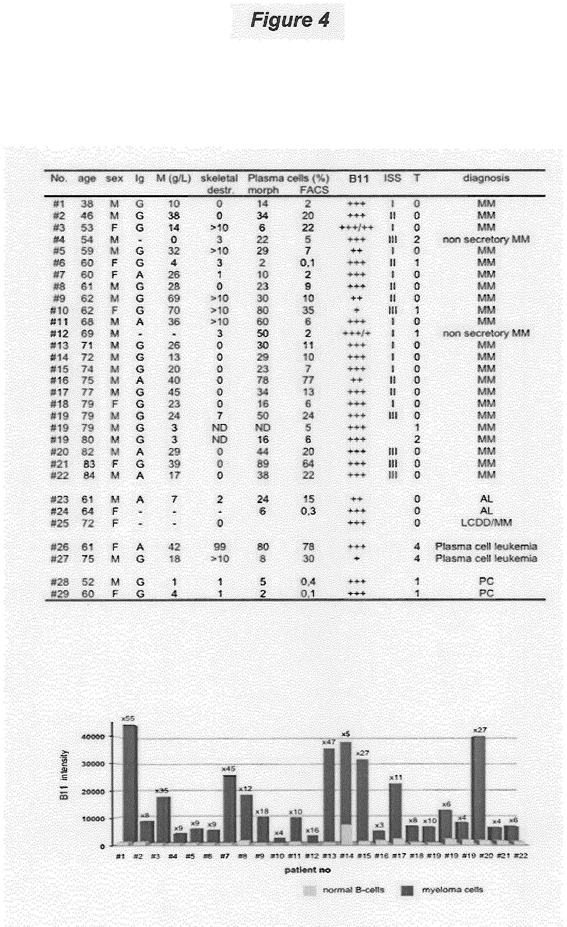

Bone marrow aspirates from eighteen patients investigated for multiple myeloma or related diseases (plasmocytoma, plasma cell leukemia, amyloid light chain amyloidosis) at Department of Hematology, Skånes University Hospital, Lund were analyzed by flow cytometry using four antibody panels recognizing plasma cells (see Example 2) after informed consent and with approval from the local ethical committee. Clinical data were obtained from patients charts (Figure 4A).

### Programmed Cell Death (PCD) assay

Target cells were seeded in 96-well culture plates at a density of 2x10⁶ cells/mL culture medium. Titrated concentrations of IgG₁ B11 or different negative and positive control antibodies were added to the cells, in the absence or presence of anti-human Fab fragment (Jackson ImmunoResearch) for cross-linking. The cells were then incubated for 16 hours at 37°C in a humidified atmosphere of 5% CO₂. The cells were harvested and stained for Annexin V-488/ Propidium Iodide (Invitrogen, Sweden) and analysed using flow cytometer (FACS Calibur, BD Bioscience).

### Antibody-dependant cell-mediated cytotoxicity (ADCC)

Buffy coats from human donors (ordered through Blodcentralen, Lund) were used to isolate peripheral blood mononuclear cells (PBMCs), and subsequently NK cells. Briefly, peripheral blood components were separated using Ficoll Paque PLUS (Amersham Biosciences, Sweden) in LeucoSep tubes (Greiner Bio-One). The PBMC fraction was removed and thoroughly washed in ice-cold DPBS (Invitrogen), before magnetic labeling and separation of the NK cell population using positive or negative NK cell isolation kits and MACS LS columns (Miltenyi Biotec). The purity of the obtained NK cell fractions was analyzed using flow cytometry, after staining with α-CD56 antibodies (BD Biosciences). Target cells were harvested, and incubated in medium with or without the respective antibodies (2 µg/mL) for 60 min on ice. Cells were thereafter washed and resuspended in cold medium before dispension into FACS tubes. Subsequently, isolated NK cells were diluted in ADCC medium and dispensed together with the respective antibody-coated target cells at varying effector/target cell ratios (40:1, 20:1, 5:1 and 1:1). All experiments were performed in triplicates. After completed incubation ToPo-Pro-3 dye and counting beads (Invitrogen) were added and cells were analyzed for membrane permeabilization using flow cytometry.

### Complement-dependent cytotoxicity (CDC)

Target cells were harvested and incubated in RPMI medium with antibodies at 5 µg/mL or at titrated concentrations between 0,01-100 µg/mL for 60 min on ice. Cells were thereafter washed and resuspended in cold medium before dispension into flow cytometry tubes. Treatments were performed in triplicates. Human serum, normal or heat-inactivated, (Sigma, Sweden) was added to tubes and the samples were incubated for 2 h at 37 °C. After completed incubation ToPo-Pro-3 was added at a final concentration of 0.3 µM and cells were analyzed for membrane permeabilization using flow cytometry.

### B11 isotype variant binding to FcγR

His-tagged human FcyRllla or mouse FcγRIV were expressed transient in adherent HEK293E cells, purified using Ni-NTA chromatography and characterized using SDS-PAGE and/or Biacore. Surface plasmon resonance (SPR) measurements were perfomed using a Biacore 3000 instrument. Goat α-human-F(ab)'2 F(ab)'2 fragment (Jackson laboratories) was immobilised with a CM-5 chip using a standard amine coupling protocol. IgG₁ B11, IgG₄ B11, or 297Q B11were diluted to 15 and 60µg/mL respectively and added to the surface at 10µL/min for 3 min. His tagged human FcγRIIIa or mouse FcγRIV were pre-incubated with an α-HIS antibody (R&D Systems) at a 2:1 molar ratio before addition to the chip surface, 30µL/min, 1 min. After each cycle the surface was regenerated twice with glycine buffer pH 1.7.

### Tumor growth

### Subcutaneous grafting:

Mice were anaesthetized with a mixture of sevofluran and oxygen prior to myeloma cell inoculation and 1-5x10⁶ myeloma cells were then subcutaneously injected in a volume of 100 µl into the left flank. Treatment with antibodies by intraperitonial (i.p.) injections was started either the day after cell inoculation (prophylactic model) or when tumors reached a size of approximately 100 mm³ (established model). Antibodies were administrated in PBS in a total volume of 200 µL. Treatment with PBS or isotype control was used as control. Tumors were measured with a digital calliper and the tumor volumes were calculated according to formula: width² x length x 0.52. Animals were sacrificed when the tumor sizes reached the ethical limit of 1.5 cm. Surviving mice were sacrificed after a maximum of 5 months. Blood samples collected from the vena cava were centrifuged at 2500 g for 15 min to obtain serum and the samples were stored at -20°C. Tumors were removed for immunohistochemistry, snap frozen and were kept at -85°C.

### Disseminated model of multiple myeloma:

The anti-myeloma effect of B11 was examined in a disseminated model of multiple myeloma. The early and advanced disseminated model of multiple myeloma was performed at Oncodesign, Dijon, France. Briefly: 1x10⁶ (early) or 5x10⁶ (advanced) ARH-77 tumor cells in 200 µL of RPMI 1640 were injected intravenously (i.v) into the caudal vein of female *scid* mice (D0). Tumor cell injections were performed twenty four to forty eight hours after a whole body irradiation of mice (1.8 Gy, ⁶⁰Co, INRA, BRETENNIERES). The treatment was started at D5 RPMI-8226 model or D7 (ARH-77 model) was administered at D10. B11 or control mAb was administered i.v. at 2mg/kg, twice weekly for 8 weeks, bortezomib, at 1mg/kg, once weekly for 8 weeks, lenalidomide p.o with 2mg/kg for 2 cycles consisting of 5 days of treatment and 2 days of wash out, melphalan i.v. at 3mg/kg, once weekly for 8 weeks, and dexamethasone at 6mg/kg 1 injected 3 times a week for 2 consecutive weeks.

### Statistical analyses

Statistical analyses of tumor growth inhibition was calculated relative to control antibody treatment using Kruskal-Wallis Test (non-parametric ANOVA) with Dunn's Multiple Compositions Test or Mann Whitney non-parametric analysis as stated in figure legends. Statistical analyses of antibody mediated mouse survival were calculated using the Log-rank test and Graphpad Prism software. Statistical significance was considered for *=p<0.05, **=p<0.01 ***=p<0.001.

### Example 2: A novel ICAM-1 antibody isolated by combined differential biopanning and programmed cell death screening has competitive anti-tumor activity against CD20-expressing tumors in vivo.

We applied sequential differential biopanning and high-throughput programmed cell death screening (see example 1) to isolate multiple B cell lymphoma Programmed Cell Death (PCD)-inducing antibodies targeting different tumor cell associated surface receptors from the *in vitro* CDR shuffled naive human antibody library n-CoDeR (Biolvent), as summarised in Figure 1. Among isolated antibodies we identified those specific for ICAM-1 - a receptor not previously associated with antibody induced tumor PCD. Anti-ICAM-1 antibodies induced PCD in both CD20 expressing and CD20 negative tumor cell lines, indicating broad therapeutic applicability. The high specificity of IgG B11 for ICAM-1 and its dose dependent PCD-induction in ICAM-1 expressing Daudi lymphoma cells is illustrated in Figure 1.

In order to further investigate the therapeutic potential of PCD-inducing ICAM-1 antibodies, we evaluated the *in vivo* anti-tumor activity of IgG B11 in tumor models comprising immunodeficient *scid* mice transplanted with either of two well-characterized CD20-expressing B cell malignant cell lines; ARH-77 or Daudi (Fig 1 panel IV). These cell lines have been extensively utilized to investigate and characterize drug efficacy and potency in different models of multiple myeloma (24-48) and non-Hodgkin's lymphoma (49, 50), respectively. Both cell lines express the CD20 antigen making possible the comparison of anti-tumor efficacy and potency with the clinically validated CD20-specific antibody rituximab.

Sub-cutaneous injection of ARH-77 cells resulted in rapid establishment and growth of tumor cells in *scid* mice with tumors being readily palpable between twelve and fourteen days after tumor cell injection. Twice weekly injections of a 20mg/kg dose of IgG B11, commencing one day following tumor cell inoculation, were shown to completely prevent tumor-growth in xenografted mice (Fig 2A, left panel). The CD20-specific positive control mAb rituximab also conferred significant anti-tumor activity albeit less efficaciously so compared to IgG B11. Furthermore, IgG B11 conferred complete survival, even when administered at a ten-fold lower dose (2mg/kg) compared to rituximab (Fig. 2A and B, left panel). Therefore, in this aggressive model of CD20 positive B cell malignancy IgG B11 was more efficacious and more potent in conferring anti-tumor activity and survival compared with rituximab.
We proceeded to investigate IgG B11 *in vivo* anti-tumor activity against Daudi non-We proceeded to investigate IgG B11 *in vivo* anti-tumor activity against Daudi non-Hodgkin lymphoma xenografts. Also in this model, IgG B11 significantly and equally efficaciously compared to rituximab, prevented tumor growth and prolonged survival of tumor bearing mice (Fig 2 A and B), right panel. The enhanced anti-tumor activity of IgG B11 did not result from tumor cells expressing higher numbers of B11 compared to rituximab epitopes. Conversely, flow-cytometric analysis revealed that both ARH-77 and Daudi cells expressed significantly fewer ICAM-1 compared to rituximab epitopes. (Fig 2C left and right panels). These results demonstrated that IgG B11 has significant *in vivo* anti-tumor activity against two different well-characterized CD20-expressing tumor cell lines.

We next performed a dose-titration experiment to establish IgG B11 *in vivo* potency and the minimal dose achieving maximal anti-tumor activity, using the *scid*/ARH-77 model system. IgG B11 showed titratable anti-tumor activity, which followed a sigmoidal curve and peaked at the 2mg/kg dose and remained near maximal at a dose of 0.2 mg/kg (Fig. 3A). Mouse serum antibody trough concentrations were determined by ELISA at the end of experimentation. Trough antibody concentrations were then plotted against percent of maximal *in vivo* anti-tumour activity, and against percent of maximal ICAM-1 receptor occupancy and tumour cell PCD at corresponding antibody concentrations *in vitro* (Figs. 3C, 3D, 3E, 3F and 3G). Strikingly, a near perfect correlation between antibody concentration and *in vitro* tumour cell receptor occupancy, *in vitro* tumor cell PCD, and *in vivo* anti-tumour activity was observed, consistent with ICAM-1 dependent direct cell cytotoxicity underlying *in vivo* anti-tumour activity.

The high efficacy and potency of IgG B11 was confirmed in the analogous but more advanced scid/ARH-77 xenograft model (Fig. 8A). *Scid* mice carrying palpable ARH-77 tumors were treated with different doses of IgG B11, rituximab or control antibodies. In this model rituximab was incapable of reducing tumor growth or promoting animal survival (p>0.05). In contrast, IgG B11 significantly prevented tumor growth and prolonged animal survival (Fig. 8A, left panel) compared to rituximab-treatment, even when administered at a 100-fold lower dose (0.2mg/kg). The lack of a rituximab anti-tumor effect in the advanced tumor model was not the result of tumor evasion or downregulated antigen expression. Immunohistochemical analysis of tumor tissue harvested from antibody and control treated mice at the completion of experimentation revealed that tumors expressed similar and significant amounts of CD20 and ICAM-1 antibody-targeted epitopes throughout experimentation Fig. 9).

### Example 3: ICAM-1 and the B11 epitope is broadly expressed in lymphoproliferative disorders including multiple myeloma

The highly efficacious and potent *in vitro* and *in vivo* anti-tumor activity of ICAM-1 B11 IgG spurred us to comprehensively assess ICAM-1 expression in different lymphoproliferative disorders. Firstly, ICAM-1 expression in chronic lymphocytic leukaemia (CLL), follicular cell lymphoma (FCL), mantle cell lymphoma (MCL) and diffuse large cell B cell lymphoma (DLBCL), was determined by tissue microarray immunohistochemical analysis (Table 1).

**Table 1. ICAM-1 expression in B and T cell lymphomas**

| | **CLL** | **FCL** | **MCL** | **DLCBL** |
|---|---|---|---|---|
| ICAM-1 expressing tissue: | 27% (9/33) | 95% (40/42) | 89% (8/9) | 98% (47/48) |
| % Positive Cells in Positive Tissue | 23 | 56 | 23 | 81 |
| Relative Intensity Scale 0-3 | 0.8 | 1.4 | 1.0 | 1.3 |

ICAM-1 was expressed in 27% of CLL, 95% of FCL, 89% of MCL, and 98% of DLBCL at medium to strong intensity compared to positive control tonsil tissue (Table 1).

Previous reports described a strong association of ICAM-1 with multiple myeloma disease progression with ICAM-1 being highly expressed in myeloma *per* se and being upregulated in advanced disease and in multiple myeloma (MM) patients refractory to chemotherapy (14, 22, 51).

Based on these observations, we evaluated ICAM-1 B11 epitope expression on bone marrow cells in multiple myeloma patients and related diseases (plasmacytoma, plasma cell leukemia, light chain amyloidosis) by flow cytometry (Fig. 4) Myeloma cells were identified based on expression of CD38/CD138/CD45 and CD56 according to European Network on multiparameter flow cytometry in multiple myeloma guidelines (Rawstron et al Haematologica (2008 93 pp431-8).

### Flow cytometry staining panel

Bone marrow aspirates approximately 5-7ml were taken from iliac crest in local anesthesia (10ml Xylocain) according to local routines at Department of Haematology, Skånes University Hospital. Erytrocytes were removed from bone marrow cells by FACSlysis (Becton Dickinson, Stockholm, Sweden) according to manufacturers instruction followed by staining with four antibody panels, as indicated in table A (Fig. 10). In panel 4 the surface staining was followed by permeabilization with Perm Fix, BD and internal staining against kappa and lambda light chain to confirm monoclonality of the multiple myeloma cells.

### Analysis of myeloma cells in bone marrow from multiple myeloma patients.

Stained bone marrow cells were analysed by flow cytometry using a FACS Canto II (Fig. 10B). Myeloma cells were gated based on high expression of CD138 and CD38 and further confirmed by high CD56 expression and loss of CD45. Furthermore, intracellular staining was used to confirm monoclonal expression by kappa and lambda staining. BII epitope expression were categorized as shown in the histogram (right position) with (+), (++) and (+++) corresponding to patients #8, #7 and #10. BII negativ cells (-) are B-cells from patient #8(Fig. 10B).

### BII epitope expression on myeloma cells during disease progression in a patient with multiple myeloma.

Bone marrow plasma cells were taken at diagnosis (bone marrow no 1) of a 79 year old man with multiple myeloma. Treatment was initiated with orally melphalan and dexamethasone pulses (six cycles) in combination with continuous thalidomide with major response. However, two months after ending the treatment the patient relapsed (bone marrow no 2). This time the patient received two cycles of cyclophosphamide and dexamethasone pulses followed by a new evaluation of the bone marrow (bone marrow no 3). Mean B11 expression in myleoma cells increased two-fold after first relapse (histogram, right) (Fig. 10C).

All Multiple Myeloma patients expressed the ICAM-1 B11 epitope on myeloma cells. The level of ICAM-1 B11 expression was generally very high on myeloma cells with mean expression levels 17-fold greater compared to patient's normal B cells (Figure 4).

We conclude that ICAM-1 is strongly expressed in several lymphoproliferative disorders including FCL and DLBCL and that the ICAM-1 B11 epitope is strongly expressed by Multiple Myeloma plasma cells.

### Example 4: IgG B11 has broad anti-myeloma activity in vivo

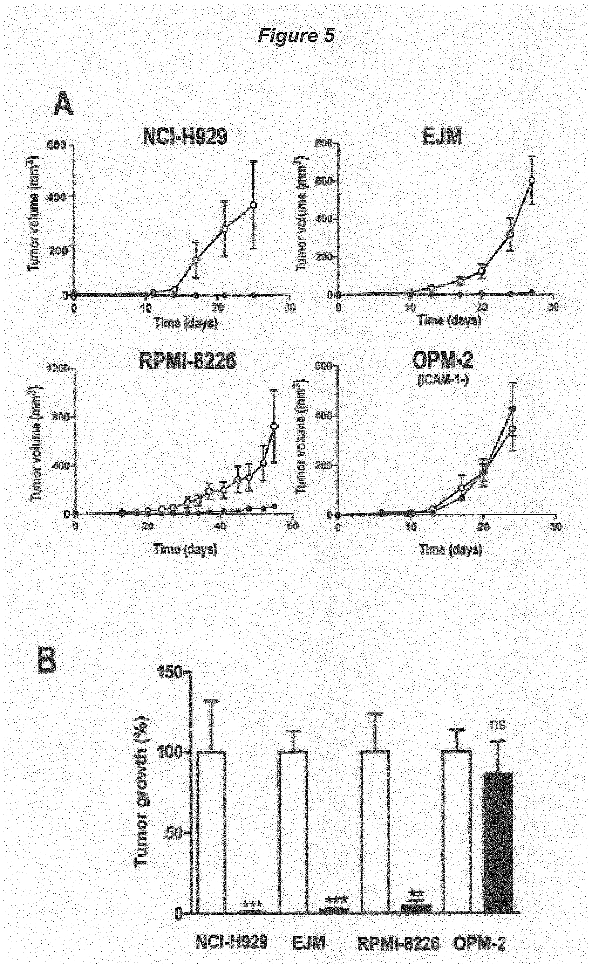

Based on the observed high expression of the B11 epitope in multiple myeloma, the previously reported association of ICAM-1 with multiple myeloma and resistance to currently available treatment, and the apparent significant *in vivo* anti-tumor activity of B11 against CD20-expressing malignant B cell tumors, we proceeded to assess IgG B11 *in vivo* anti-myeloma activity in *scid*/xenograft models comprising four different well characterised multiple myeloma cell-lines. These cell lines express the myeloma markers CD38 and CD138, but do not express CD20. Twice weekly dosing with 2mg/kg of IgG B11 reduced myeloma tumor growth in mice xenografted with ICAM-1 expressing cell lines EJM, RPMI-8226, and NCI-H929 by 98%, 96% and 99%, respectively (Fig. 5A and B, *p* _{EJM}<0.0009, *p* _{RPMI-8226}<0.0037, *p* _{NCI-H929}<0.0002). In contrast, IgG B11 did not affect tumor growth in mice xenografted with the ICAM-1 negative cell line OPM-2 (Fig.5A and B, *p* _{OPM-2}>0.05). Taken together, these studies demonstrated a highly efficacious, broad, and ICAM-1 dependent *in vivo* anti-myeloma activity of IgG B11.

### Example 5: IgG B11 confers enhanced survival compared to currently used treatment in disseminated experimental models of advanced multiple myeloma

The disseminated model of scid/ARH-77 is a well-established experimental model that resembles human multiple myeloma disease progression and disease manifestation in many respects (33). We utilized this model to further characterize IgG B11 anti-myeloma activity (Fig. 6A). Intravenous injection of irradiated *scid* mice with 1x10⁶ ARH-77 cells was previously shown to result in their dissemination and establishment in mouse bone-marrow, and resulting osteolytic bone-lesions and hypercalcemia, and ultimately, in animal paralysis or difficulty of breathing at which time animals were immediately sacrificed.

Starting seven days following i.v. injection of myeloma cells, animals received four consecutive treatments with the proteasome inhibitor bortezomib ("Velcade"), IgG B11, ctrl IgG or saline. At 22 days following tumor cell injection control-treated animals started showing either significant weight-loss (>15% over a period of 3 days or paralysis and had to be sacrificed (Fig. 6A). Control-treated mice progressively developed symptoms of multiple myeloma and did not survive past 39 days following tumor cell-injection. Treatment with the proteasome-inhibitory drug bortezomib (Velcade) insignificantly delayed disease onset and did not significantly enhance animal survival compared to control-treatment (*p* _{BZB vs Saline}<0.554x, *p* _{bZb vs ctrl IgG}<0.3509). In contrast, treatment with IgG B11 showed a dramatic anti-tumor effect and doubled the time to symptomatic disease onset and increased mean survival time compared to control- or Velcade-treatment (*p* _{BZB vs Saline}<0.0001, *p* _{BZB vs ctrl IgG}<0.0001). These p values demonstrate statiscally significant differences in tumour growth of animal survival between treatments.

The anti-myeloma activity of IgG B11 compared to currently used treatment was further examined in an analogous advanced disseminated multiple myeloma model comprising RPMI-8226 myeloma cells (Fig. 6B). In this model, treatment with IgG B11 significantly enhanced survival and delayed disease onset compared to treatment with bortezomib and compared to treatment with dexamethasone, both drugs administered at clinically relevant doses showing maximal in vivo therapeutic efficacy yet no apparent toxicity. Furthermore, there was a trend towards improved survival in IgG B11 treated mice compared to mice treated with the immunomodulatory drug revlimid.

To study the ICAM-1 level on human cells, the cells from different organs were stained and gated for CD38+/mCD45-/B11+. Figure 6C shows the percentage of B11 positive cells in CD38+/mCD45 population and the mean fluorescent intensity of the positive cells.

### Detection of ICAM-1 expression on myeloma cells

Organs for FACS analysis were collected at scarification of mice, and cells from tissues were prepared by mechanistic dissociation and dispase/collagenase enzymatic digestion (Gibco, France). Cells were stained with anti-human CD38 antibody (PerCP-Cy5, Becton Dickinson), an anti-mouse CD45 (PE, Becton Dickinson) and human ICAM-1 (B11 IgG1 AF647, BioInvent) and incubated in the dark for 15 min at room temperature. After the incubation, cells were washed twice and the surface fluorescence of cells was analyzed with a flow cytometer apparatus (LSRII) at the Flow Cytometry Facility of the University of Burgundy.

### Example 6: IgG B11 in vivo and in vitro anti-tumor activity is Fc-dependent and correlates with binding to mouse and human Fc gamma receptors

Previous studies demonstrated the broad and potent PCD-inducing properties of IgG B11 in a wide panel of B cell malignant cell lines (10). Its ability to engage cellular effector mechanisms was, however, not previously investigated. Given the highly potent and efficacious *in vivo* anti-tumor activity of B11 IgG, and the critical importance of FcγR-mediated anti-tumor mechanisms for clinical and *in vivo* therapeutic activity of clinically validated cancer mAbs including rituximab (52) (53) (54), we next addressed the contribution of antibody Fc: host FcyR-dependent mechanisms for IgG B11 therapeutic activity.

To this end we generated human IgG1, IgG4 and FcγR-binding deficient mutant IgG1 (N297Q IgG1) isotype switch variants with documented differential affinity for human FcγRs (55) and differential ability to engage human FcγR-dependent anti-tumor activity (56), and investigated their respective *in vivo* therapeutic efficacy in relation to their FcγR binding properties. Retained affinities for targeted antigen of generated isotype switch variants was demonstrated by their near identical EC₅₀ values for binding to recombinant or cell surface expressed target protein (Fig. 11).

Strikingly, anti-tumor activities of IgG B11 isotype switch variants correlated perfectly with binding to m FcγRIV - the structural and functional homologue of human FcgRIIIa and a principal murine FcγR conferring antibody mediated cellular cytotoxicity *in vivo* and increased in the order of IgG1_{N297Q}< IgG4< IgG1 (Fig 7A and B). Importantly, mice treated with IgG₁, IgG₄, or IgG_{1 N297Q} variant antibodies of B11 had similar serum antibody titers at the end of experimentation (Table 2) indicating that the different antibody variants had similar *in vivo* half-lives, and demonstrating that differential anti-tumor activity did not result from differences in pharmacokinetics.

**Table 3: Serum antibody titers of mice treated with IgG1, IgG4 or N297Q-mutant.**

| In vivo IgG Levels (µg/mL) | | | |
|---|---|---|---|
| Dose (mg/kg) | IgG1 | IgG4 | N297Q-mutant |
| 2 | 20±2.9 | 13 ± 3.1 | 9.6±3. |
| 0.2 | 1.2 ± 0.4 | NDA | NDA |
| 0.02 | 0.06±0.02 | NDA | NDA |

| | | | |
|---|---|---|---|
| NDA= No data available | | | |

Moreover, immunohistochemical analyses of tumor tissue demonstrated massive influx of F4/80⁺ host effector cells in IgG B11 treated compared to control IgG treated and untreated mice and, interestingly, compared to rituximab-treated mice (Figure 7E). These findings indicated that Fc:FcγR-dependent host effector cell-mediated mechanisms significantly contributed to IgG B11 anti-tumor activity *in vivo.*

To confirm a role for Fc:FcγR -dependent mechanisms in IgG B11 anti-tumor activity, we examined its ability to mediate antibody-dependant cell-mediated cytotoxicity (ADCC) of human target tumor cells. As expected, IgG B11 IgG₁ bound to human FcγRIIIa and mediated ADCC of target tumor cells in the presence of human Natural Killer effector cells (Fig. 7C). In contrast, B11 IgG₄ and IgG1_{N297Q} variants did not bind to human FcγRIIIa and did not mediate ADCC of target tumor cells. Analogous to the *in vivo* setting therefore, IgG B11 mediated ADCC *in vitro,* was Fc-dependent and correlated with binding to the principal human ADCC-mediating receptor FcγRIIIA (Fig. 7D). Cancer mAb Fc-dependent anti-tumor activity may, besides Fc:FcγR-dependent anti-tumor mechanisms, result from activation of the complement cascade by so called complement-dependent cytotoxicity (CDC). We therefore examined the ability of IgG B11 to induce CDC in a panel of ICAM-1 expressing tumor cell lines. IgG B11 did not induce CDC in either of monitored tumor cell lines . In contrast, and as previously reported, treatment with the positive control rituximab effectively induced CDC (57-59).

### Example 7: IgG B11 is not cytotoxic against normal ICAM-1 expressing cells in vitro

Under normal physiological circumstances ICAM-1 is constitutively expressed at low levels on vascular endothelium, epithelial cells, fibroblasts, keratinocytes, leukocytes as well as on conventional antigen presenting cells (APC) (60). ICAM-1 expression is, however, upregulated by several cytokines and pro-inflammatory agents including IFN-γ, TNF- α, lipopolysaccaride (LPS), oxygen radicals and hypoxia released in response to trauma or during inflammatory responses (60, 61), raising safety concerns regarding treatment with an anti-ICAM-1 antibody like IgG B11.

Based on IgG B11's documented ability to confer programmed cell death and ADCC of malignant B cells, and a proposed general negative role for complement activation with regard to antibody tolerability (Lim et al (2010) Haematologica, 95, pp135-143), we therefore examined programmed cell death-inducing, ADCC or CDC effects of IgG B11 in ICAM-1 expressing normal (untransformed) human peripheral blood B cells and endothelial cell (Fig 12). Whereas peripheral blood B cells and naive B cells showed low endogenous expression of ICAM-1, Human Umbilical Vein Endothelial Cells (HUVEC) and Human Dermal Microvascular Endothelial Cell (HMVEC) cells showed significant ICAM-1 expression, which was further upregulated in response to IFN-γ stimulation as determined by flow-cytometric analyses (data not shown).

IgG B11 did not, however, induce PCD in either of the examined resting or activated normal ICAM-1 expressing cell types (Fig. 12). In contrast and as expected, treatment of endothelial cells with paclitaxel and treatment of B cells with positive control anti-HLA-DR or anti-CD20 antibody induced significant endothelial cell and B cell programmed cell death, respectively. Similarly, IgG B11 did not confer ADCC or CDC of HUVEC, HMVEC or peripheral blood B cells (Fig. 12).

### Example 8: IgG B11 does not modulate Peripheral Blood Mononuclear Cell (PBMC) cytokine release or T cell proliferation in vitro

We next assessed IgG B11 effects on PBMC cytokine release and cell proliferation. In order to maximize chances of identifying any PBMC agonistic properties of IgG B11, we used two different antibody coating-protocols where antibody is hyper-cross-linked as previously described (62). IgG B11 immobilised by either protocol induced programmed cell death of Daudi lymphoma cells, demonstrating that biological activity was retained following immobilization (data not shown). IgG B11 did not, however, induce PBMC cytokine release and did not induce cell proliferation by either immobilization protocol or when added in solution in the presence or absence of cross-linking reagent (Fig. 12).

In contrast, and as expected, incubation of PBMCs with immobilized positive control anti-CD3 antibody resulted in significant PBMC release of IL-1β, IL-2, IL-6, IL-8, TNF-α and IFN-γ (Fig. 12) . Analogous experiments demonstrated that IgG B11 added in solution did not induce or enhance cytokine release from resting or lipopolysaccharide pre-stimulated PBMCs, and did not induce cell proliferation (Fig. 12).

### Example 9 - Anti-ICAM-1 antibody has superior effect in disseminated Multiple Myeloma in vivo compared to standard treatments and ICAM-1 expression itself is unaffected by such standard treatments

### Cell lines

The RPMI 8226 was obtained from Pharmacell (France) and ARH-77 was from ATCC. Tumour cells were grown in suspension at 37°C in a humidified atmosphere (5% CO₂, 95% air). For both cell lines the culture medium was RPMI 1640 containing 2mM L-glutamine supplemented with 10% fetal bovine serum. For ARH-77, it was also supplemented with 25mM HEPES, 1mM sodium pyruvate and glucose to a final concentration of 4.5 g/L. For *in vivo* tumor studies, cells were harvested when in a proliferating state and viability was checked using 0.25% trypan blue exclusion.

### Animals

Female CB-17 SCID *scid*/*scid* mice, 6-8 week-old were be obtained from CHARLES RIVER (L'Arbresles, France) or Taconic (Denmark) and housed in SPF facilities with food and water *ad libitum.* All animal experiments were performed according to ethical guidelines. Mice were observed for a minimum of 7 days before experiment start.

### Disseminated RPMI-8226 in vivo model

200 µl containing 10x10⁶ of RPMI 8226 tumour cells was intravenously injected into the caudal vein of female SCID mice. The mice had been subjected to a whole body irradiation of mice (1.8 Gy, ⁶⁰Co, BioMEP) 24 to 72 hours prior to cell injection. At D1, tumor injected mice were randomized according to their individual body weight. All treatment started day 5 after tumor cell administration except for ALKERAN®, which started at day 10. All treatments were injected in a dose volume of 10 ml/kg/inj. Mice received one of the following treatments:
Monoclonal anti-ICAM mAb or control mAb was administered i.v in a PBS solution containing at 2 mg /kg, 2 times / week for 8 consecutive weeks.

VELCADE® (Bortezomib, 3.5 mg, Janssen-Cilag) was solubilized in NaCl 0.9% and thereafter aliquoted and kept at -20°C. One vial was thawed just prior to injection and thereafter discarded. Mice received i.v injections of 0.5, 1 or 2 mg/kg, 1 time / week for 8 consecutive weeks.

Revlimid® (Lenalidomide, 5 mg/capsule, Celgene) capsules was first solubilized in DMSO which was thereafter diluted to a final concentration of 5% DMSO, 0.2% HCl 1N (Sigma), 5% Tween 80 (Sigma) and 89.8% NaCl 0.9%. The solution was prepared fresh each day of treatment. Mice were treated p.o with 1, 2 or 3 mg/kg for 2 cycles consisting of 5 days of treatment and 2 days of wash out.

ALKERAN® (Melphalan, 50 mg, GlaxoSmithKline) was prepared by mixing the vial containing 50 mg of ALKERAN® with the supplied vehicle and thereafter aliquoted and kept at -20°C. Before each administration, one aliquot was thawed and diluted in NaCl 0.9% and injected i.v at 3, 6 or 12 mg/kg, 1 time / week for for 8 consecutive weeks.

Dexamethasone® (4 mg/ml, Mylan) was diluted in NaCl 0.9%. A fresh working solution was prepared each day of treatment. Mice received 2, 4 and 6 mg/kg/inj 3 times / week for 2 consecutive weeks.

### Termination, ICAM-1 expression

The health status and behavior of mice was recorded every day and body weight of mice was recorded twice a week. Mice were terminated if they showed signs of cachexia, compound toxicity, hind leg paralysis or severe body weight loss. Upon termination, bone marrow, spinal cord, adrenal glands and kidneys were collected and examined for ICAM-1 expression on tumor cells. To distinguish cells of human versus murine origin, an antimouse CD45 mAb was used CD45. Hence MM cells were defined as CD38⁺/mCD45⁻ (Becton Dickinson). In addition, cells were stained with AF647 conjugated anti ICAM-1 mAb and thereafter analyzed in a FACS LSRII. Bone marrow was mechanically dissociated to a single cell suspension whereas adrenal glands, kidneys and spinal cord cells were prepared using a combination of mechanistic dissociation and dispase/collagenase digestion (Gibco, France). Prior to the experiment, dispase and collagenase treatments were shown not to effect ICAM-1 levels on MM cell lines.

### Subcutaneous, established ARH-77 in vivo model

100 µl containing 5x10⁶ ARH-77 cells were injected sub-cutaneously in the flank of female SCID mice. Tumor volumes was measured 3 times / week after tumor cell injection and throughout the experiment. When approaching a mean value of 100mm³ (day 12), the mice were randomized according to tumor volume and treatments started.

Mice received either anti-ICAM mAb alone or in combination with one of two doses of Revlimid® or VELCADE®.

Control mice received either control mAb alone or in combination with Revlimid® or VELCADE®.

Monoclonal anti-ICAM mAb or control mAb was administered i.p in a 200 µl PBS solution containing at 2 mg /kg, 2 times / week for throughout the experiment.

VELCADE® (Bortezomib, 3.5 mg, Janssen-Cilag) was prepared as described and administered in 200 µl i.p at 0.5 or 1 mg/kg, 2 times / week throughout the experiment.

Revlimid® (Lenalidomide, 5 mg/capsule, Celgene) was prepared as described and administered in 200 µl p.o at 1 or 2 mg/kg, 5 times / week throughout the experiment.

The health status and behaviour of mice was recorded every day. Mice were terminated if they showed signs of cachexia, compound toxicity, severe body weight loss or when the tumor had reach a size of 1.5 cm in diameter.

### RESULTS

### Superior effect of anti-ICAM-1 in disseminated MM in vivo model

Disseminated xenograft model (multiple myeloma cell line RPMI-8226) treated with B11 and four different standard of care (SOC) therapies show a highly improved survival in B11 treated compared to isotype control treated group, see figure 13. In addition, the anti ICAM-1 mAb is more effective compared to other SOC single therapies at doses which were not acutely toxic, see Fig.13.

### In vivo MM ICAM-1 expression is unaffected by various treatments

When tumor cells are isolated from treated mice, ICAM-1 is still expressed at unchanged, high levels on tumor cells collected from mice treated with standard of care therapies (AIKERAN®, Revlimid®, Dexamethasone® or VELCADE®), see Fig. 4.

### Example 10 - Combination therapy of B11 antibody and chemotherapeutics

### Cells

The human multiple myeloma cell lines RPMI-8226 and U266 were obtained from American Type Culture Collection (ATCC). Cells were maintained in RPMI 1640 medium containing 2 mM L-glutamine supplemented with 10% Fetal Calf Serum (FCS). Logarithmic growth phase of cells was ensured before harvesting cells for the xenografting.

### Tumour models

Mice were kept in groups of 4-5 mice/cage Animal experiments were performed according to ethical guidelines of animal experimentation and all procedures with animals were reviewed and approved by local Lund/Malmö ethical committee.

### Subcutaneous RPMI-8226 model

Six-eight weeks-old female NOD/SCID mice were obtained from Taconic, Denmark. Mice were anaesthetized with a mixture of isofluran and oxygen prior to myeloma cell inoculation and 5x10⁶ RPMI-8226 cells were subcutaneously injected in a volume of 100 µl into the left flank. Tumours were measured with a digital calliper twice a week and the tumour volumes were calculated according to the formula: width² x length x 0.52. Treatment according to the scheme below was started when tumours reached 3 x 3 mm. Animals were sacrificed when the tumour sizes reached a diameter of 15 mm.

The treatment schedule:

| **Groups** | **No. mice** | **Treatment** | **Dose (mg/kg/inj)** | **Adm. route** | **Schedule** |
|---|---|---|---|---|---|
| 1 | 5 | FITC-8GA | 10 | i.p | TWx8 |
| 2 | 5 | B11 | 10 | i.p | TWx8 |
| 3 | 5 | REVLIMID® | 4 | P.O | (Q1Dx5)x2W* |
| 4 | 5 | VELCADE® (Bortezomib) | 1 | i.p | Q7Dx8 |
| 5 | 5 | B11 + REVLIMID® | 10+4 | i.p + P.O | TWx8 + (Q1 Dx5)x2W* |
| 6 | 5 | B11 + VELCADE® | 10 + 1 | P.O | TWx8 + Q7Dx8 |

| | | | | | |
|---|---|---|---|---|---|
| * REVLIMID® was given according to schedule for 2W, followed by a recovery period for 2W and then the cycle was repeated. TW = twice weekly, 2W = 2 weeks, Q1D = every day, Q7D = every 7 days. | | | | | |

### Disseminated U266 model

Six-eight weeks-old female NOD/SCID/γ^{null} (NOG) mice were obtained from Taconic, Denmark. Five millions (5x10⁶) of U266 tumour cells in 200 µL of RPMI 1640 were intravenously injected into the caudal vein (D0). The tumour cell injection was performed 2 hours after whole body irradiation of mice (1.5 Gy, Cesium 137, IBL 637). At D4, mice were randomized into 6 groups and treatment was initiated according to the schedule below. Animals were sacrificed when they displayed hind limb paralysis or >15 % loss in body weight (relative weight at D0).

The treatment schedule:

| **Groups** | **No. mice** | **Treatment** | **Dose (mg/kg/inj)** | **Adm. route** | **Schedule** |
|---|---|---|---|---|---|
| 1 | 8 | FITC-8GA | 10 | i.p | TWx8 |
| 2 | 8 | B11 | 10 | i.p | TWx8 |
| 3 | 8 | REVLIMID® | 4 | P.O | (Q1Dx5)x2W* |
| 4 | 8 | VELCADE® | 1 | i.p | Q7Dx8 |
| 5 | 8 | B11 + REVLIMID® | 10+4 | i.p + P.O | TWx8 + (Q1 Dx5)x2W* |
| 6 | 8 | B11 + VELCADE® | 10 + 1 | P.O | TWx8 + Q7Dx8 |

| | | | | | |
|---|---|---|---|---|---|
| * REVLIMID® was given according to schedule for 2W, followed by a recovery period for 2W and then the cycle was repeated. | | | | | |

The results are shown in figures 16, 17 and 18 and demonstrate that combination therapy using B11 with either Velcade or Revlimid provide improved outcomes, namely a greater reduction in tumour size and growth and an improved survival time.

### Example 11 - Preferred pharmaceutical formulations and modes and doses of administration

The antibodies or antigen-binding fragments thereof of the present invention may be delivered using an injectable sustained-release drug delivery system. These are designed specifically to reduce the frequency of injections. An example of such a system is Nutropin Depot which encapsulates recombinant human growth hormone (rhGH) in biodegradable microspheres that, once injected, release rhGH slowly over a sustained period.

The antibodies or antigen-binding fragments thereof of the present invention can be administered by a surgically implanted device that releases the drug directly to the required site. For example, Vitrasert releases ganciclovir directly into the eye to treat CMV retinitis. The direct application of this toxic agent to the site of disease achieves effective therapy without the drug's significant systemic side-effects.

Electroporation therapy (EPT) systems can also be employed for administration. A device which delivers a pulsed electric field to cells increases the permeability of the cell membranes to the drug, resulting in a significant enhancement of intracellular drug delivery.

Antibodies or antigen-binding fragments thereof of the invention can also be delivered by electroincorporation (EI). EI occurs when small particles of up to 30 microns in diameter on the surface of the skin experience electrical pulses identical or similar to those used in electroporation. In EI, these particles are driven through the stratum corneum and into deeper layers of the skin. The particles can be loaded or coated with drugs or genes or can simply act as "bullets" that generate pores in the skin through which the drugs can enter.

An alternative method of administration is the ReGel injectable system that is thermosensitive. Below body temperature, ReGel is an injectable liquid while at body temperature it immediately forms a gel reservoir that slowly erodes and dissolves into known, safe, biodegradable polymers. The active drug is delivered over time as the biopolymers dissolve.

Antibodies or antigen-binding fragments of the invention can be introduced to cells by "Trojan peptides". These are a class of polypeptides called penetratins which have translocating properties and are capable of carrying hydrophilic compounds across the plasma membrane. This system allows direct targeting of antibodies or antigen binding fragments thereof to the cytoplasm and nucleus, and may be non-cell type specific and highly efficient (Derossi et al., 1998, Trends Cell Biol., 8, 84-87).
dPreferably, the pharmaceutical formulation of the present invention is a unit dosage Preferably, the pharmaceutical formulation of the present invention is a unit dosage containing a daily dose or unit, daily sub-dose or an appropriate fraction thereof, of the active ingredient.

The antibodies or antigen-binding fragments of the invention can be administered by any parenteral route, in the form of a pharmaceutical formulation comprising the active ingredient, optionally in the form of a non-toxic organic, or inorganic, acid, or base, addition salt, in a pharmaceutically acceptable dosage form. Depending upon the disorder and patient to be treated, as well as the route of administration, the compositions may be administered at varying doses.

In human therapy, the antibodies or antigen-binding fragments of the invention can be administered alone but will generally be administered in admixture with a suitable pharmaceutical exipient diluent or carrier selected with regard to the intended route of administration and standard pharmaceutical practice.

The antibodies or antigen-binding fragments of the invention can also be administered parenterally, for example, intravenously, intra-arterially, intraperitoneally, intra-thecally, intraventricularly, intrasternally, intracranially, intra-muscularly or subcutaneously, or they may be administered by infusion techniques. They are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Generally, in humans, oral or parenteral administration of the antibodies of the invention is the preferred route, being the most convenient.

For veterinary use, the antibodies or antigen-binding fragments of the invention are administered as a suitably acceptable formulation in accordance with normal veterinary practice and the veterinary surgeon will determine the dosing regimen and route of administration which will be most appropriate for a particular animal.

The formulations of the pharmaceutical compositions of the invention may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Preferred unit dosage formulations are those containing a daily dose or unit, daily sub-dose or an appropriate fraction thereof, of an active ingredient.

A preferred delivery system of the invention may comprise a hydrogel impregnated with a polypeptides, polynucleotides and antibodies of the invention, which is preferably carried on a tampon which can be inserted into the cervix and withdrawn once an appropriate cervical ripening or other desirable affect on the female reproductive system has been produced.

It should be understood that in addition to the ingredients particularly mentioned above the formulations of this invention may include other agents conventional in the art having regard to the type of formulation in question.

### Example 12 - Exemplary pharmaceutical formulations

Whilst it is possible for antibodies of the invention to be administered alone, it is preferable to present it as a pharmaceutical formulation, together with one or more acceptable carriers. The carrier(s) must be "acceptable" in the sense of being compatible with the compound of the invention and not deleterious to the recipients thereof. Typically, the carriers will be water or saline which will be sterile and pyrogen-free.

The following examples illustrate pharmaceutical formulations according to the invention in which the active ingredient is a compound of the invention.

**Example 12A: Injectable Formulation**

| | |
|---|---|
| Active ingredient | 0.200 g |
| Sterile, pyrogen free phosphate buffer (pH7.0) to | 10 ml |

The active ingredient is dissolved in most of the phosphate buffer (35-40°C), then made up to volume and filtered through a sterile micropore filter into a sterile 10 ml amber glass vial (type 1) and sealed with sterile closures and overseals.

**Example 12B: Intramuscular injection**

| | |
|---|---|
| Active ingredient | 0.20 g |
| Benzyl Alcohol | 0.10 g |
| Glucofurol 75® | 1.45 g |
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

**Example 12C: Tablet**

| | |
|---|---|
| Active ingredient | 100 mg |
| Lactose | 200 mg |
| Starch | 50 mg |
| Polyvinylpyrrolidone | 5 mg |
| Magnesium stearate | 4 mg |
| | 359 mg |

Tablets are prepared from the foregoing ingredients by wet granulation followed by compression.

### Numbered References:

1. Weiner, L.M., Surana, R., and Wang, S. Monoclonal antibodies: versatile platforms for cancer immunotherapy. Nat Rev Immunol 10:317-327.
2. McLaughlin, P., Grillo-Lopez, A.J., Link, B.K., Levy, R., Czuczman, M.S., Williams, M.E., Heyman, M.R., Bence-Bruckler, I., White, C.A., Cabanillas, F., et al. 1998. Rituximab chimeric anti-CD20 monoclonal antibody therapy for relapsed indolent lymphoma: half of patients respond to a four-dose treatment program. J Clin Oncol 16:2825-2833.
3. Davis, T.A., White, C.A., Grillo-Lopez, A.J., Velasquez, W.S., Link, B., Maloney, D.G., Dillman, R.O., Williams, M.E., Mohrbacher, A., Weaver, R., et al. 1999. Single-agent monoclonal antibody efficacy in bulky non-Hodgkin's lymphoma: results of a phase II trial of rituximab. J Clin Oncol 17:1851-1857.
4. Hiddemann, W., Kneba, M., Dreyling, M., Schmitz, N., Lengfelder, E., Schmits, R., Reiser, M., Metzner, B., Harder, H., Hegewisch-Becker, S., et al. 2005. Frontline therapy with rituximab added to the combination of cyclophosphamide, doxorubicin, vincristine, and prednisone (CHOP) significantly improves the outcome for patients with advanced-stage follicular lymphoma compared with therapy with CHOP alone: results of a prospective randomized study of the German Low-Grade Lymphoma Study Group. Blood 106:3725-3732.
5. Herold, M., Haas, A., Srock, S., Neser, S., Al-Ali, K.H., Neubauer, A., Dolken, G., Naumann, R., Knauf, W., Freund, M., et al. 2007. Rituximab added to first-line mitoxantrone, chlorambucil, and prednisolone chemotherapy followed by interferon maintenance prolongs survival in patients with advanced follicular lymphoma: an East German Study Group Hematology and Oncology Study. J Clin Oncol 25:1986-1992.
6. Marcus, R., Imrie, K., Solal-Celigny, P., Catalano, J.V., Dmoszynska, A., Raposo, J.C., Offner, F.C., Gomez-Codina, J., Belch, A., Cunningham, D., et al. 2008. Phase III study of R-CVP compared with cyclophosphamide, vincristine, and prednisone alone in patients with previously untreated advanced follicular lymphoma. J Clin Oncol 26:4579-4586.
7. Coiffier, B. 2007. Rituximab therapy in malignant lymphoma. Oncogene 26:3603-3613.
8. Cheson, B.D., and Leonard, J.P. 2008. Monoclonal antibody therapy for B-cell non-Hodgkin's lymphoma. N Engl J Med 359:613-626.
9. Smith, M.R. 2003. Rituximab (monoclonal anti-CD20 antibody): mechanisms of action and resistance. Oncogene 22:7359-7368.
10. Fransson, J., Tornberg, U.C., Borrebaeck, C.A., Carlsson, R., and Frendeus, B. 2006. Rapid induction of apoptosis in B-cell lymphoma by functionally isolated human antibodies. Int J Cancer 119:349-358.
11. Horst, E., Radaszkiewicz, T., Hooftman-den Otter, A., Pieters, R., van Dongen, J.J., Meijer, C.J., and Pals, S.T. 1991. Expression of the leucocyte integrin LFA-1 (CD11a/CD18) and its ligand ICAM-1 (CD54) in lymphoid malignancies is related to lineage derivation and stage of differentiation but not to tumor grade. Leukemia 5:848-853.
12. Hideshima, T., Mitsiades, C., Tonon, G., Richardson, P.G., and Anderson, K.C. 2007. Understanding multiple myeloma pathogenesis in the bone marrow to identify new therapeutic targets. Nat Rev Cancer 7:585-598.
13. Huang, Y.W., Richardson, J.A., and Vitetta, E.S. 1995. Anti-CD54 (ICAM-1) has antitumor activity in SCID mice with human myeloma cells. Cancer Res 55:610-616.
14. Schmidmaier, R., Morsdorf, K., Baumann, P., Emmerich, B., and Meinhardt, G. 2006. Evidence for cell adhesion-mediated drug resistance of multiple myeloma cells in vivo. Int J Biol Markers 21:218-222.
15. Johnson, J.P., Stade, B.G., Hupke, U., Holzmann, B., and Riethmuller, G. 1988. The melanoma progression-associated antigen P3.58 is identical to the intercellular adhesion molecule, ICAM-1. Immunobiology 178:275-284.
16. Johnson, J.P., Lehmann, J.M., Stade, B.G., Rothbacher, U., Sers, C., and Riethmuller, G. 1989. Functional aspects of three molecules associated with metastasis development in human malignant melanoma. Invasion Metastasis 9:338-350.
17. Grothey, A., Heistermann, P., Philippou, S., and Voigtmann, R. 1998. Serum levels of soluble intercellular adhesion molecule-1 (ICAM-1, CD54) in patients with non-small-cell lung cancer: correlation with histological expression of ICAM-1 and tumour stage. Br J Cancer 77:801-807.
18. Maruo, Y., Gochi, A., Kaihara, A., Shimamura, H., Yamada, T., Tanaka, N., and Orita, K. 2002. ICAM-1 expression and the soluble ICAM-1 level for evaluating the metastatic potential of gastric cancer. Int J Cancer 100:486-490.
19. Roche, Y., Pasquier, D., Rambeaud, J.J., Seigneurin, D., and Duperray, A. 2003. Fibrinogen mediates bladder cancer cell migration in an ICAM-1-dependent pathway. Thromb Haemost 89:1089-1097.
20. Rosette, C., Roth, R.B., Oeth, P., Braun, A., Kammerer, S., Ekblom, J., and Denissenko, M.F. 2005. Role of ICAM1 in invasion of human breast cancer cells. Carcinogenesis 26:943-950.
21. Aalinkeel, R., Nair, M.P.N., Sufrin, G., Mahajan, S.D., Chadha, K.C., Chawda, R.P., and Schwartz, S.A. 2004. Gene Expression of Angiogenic Factors Correlates with Metastatic Potential of Prostate Cancer Cells. Cancer Res 64:5311-5321.
22. Migkou, M., Terpos, E., Christoulas, M., Gavriatopoulou, M., Boutsikas, M., Gkotzamanidou, M., lakovaki, M., Kastritis, M., Papatheodorou, M., and Dimopoulos, M.A. 2009. Increased levels of Vascular Cell Adhesion Molecule-1 (VCAM-1) and InterCellular Adhesion Molecule-1 (ICAM-1) Correlate with Advanced Disease Features and Poor Survival in Newly Diagnosed Patients with Multiple Myeloma. Reduction Post-Bortezomib- and Lenalidomide-Based Regimens In 51st ASH annual meeting and exposition. New Orleans, LA.
23. Kapoor, P., Greipp, P.T., Morice, W.G., Rajkumar, S.V., Witzig, T.E., and Greipp, P.R. 2008. Anti-CD20 monoclonal antibody therapy in multiple myeloma. Br J Haematol 141:135-148.
24. Tai, Y.-T., Catley, L.P., Mitsiades, C.S., Burger, R., Podar, K., Shringpaure, R., Hideshima, T., Chauhan, D., Hamasaki, M., Ishitsuka, K., et al. 2004. Mechanisms by which SGN-40, a Humanized Anti-CD40 Antibody, Induces Cytotoxicity in Human Multiple Myeloma Cells: Clinical Implications. Cancer Res 64:2846-2852.
25. Treon, S.P., Pilarski, L.M., Belch, A.R., Kelliher, A., Preffer, F.I., Shima, Y., Mitsiades, C.S., Mitsiades, N.S., Szczepek, A.J., Ellman, L., et al. 2002. CD20-directed serotherapy in patients with multiple myeloma: biologic considerations and therapeutic applications. J Immunother 25:72-81.
26. Mitsiades, C.S., Treon, S.P., Mitsiades, N., Shima, Y., Richardson, P., Schlossman, R., Hideshima, T., and Anderson, K.C. 2001. TRAIUApo2L ligand selectively induces apoptosis and overcomes drug resistance in multiple myeloma: therapeutic applications. Blood 98:795-804.
27. Treon, S.P., Mitsiades, C., Mitsiades, N., Young, G., Doss, D., Schlossman, R., and Anderson, K.C. 2001. Tumor Cell Expression of CD59 Is Associated With Resistance to CD20 Serotherapy in Patients With B-Cell Malignancies. J Immunother 24:263-271.
28. Ralph, P. 1985. The human B-cell lineage cell line ARH-77. Cancer 56:2544-2545.
29. Huang, Y.W., Richardson, J.A., Tong, A.W., Zhang, B.Q., Stone, M.J., and Vitetta, E.S. 1993. Disseminated growth of a human multiple myeloma cell line in mice with severe combined immunodeficiency disease. Cancer Res 53:1392-1396.
30. Tong, A.W., Huang, Y.W., Zhang, B.Q., Netto, G., Vitetta, E.S., and Stone, M.J. 1993. Heterotransplantation of human multiple myeloma cell lines in severe combined immunodeficiency (SCID) mice. Anticancer Res 13:593-597.
31. Lokhorst, H.M., Lamme, T., de Smet, M., Klein, S., de Weger, R.A., van Oers, R., and Bloem, A.C. 1994. Primary tumor cells of myeloma patients induce interleukin-6 secretion in long-term bone marrow cultures. Blood 84:2269-2277.
32. Tong, A.W., Zhang, B.Q., Mues, G., Solano, M., Hanson, T., and Stone, M.J. 1994. Anti-CD40 antibody binding modulates human multiple myeloma clonogenicity in vitro. Blood 84:3026-3033.
33. Alsina, M., Boyce, B.F., Mundy, G.R., and Roodman, G.D. 1995. An in vivo model of human multiple myeloma bone disease. Stem Cells 13 Suppl 2:48-50.
34. Bellamy, W.T., Mendibles, P., Bontje, P., Thompson, F., Richter, L., Weinstein, R.S., and Grogan, T.M. 1996. Development of an orthotopic SCID mouse-human tumor xenograft model displaying the multidrug-resistant phenotype. Cancer Chemother Pharmacol 37:305-316.
35. Chauhan, D., Uchiyama, H., Akbarali, Y., Urashima, M., Yamamoto, K., Libermann, T.A., and Anderson, K.C. 1996. Multiple myeloma cell adhesion-induced interleukin-6 expression in bone marrow stromal cells involves activation of NF-kappa B. Blood 87:1104-1112.
36. Urashima, M., Chen, B.P., Chen, S., Pinkus, G.S., Bronson, R.T., Dedera, D.A., Hoshi, Y., Teoh, G., Ogata, A., Treon, S.P., et al. 1997. The development of a model for the homing of multiple myeloma cells to human bone marrow. Blood 90:754-765.
37. Kobune, M., Chiba, H., Kato, J., Kato, K., Nakamura, K., Kawano, Y., Takada, K., Takimoto, R., Takayama, T., Hamada, H., et al. 2007. Wnt3/RhoA/ROCK signaling pathway is involved in adhesion-mediated drug resistance of multiple myeloma in an autocrine mechanism. Mol Cancer Ther 6:1774-1784.
38. Nadav, L., Katz, B.Z., Baron, S., Cohen, N., Naparstek, E., and Geiger, B. 2006. The generation and regulation of functional diversity of malignant plasma cells. Cancer Res 66:8608-8616.
39. Kawai, S., Yoshimura, Y., lida, S., Kinoshita, Y., Koishihara, Y., Ozaki, S., Matsumoto, T., Kosaka, M., and Yamada-Okabe, H. 2006. Antitumor activity of humanized monoclonal antibody against HM1.24 antigen in human myeloma xenograft models. Oncol Rep 15:361-367.
40. Ural, A.U., Yilmaz, M.I., Avcu, F., Pekel, A., Zerman, M., Nevruz, O., Sengul, A., and Yalcin, A. 2003. The bisphosphonate zoledronic acid induces cytotoxicity in human myeloma cell lines with enhancing effects of dexamethasone and thalidomide. Int J Hematol 78:443-449.
41. Drucker, L., Uziel, O., Tohami, T., Shapiro, H., Radnay, J., Yarkoni, S., Lahav, M., and Lishner, M. 2003. Thalidomide down-regulates transcript levels of GC-rich promoter genes in multiple myeloma. Mol Pharmacol 64:415-420.
42. Choi, S.J., Oba, T., Callander, N.S., Jelinek, D.F., and Roodman, G.D. 2003. AML-1A and AML-1B regulation of MIP-1alpha expression in multiple myeloma. Blood 101:3778-3783.
43. Tian, J.Y., Hu, W.X., Tian, E.M., Shi, Y.W., Shen, Q.X., Tang, L.J., and Jiang, Y.S. 2003. Cloning and sequence analysis of tumor-associated gene hMMTAG2 from human multiple myeloma cell line ARH-77. Sheng Wu Hua Xue Yu Sheng Wu Wu Li Xue Bao (Shanghai) 35:143-148.
44. Tong, A.W., Seamour, B., Chen, J., Su, D., Ordonez, G., Frase, L., Netto, G., and Stone, M.J. 2000. CD40 ligand-induced apoptosis is Fas-independent in human multiple myeloma cells. Leuk Lymphoma 36:543-558.
45. Feinman, R., Koury, J., Thames, M., Barlogie, B., Epstein, J., and Siegel, D.S. 1999. Role of NF-kappaB in the rescue of multiple myeloma cells from glucocorticoid-induced apoptosis by bcl-2. Blood 93:3044-3052.
46. Thomas, X., Anglaret, B., Magaud, J.P., Epstein, J., and Archimbaud, E. 1998. Interdependence between cytokines and cell adhesion molecules to induce interleukin-6 production by stromal cells in myeloma. Leuk Lymphoma 32:107-119.
47. Roodman, G.D. 1997. Mechanisms of bone lesions in multiple myeloma and lymphoma. Cancer 80:1557-1563.
48. Ozaki, S., Kosaka, M., Wakatsuki, S., Abe, M., Koishihara, Y., and Matsumoto, T. 1997. Immunotherapy of multiple myeloma with a monoclonal antibody directed against a plasma cell-specific antigen, HM1.24. Blood 90:3179-3186.
49. Lopes de Menezes, D.E., Denis-Mize, K., Tang, Y., Ye, H., Kunich, J.C., Garrett, E.N., Peng, J., Cousens, L.S., Gelb, A.B., Heise, C., et al. 2007. Recombinant interleukin-2 significantly augments activity of rituximab in human tumor xenograft models of B-cell non-Hodgkin lymphoma. J Immunother (1997) 30:64-74.
50. de Bont, E.S., Guikema, J.E., Scherpen, F., Meeuwsen, T., Kamps, W.A., Vellenga, E., and Bos, N.A. 2001. Mobilized human CD34+ hematopoietic stem cells enhance tumor growth in a nonobese diabetic/severe combined immunodeficient mouse model of human non-Hodgkin's lymphoma. Cancer Res 61:7654-7659.
51. Sampaio, M.S., Vettore, A.L., Yamamoto, M., Chauffaille Mde, L., Zago, M.A., and Colleoni, G.W. 2009. Expression of eight genes of nuclear factor-kappa B pathway in multiple myeloma using bone marrow aspirates obtained at diagnosis. Histol Histopathol 24:991-997.
52. Weng, W.K., and Levy, R. 2003. Two immunoglobulin G fragment C receptor polymorphisms independently predict response to rituximab in patients with follicular lymphoma. J Clin Oncol 21:3940-3947.
53. Musolino, A., Naldi, N., Bortesi, B., Pezzuolo, D., Capelletti, M., Missale, G., Laccabue, D., Zerbini, A., Camisa, R., Bisagni, G., et al. 2008. Immunoglobulin G fragment C receptor polymorphisms and clinical efficacy of trastuzumab-based therapy in patients with HER-2/neu-positive metastatic breast cancer. J Clin Oncol 26:1789-1796.
54. Clynes, R.A., Towers, T.L., Presta, L.G., and Ravetch, J.V. 2000. Inhibitory Fc receptors modulate in vivo cytoxicity against tumor targets. Nat Med 6:443-446.
55. Bruhns, P., lannascoli, B., England, P., Mancardi, D.A., Fernandez, N., Jorieux, S., and Daeron, M. 2009. Specificity and affinity of human Fcgamma receptors and their polymorphic variants for human IgG subclasses. Blood 113:3716-3725.
56. Carter, P.J. 2006. Potent antibody therapeutics by design. Nat Rev Immunol 6:343-357.
57. Cragg, M.S., Morgan, S.M., Chan, H.T., Morgan, B.P., Filatov, A.V., Johnson, P.W., French, R.R., and Glennie, M.J. 2003. Complement-mediated lysis by anti-CD20 mAb correlates with segregation into lipid rafts. Blood 101:1045-1052.
58. Manches, O., Lui, G., Chaperot, L., Gressin, R., Molens, J.P., Jacob, M.C., Sotto, J.J., Leroux, D., Bensa, J.C., and Plumas, J. 2003. In vitro mechanisms of action of rituximab on primary non-Hodgkin lymphomas. Blood 101:949-954.
59. Cragg, M.S., and Glennie, M.J. 2004. Antibody specificity controls in vivo effector mechanisms of anti-CD20 reagents. Blood 103:2738-2743.
60. Smith, M.E., and Thomas, J.A. 1990. Cellular expression of lymphocyte function associated antigens and the intercellular adhesion molecule-1 in normal tissue. J Clin Pathol 43:893-900.
61. Roebuck, K.A., and Finnegan, A. 1999. Regulation of intercellular adhesion molecule-1 (CD54) gene expression. J Leukoc Biol 66:876-888.
62. Stebbings, R., Findlay, L., Edwards, C., Eastwood, D., Bird, C., North, D., Mistry, Y., Dilger, P., Liefooghe, E., Cludts, I., et al. 2007. "Cytokine storm" in the phase I trial of monoclonal antibody TGN1412: better understanding the causes to improve preclinical testing of immunotherapeutics. J Immunol 179:3325-3331.
63. Davis, T.A., Grillo-Lopez, A.J., White, C.A., McLaughlin, P., Czuczman, M.S., Link, B.K., Maloney, D.G., Weaver, R.L., Rosenberg, J., and Levy, R. 2000. Rituximab anti-CD20 monoclonal antibody therapy in non-Hodgkin's lymphoma: safety and efficacy of re-treatment. J Clin Oncol 18:3135-3143.
64. Kyle, R.A., and Rajkumar, S.V. 2004. Multiple myeloma. N Engl J Med 351: 1860-1873.
65. Zhang, W., Gordon, M., Schultheis, A.M., Yang, D.Y., Nagashima, F., Azuma, M., Chang, H.M., Borucka, E., Lurje, G., Sherrod, A.E., et al. 2007. FCGR2A and FCGR3A polymorphisms associated with clinical outcome of epidermal growth factor receptor expressing metastatic colorectal cancer patients treated with single-agent cetuximab. J Clin Oncol 25:3712-3718.
66. Lejeune, J., Thibault, G., Ternant, D., Cartron, G., Watier, H., and Ohresser, M. 2008. Evidence for linkage disequilibrium between Fcgamma Rllla-V158F and Fcgamma RIIa-H131R polymorphisms in white patients, and for an Fcgamma RIIIa-restricted influence on the response to therapeutic antibodies. J Clin Oncol 26:5489-5491; author reply 5491-5482.
67. Bibeau, F., Lopez-Crapez, E., Di Fiore, F., Thezenas, S., Ychou, M., Blanchard, F., Lamy, A., Penault-Llorca, F., Frebourg, T., Michel, P., et al. 2009. Impact of Fc{gamma}Rlla-Fc{gamma}Rllla polymorphisms and KRAS mutations on the clinical outcome of patients with metastatic colorectal cancer treated with cetuximab plus irinotecan. J Clin Oncol 27:1122-1129.
68. Schmidmaier, R., Baumann, P., Simsek, M., Dayyani, F., Emmerich, B., and Meinhardt, G. 2004. The HMG-CoA reductase inhibitor simvastatin overcomes cell adhesion-mediated drug resistance in multiple myeloma by geranylgeranylation of Rho protein and activation of Rho kinase. Blood 104:1825-1832.
69. Urashima, M., Chauhan, D., Hatziyanni, M., Ogata, A., Hollenbaugh, D., Aruffo, A., and Anderson, K.C. 1996. CD40 ligand triggers interleukin-6 mediated B cell differentiation. Leuk Res 20:507-515.
70. Yaccoby, S., Barlogie, B., and Epstein, J. 1998. Primary myeloma cells growing in SCID-hu mice: a model for studying the biology and treatment of myeloma and its manifestations. Blood 92:2908-2913.
71. Yaccoby, S., Wezeman, M.J., Henderson, A., Cottier-Fox, M., Yi, Q., Barlogie, B., and Epstein, J. 2004. Cancer and the microenvironment: myeloma-osteoclast interactions as a model. Cancer Res 64:2016-2023.
72. Mitsiades, N., Mitsiades, C.S., Poulaki, V., Chauhan, D., Richardson, P.G., Hideshima, T., Munshi, N.C., Treon, S.P., and Anderson, K.C. 2002. Apoptotic signaling induced by immunomodulatory thalidomide analogs in human multiple myeloma cells: therapeutic implications. Blood 99:4525-4530.
73. Chauhan, D., Pandey, P., Hideshima, T., Treon, S., Raje, N., Davies, F.E., Shima, Y., Tai, Y.T., Rosen, S., Avraham, S., et al. 2000. SHP2 mediates the protective effect of interleukin-6 against dexamethasone-induced apoptosis in multiple myeloma cells. J Biol Chem 275:27845-27850.
74. Hideshima, T., Chauhan, D., Shima, Y., Raje, N., Davies, F.E., Tai, Y.T., Treon, S.P., Lin, B., Schlossman, R.L., Richardson, P., et al. 2000. Thalidomide and its analogs overcome drug resistance of human multiple myeloma cells to conventional therapy. Blood 96:2943-2950.
75. Schneider, D., Berrouschot, J., Brandt, T., Hacke, W., Ferbert, A., Norris, S.H., Polmar, S.H., and Schafer, E. 1998. Safety, pharmacokinetics and biological activity of enlimomab (anti-ICAM-1 antibody): an open-label, dose escalation study in patients hospitalized for acute stroke. Eur Neurol 40:78-83.
76. Salmela, K., Wramner, L., Ekberg, H., Hauser, I., Bentdal, O., Lins, L.E., Isoniemi, H., Backman, L., Persson, N., Neumayer, H.H., et al. 1999. A randomized multicenter trial of the anti-ICAM-1 monoclonal antibody (enlimomab) for the prevention of acute rejection and delayed onset of graft function in cadaveric renal transplantation: a report of the European Anti-ICAM-1 Renal Transplant Study Group. Transplantation 67:729-736.
77. Mileski, W.J., Burkhart, D., Hunt, J.L., Kagan, R.J., Saffle, J.R., Herndon, D.N., Heimbach, D.M., Luterman, A., Yurt, R.W., Goodwin, C.W., et al. 2003. Clinical effects of inhibiting leukocyte adhesion with monoclonal antibody to intercellular adhesion molecule-1 (enlimomab) in the treatment of partial-thickness burn injury. J Trauma 54:950-958.
78. Kavanaugh, A.F., Davis, L.S., Nichols, L.A., Norris, S.H., Rothlein, R., Scharschmidt, L.A., and Lipsky, P.E. 1994. Treatment of refractory rheumatoid arthritis with a monoclonal antibody to intercellular adhesion molecule 1. Arthritis Rheum 37:992-999.
79. Kavanaugh, A.F., Davis, L.S., Jain, R.I., Nichols, L.A., Norris, S.H., and Lipsky, P.E. 1996. A phase I/II open label study of the safety and efficacy of an anti-ICAM-1 (intercellular adhesion molecule-1; CD54) monoclonal antibody in early rheumatoid arthritis. J Rheumatol 23:1338-1344.
80. Kavanaugh, A.F., Schulze-Koops, H., Davis, L.S., and Lipsky, P.E. 1997. Repeat treatment of rheumatoid arthritis patients with a murine anti-intercellular adhesion molecule 1 monoclonal antibody. Arthritis Rheum 40:849-853.

## Claims

1. A composition comprising: (i) an antibody or an antigen-binding fragment thereof with binding specificity for ICAM-1, wherein the antibody or antigen-binding fragment thereof comprises the following amino acid sequences:
FSNAWMSWVRQAPG and
AFIWYDGSNKYYADSVKGR and
ARYSGWYFDY and
CTGSSSNIGAGYDVH and
DNNNRPS and
CQSYDSSLSAWL; (ii) a further anticancer agent selected from IMiDs and bortezomib; and (iii) a pharmaceutically acceptable excipient, diluent or carrier.

2. A therapeutic system for the treatment of cancer comprising a combination of component (i) an antibody or an antigen-binding fragment thereof with binding specificity for ICAM-1,wherein the antibody or antigen-binding fragment thereof comprises the following amino acid sequences:
FSNAWMSWVRQAPG and
AFIWYDGSNKYYADSVKGR and
ARYSGWYFDY and
CTGSSSNIGAGYDVH and
DNNNRPS and
CQSYDSSLSAWL; and component (ii) a further anticancer agent selected from IMiDs and bortezomib, the components (i) and (ii) being provided for the use in the treatment of cancer, wherein each of components (i) and (ii) optionally additionally comprises a pharmaceutically acceptable excipient, diluent or carrier, and wherein components (i) and (ii) are administered in combination with one another.

3. A therapeutic system for use as claimed in Claim 2 wherein administration of component (i) precedes administration of component (ii).

4. A therapeutic system for use as claimed in Claim 2 wherein administration of component (ii) precedes administration of component (i).

5. An antibody or an antigen-binding fragment thereof with binding specificity for ICAM-1,wherein the antibody or antigen-binding fragment thereof comprises the following amino acid sequences:
FSNAWMSWVRQAPG and
AFIWYDGSNKYYADSVKGR and
ARYSGWYFDY and
CTGSSSNIGAGYDVH and
DNNNRPS and
CQSYDSSLSAWL; together with a further anticancer agent selected from IMiDs and bortezomib, for use in the treatment of cancer.

6. The composition, system, or antibody for use of any previous claim wherein the further anticancer agent is selected from bortezomib and lenalidomide.

7. The composition, system, or antibody for use of any previous claim wherein the further anticancer agent is present in an individually effective dose.

8. The composition, system, or antibody for use of any previous claim wherein the further anticancer agent is present in a lower than individually effective dose.

9. The composition, system, or antibody for use of any previous claim wherein the cancer to be treated is a lymphoproliferative disorder, preferably multiple myeloma.

10. The composition, system, or antibody for use according to any one of the preceding claims wherein the effective amount of the antibodyor antigen-binding fragment thereof is between about 0.1µg to 5g of the antibody or antigen-binding fragment thereof.

11. The composition, system, or antibody for use according to any preceding claim wherein the antibody or antigen-binding fragment thereof, comprises or consists of an intact antibody.

12. The composition, system, or antibody for use according to any one of the preceding claims wherein the antibody or antigen-binding fragment thereof, comprises or consists of an antigen-binding fragment selected from the group consisting of: a single chain Fv fragment, a disulphide-bonded Fv fragment, an Fab fragment, an Fab' fragment, and an F(ab)₂ fragment.

13. The composition, system, or antibody for use according to any one of the preceding claims wherein the antibody or antigen-binding fragment thereof is a recombinant antibody, a monoclonal antibody, and/or a human antibody or humanised antibody.

14. The composition, system, or antibody for use according to any one of the preceding claims wherein the antibody or antigen-binding fragment thereof has one or more of the variable regions having the following amino acid sequences shown in Fig. 15.

15. A kit of parts comprising:
(a) a composition as defined in claim 1
(b) apparatus for administering the composition; and
(c) instructions for use.

## Patentansprüche

1. Zusammensetzung, umfassend: (i) einen Antikörper oder ein antigenbindendes Fragment davon mit Bindungsspezifität für ICAM-1, wobei der Antikörper oder das antigenbindende Fragment davon die folgenden Aminosäuresequenzen umfasst:
FSNAWMSWVRQAPG und
AFIWYDGSNKYYADSVKGR und
ARYSGWYFDY und
CTGSSSNIGAGYDVH und
DNNNRPS und
CQSYDSSLSAWL; (ii) ein weiteres Antikrebsmittel, das aus IMiDs und Bortezomib ausgewählt ist; und (iii) einen pharmazeutisch unbedenklichen Hilfsstoff, ein pharmazeutisch unbedenkliches Verdünnungsmittel oder einen pharmazeutisch unbedenklichen Träger.

2. Therapeutisches System zur Behandlung von Krebs, umfassend eine Kombination einer Komponente (i) eines Antikörpers oder eines antigenbindenden Fragments davon mit Bindungsspezifität für ICAM-1, wobei der Antikörper oder das antigenbindende Fragment davon die folgenden Aminosäuresequenzen umfasst:
FSNAWMSWVRQAPG und
AFIWYDGSNKYYADSVKGR und
ARYSGWYFDY und
CTGSSSNIGAGYDVH und
DNNNRPS und
CQSYDSSLSAWL; und einer Komponente (ii) eines weiteren Antikrebsmittels, das aus IMiDs und Bortezomib ausgewählt ist, wobei die Komponenten (i) und (ii) zur Verwendung bei der Behandlung von Krebs bereitgestellt sind, wobei jede der Komponenten (i) und (ii) optional zusätzlich einen pharmazeutisch unbedenklichen Hilfsstoff, ein pharmazeutisch unbedenkliches Verdünnungsmittel oder einen pharmazeutisch unbedenklichen Träger umfasst und wobei die Komponenten (i) und (ii) in Kombination miteinander verabreicht werden.

3. Therapeutisches System zur Verwendung nach Anspruch 2, wobei die Verabreichung der Komponente (i) der Verabreichung der Komponente (ii) vorausgeht.

4. Therapeutisches System zur Verwendung nach Anspruch 2, wobei die Verabreichung der Komponente (ii) der Verabreichung der Komponente (i) vorausgeht.

5. Antikörper oder ein antigenbindendes Fragment davon mit Bindungsspezifität für ICAM-1, wobei der Antikörper oder das antigenbindende Fragment davon die folgenden Aminosäuresequenzen umfasst:
FSNAWMSWVRQAPG und
AFIWYDGSNKYYADSVKGR und
ARYSGWYFDY und
CTGSSSNIGAGYDVH und
DNNNRPS und
CQSYDSSLSAWL; zusammen mit einem weiteren Antikrebsmittel, das aus IMiDs und Bortezomib ausgewählt ist, zur Verwendung bei der Behandlung von Krebs.

6. Zusammensetzung, System oder Antikörper zur Verwendung nach einem der vorhergehenden Ansprüche, wobei das weitere Antikrebsmittel aus Bortezomib und Lenalidomid ausgewählt ist.

7. Zusammensetzung, System oder Antikörper zur Verwendung nach einem der vorhergehenden Ansprüche, wobei das weitere Antikrebsmittel in einer individuell wirksamen Dosis vorhanden ist.

8. Zusammensetzung, System oder Antikörper zur Verwendung nach einem der vorhergehenden Ansprüche, wobei das weitere Antikrebsmittel in einer niedrigeren als individuell wirksamen Dosis vorhanden ist.

9. Zusammensetzung, System oder Antikörper zur Verwendung nach einem der vorhergehenden Ansprüche, wobei es sich bei dem zu behandelnden Krebs um eine lymphoproliferative Krankheit, vorzugsweise um ein multiples Myelom, handelt.

10. Zusammensetzung, System oder Antikörper zur Verwendung nach einem der vorhergehenden Ansprüche, wobei die wirksame Menge des Antikörpers oder des antigenbindenden Fragments davon zwischen etwa 0,1 µg und 5 µg des Antikörpers oder des antigenbindenden Fragments davon liegt.

11. Zusammensetzung, System oder Antikörper zur Verwendung nach einem der vorhergehenden Ansprüche, wobei der Antikörper oder das antigenbindende Fragment davon einen intakten Antikörper umfasst oder aus einem intakten Antikörper besteht.

12. Zusammensetzung, System oder Antikörper zur Verwendung nach einem der vorhergehenden Ansprüche, wobei der Antikörper oder das antigenbindende Fragment davon ein antigenbindendes Fragment umfasst oder aus einem antigenbindenden Fragment besteht, das aus der Gruppe ausgewählt ist, die aus einem einzelkettigen Fv-Fragment, einem Disulfid-gebundenen Fv-Fragment, einem Fab-Fragment, einem Fab'-Fragment und einem F(ab)₂-Fragment besteht.

13. Zusammensetzung, System oder Antikörper zur Verwendung nach einem der vorhergehenden Ansprüche, wobei es sich bei dem Antikörper oder dem antigenbindenden Fragment davon um einen rekombinanten Antikörper, einen monoklonalen Antikörper und/oder einen humanen Antikörper oder einen humanisierten Antikörper handelt.

14. Zusammensetzung, System oder Antikörper zur Verwendung nach einem der vorhergehenden Ansprüche, wobei der Antikörper oder das antigenbindende Fragment davon eine oder mehrere der variablen Regionen mit den folgenden, in Fig. 15 gezeigten Aminosäuresequenzen aufweist.

15. Kit mit Teilen, umfassend:
(a) eine Zusammensetzung nach Anspruch 1
(b) eine Einrichtung zum Verabreichen der Zusammensetzung; und
(c) eine Gebrauchsanleitung.

## Revendications

1. Composition comprenant : (i) un anticorps ou un fragment de liaison à l'antigène de celui-ci ayant une spécificité de liaison pour ICAM-1, l'anticorps ou le fragment de liaison à l'antigène de celui-ci comprenant les séquences d'acides aminés suivantes :
FSNAWMSWVRQAPG et
AFIWYDGSNKYYADSVKGR et
ARYSGWYFDY et
CTGSSSNIGAGYDVH et
DNNNRPS et
CQSYDSSLSAWL ; (ii) un autre agent anticancéreux choisi parmi des IMiD et le bortézomib ; et (iii) un excipient, un diluant ou un support pharmaceutiquement acceptable.

2. Système thérapeutique destiné au traitement du cancer comprenant une combinaison d'un composant (i) un anticorps ou un fragment de liaison à l'antigène de celui-ci ayant une spécificité de liaison pour ICAM-1, l'anticorps ou le fragment de liaison à l'antigène de celui-ci comprenant les séquences d'acides aminés suivantes :
FSNAWMSWVRQAPG et
AFIWYDGSNKYYADSVKGR et
ARYSGWYFDY et
CTGSSSNIGAGYDVH et
DNNNRPS et
CQSYDSSLSAWL ; et d'un component (ii) un autre agent anticancéreux choisi parmi des IMiD et le bortézomib, les composants (i) et (ii) étant destinés à être utilisés dans le traitement du cancer, chacun des composants (i) et (ii) comprenant éventuellement en outre un excipient, un diluant ou un support pharmaceutiquement acceptable, et les composants (i) et (ii) étant administrés en association l'un avec l'autre.

3. Système thérapeutique destiné à être utilisé selon la revendication 2, dans lequel l'administration du composant (i) précède l'administration du composant (ii).

4. Système thérapeutique destiné à être utilisé selon la revendication 2, dans lequel l'administration du composant (ii) précède l'administration du composant (i).

5. Anticorps ou fragment de liaison à l'antigène de celui-ci ayant une spécificité de liaison pour ICAM-1, l'anticorps ou le fragment de liaison à l'antigène de celui-ci comprenant les séquences d'acides aminés suivantes :
FSNAWMSWVRQAPG et
AFIWYDGSNKYYADSVKGR et
ARYSGWYFDY et
CTGSSSNIGAGYDVH et
DNNNRPS et
CQSYDSSLSAWL ; avec un autre agent anticancéreux choisi parmi des IMiD et le bortézomib, destiné à être utilisé dans le traitement du cancer.

6. Composition, système ou anticorps destiné à être utilisé selon l'une quelconque des revendications précédentes, dans lequel l'autre agent anticancéreux est choisi parmi le bortézomib et la lénalidomide.

7. Composition, système ou anticorps destiné à être utilisé selon l'une quelconque des revendications précédentes, dans lequel l'autre agent anticancéreux est présent dans une dose efficace individuelle.

8. Composition, système ou anticorps destiné à être utilisé selon l'une quelconque des revendications précédentes, dans lequel l'autre agent anticancéreux est présent dans une dose inférieure à une dose efficace individuelle.

9. Composition, système ou anticorps destiné à être utilisé selon l'une quelconque des revendications précédentes, dans lequel le cancer à traiter est un syndrome lymphoprolifératif, de préférence un myélome multiple.

10. Composition, système ou anticorps destiné à être utilisé selon l'une quelconque des revendications précédentes, dans lequel la quantité efficace de l'anticorps ou du fragment de liaison à l'antigène de celui-ci est comprise entre environ 0,1 µg et 5 g de l'anticorps ou du fragment de liaison à l'antigène de celui-ci.

11. Composition, système ou anticorps destiné à être utilisé selon l'une quelconque des revendications précédentes, dans lequel l'anticorps ou le fragment de liaison à l'antigène de celui-ci comprend ou est constitué d'un anticorps intact.

12. Composition, système ou anticorps destiné à être utilisé selon l'une quelconque des revendications précédentes, dans lequel l'anticorps ou le fragment de liaison à l'antigène de celui-ci comprend ou est constitué d'un fragment de liaison à l'antigène choisi dans le groupe constitué : d'un fragment Fv simple chaîne, d'un fragment Fv à liaison disulfure, d'un fragment Fab, d'un fragment Fab' et d'un fragment F(ab)₂.

13. Composition, système ou anticorps destiné à être utilisé selon l'une quelconque des revendications précédentes, dans lequel l'anticorps ou le fragment de liaison à l'antigène de celui-ci est un anticorps recombinant, un anticorps monoclonal et/ou un anticorps humain ou un anticorps humanisé.

14. Composition, système ou anticorps destiné à être utilisé selon l'une quelconque des revendications précédentes, dans lequel l'anticorps ou le fragment de liaison à l'antigène de celui-ci présente une ou plusieurs des régions variables ayant les séquences d'acides aminés suivantes présentées dans la figure 15.

15. Trousse d'éléments comprenant :
(a) une composition selon la revendication 1 ;
(b) un appareil destiné à administrer la composition ; et
(c) des instructions d'utilisation.
